# EUROPÄISCHE PATENTSCHRIFT

(11) **EP 0 644 195 B1**
(45) Veröffentlichungstag und Bekanntmachung des Hinweises auf die Patenterteilung: **07.07.1999**
(21) Anmeldenummer: 94810528.3
(22) Anmeldetag: 13.09.1994
(51) Int. Cl.: C07G 17/00, C07B 45/00, C07C 69/732, C07F 9/165, C09K 15/14, C09K 15/08, C09K 15/32, C08K 5/36, C08K 5/134, C08K 5/5398

(54) **Flüssige Antioxidantien als Stabilisatoren**
Liquid antioxidants as stabilisers
Antioxydants liquides comme stabilisants

(30) Priorität: 20.09.1993 CH 284493
(43) Veröffentlichungstag der Anmeldung: 22.03.1995
(73) Patentinhaber: Ciba Specialty Chemicals Holding Inc., 4057 Basel (CH)
(72) Erfinder: Evans, Samuel Dr., CH-1723 Marly (CH); Dubs, Paul Dr., CH-1700 Fribourg (CH); Camenzind, Hugo Dr., CH-3014 Bern (CH)

(56) Entgegenhaltungen:
- EP-A- 0 027 776
- EP-A- 0 565 487
- US-A- 3 944 594

## Beschreibung

Die vorliegende Erfindung betrifft neue flüssige und schwer flüchtige Antioxidantien, Zusammensetzungen, enthaltend ein organisches Material, bevorzugt ein Polymer oder Oel und die neuen, schwer flüchtigen, flüssigen Antioxidantien, sowie die Verwendung derselben zum Stabilisieren von organischen Materialien gegen oxidativen, thermischen oder lichtinduzierten Abbau.

Die Stabilisierung, insbesondere von Schmierstoffen oder von Kunststoffen mit Antioxidantien aus der Reihe der sterisch gehinderten Phenole, ist beispielsweise bekannt aus US-A-3 839 278, US-A-4 032 562, US-A-4 058 502, US-A-4 093 587 und US-A-4 132 702.

In WO 91/13134 ist eine Methode beschrieben, wie die Löslichkeit von Antioxidantien in einem zweiten Medium verbessert werden kann.

Die vorliegende Erfindung betrifft Produkte, erhältlich durch Umsetzung der Komponenten a), b), c) und d), wobei die Komponente a) eine Verbindung der Formel I oder ein Gemisch von Verbindungen der Formel I, die Komponente b) eine Verbindung der Formel II oder ein Gemisch von Verbindungen der Formel II, die Komponente c) eine Verbindung der Formel III oder ein Gemisch von Verbindungen der Formel III, und die Komponente d) eine Verbindung der Formel IV oder ein Gemisch von Verbindungen der Formel IV sind, 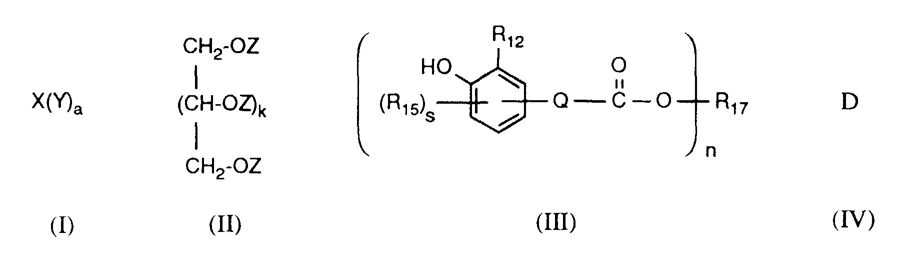 wobei in der Verbindung der Formel I die Reste
- Y: unabhängig voneinander OH, (HOCH₂CH₂)₂N- oder -HNR₁ darstellt und
- R₁: Wasserstoff, C₁-C₁₈-Alkyl, C₅-C₁₂-Cycloalkyl, 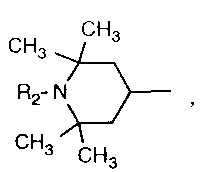 C₃-C₆-Alkenyl, C₇-C₉-Phenylalkyl, Phenyl oder durch 1 bis 3 Reste A₁ substituiertes Phenyl bedeutet, wobei die Reste A₁ unabhängig voneinander C₁-C₁₂-Alkyl, Halogen, Hydroxy, Methoxy oder Ethoxy bedeuten, wobei
- R₂: Wasserstoff, C₁-C₈-Alkyl, O^{•}, OH, NO, -CH₂CN, C₁-C₁₈-Alkoxy, C₅-C₁₂-Cycloalkoxy, C₃-C₆-Alkenyl, C₇-C₉-Phenylalkyl oder C₇-C₉-Phenylalkyl, welches am Phenylring mit C₁-C₄-Alkyl mono-, di- oder tri-substituiert ist, oder R₂ ferner C₁-C₈-Acyl oder HOCH₂CH₂- darstellt, und
- a: die Zahl 1,2,3,4 oder 6 bedeutet, wobei
wenn Y = OH und a = 1 ist,
- X: C₁-C₄₅-Alkyl, C₃-C₁₈-Alkenyl, -CH₂CH₂T₁(CH₂CH₂O)_{b}R₄ oder 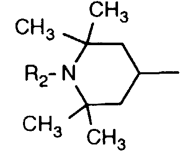 bedeutet, wobei R₂ die obige Bedeutung hat, und
- T₁: Sauerstoff, Schwefel oder 〉N-R₅,
- R₄: C₁-C₂₀-Alkyl,
- b: eine ganze Zahl aus dem Bereich von 0 bis 10 und
- R₅: Wasserstoff, C₁-C₁₈-Alkyl oder Phenyl darstellt, oder
wenn Y = OH und a = 2 ist,
- X: -CH₂CH₂T₂(CH₂CH₂O)_{b}CH₂CH₂-, wobei b die obige Bedeutung hat,

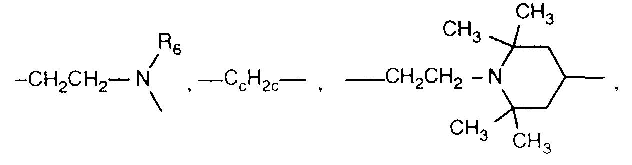

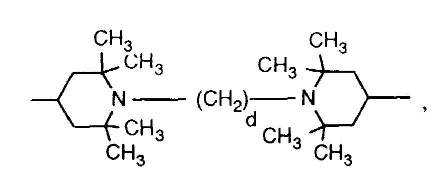

-CH₂-CH=CH-CH₂-,

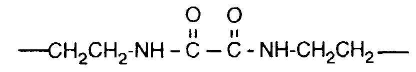

oder 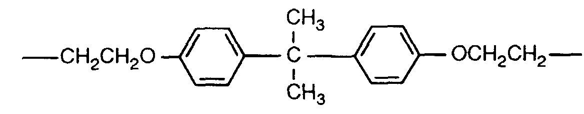 bedeutet, wobei
- T₂: Sauerstoff, Schwefel, 〉N-R₅ oder 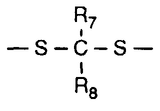 ist und R₅ die obige Bedeutung hat,
- R₆: Wasserstoff, C₁-C₁₈-Alkyl oder Phenyl,
- c: eine ganze Zahl aus dem Bereich von 2 bis 10,
- d: eine ganze Zahl aus dem Bereich von 2 bis 6 und
- R₇, R₈: unabhängig voneinander Wasserstoff, C₁-C₁₈-Alkyl oder Phenyl sind, oder R₇ und R₈ mit dem C-Atom, an das sie gebunden sind, einen C₅-C₁₂-Cycloalkylidenring bilden, oder
wenn a = 3 ist,
- X: C₃-C₁₀-Alkantriyl oder N(CH₂CH₂-)₃ bedeutet, oder
wenn Y = OH und a = 4 ist,
- X: C₄-C₁₀-Alkantetrayl, 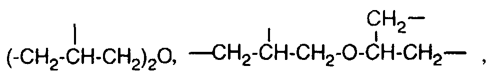 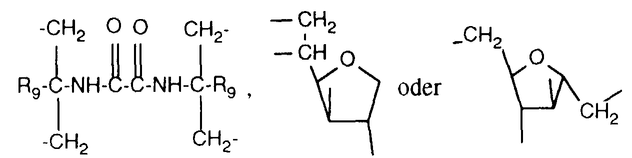 darstellt, wobei
- R₉: C₁-C₄-Alkyl bedeutet, oder
wenn Y = OH und a = 6 ist,
- X: 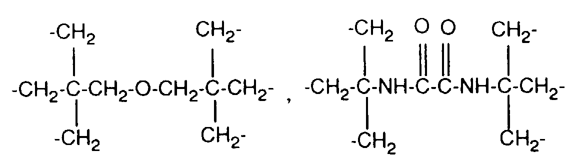 oder C₆-C₁₀-Alkanhexayl darstellt, oder
wenn Y = -HNR₁ und a = 1 ist,
- X: C₁-C₁₈-Alkyl, C₃-C₁₈-Alkenyl, C₅-C₁₂-Cycloalkyl, C₇-C₉-Phenylalkyl, Phenyl, 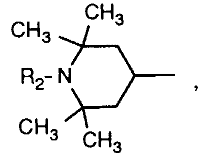 wobei R₂ die obige Bedeutung hat, oder X ferner 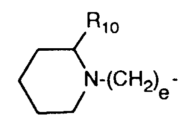 ist, oder X mit R₁ gemeinsam eine Gruppe der Formel -CH₂CH₂CH₂CH₂CH₂- oder -CH₂CH₂OCH₂CH₂- bildet, wobei
- R₁₀: Wasserstoff oder Methyl, und
- e: 2 oder 3 bedeutet, oder
wenn Y = -HNR₁ und a = 2 ist,
- X: -C_{f}H_{2f}-, 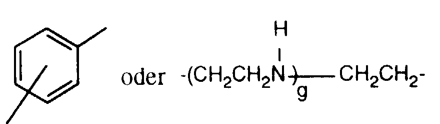 -C_{f}H_{2f}-, darstellt, wobei
- f: eine ganze Zahl aus dem Bereich von 2 bis 10 und
- g: eine ganze Zahl aus dem Bereich von 1 bis 6 bedeutet, und
in der Verbindung der Formel II die Reste
- Z: Wasserstoff oder eine Gruppe der Formel 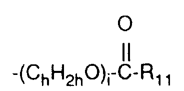 bedeutet, und
- k: eine ganze Zahl aus dem Bereich von 0 bis 6 darstellt, wobei
- h: 2 oder 3,
- i: eine ganze Zahl aus dem Bereich von 0 bis 12 und
- R₁₁: C₈-C₃₀-Alkenyl bedeutet, mit der Bedingung, dass die Verbindung der Formel II eine Gruppe 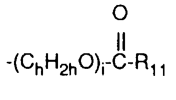 enthält;
in der Verbindung der Formel III
- R₁₂: C₁-C₁₈-Alkyl, C₅-C₁₂-Cycloalkyl, Phenyl oder C₇-C₉-Phenylalkyl bedeutet,
- R₁₅: Wasserstoff, C₁-C₁₈-Alkyl, C₅-C₁₂-Cycloalkyl, Phenyl oder C₇-C₉-Phenylalkyl darstellt,
- s: 0, 1 oder 2 bedeutet,
- Q: 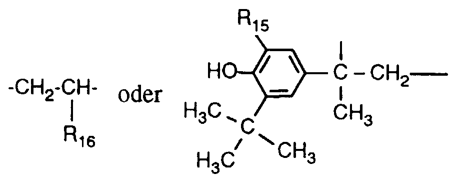 -CₘH₂ₘ-, darstellt, wobei R₁₅ die obige Bedeutung hat,
- m: eine ganze Zahl aus dem Bereich von 0 bis 3 bedeutet,
- R₁₆: C₁-C₈-Alkyl darstellt und
- n: eine ganze Zahl aus dem Bereich von 1 bis 6 bedeutet, wobei
wenn n = 1 ist,
- R₁₇: Wasserstoff, C₁-C₄₅-Alkyl, C₅-C₁₂-Cycloalkyl, C₂-C₁₈-Alkenyl, einen einwertigen Rest einer Hexose, einen einwertigen Rest eines Hexitols, 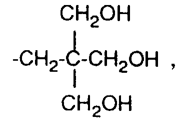 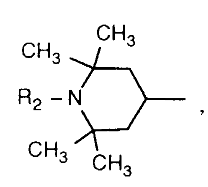 wobei R₂ die obige Bedeutung hat, oder ferner R₁₇ 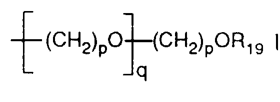 -CH₂CH₂-T₃-R₁₉ oder bedeutet, wobei
- T₃: Sauerstoff, Schwefel oder 〉N-R₂₂ darstellt,
- R₁₉: 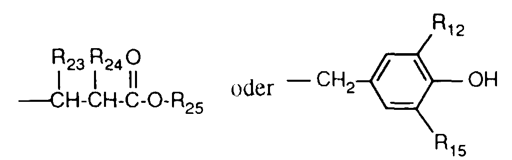 ist, wobei R₁₂ und R₁₅ die obige Bedeutung haben, oder R₁₉ ferner Wasserstoff, C₁-C₂₄-Alkyl, Phenyl, C₅-C₁₂-Cycloalkyl oder 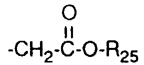 bedeutet, wobei
- p: eine ganze Zahl aus dem Bereich von 2 bis 4 darstellt,
- q: eine ganze Zahl aus dem Bereich von 2 bis 20 bedeutet,
- R₂₂: C₁-C₁₈-Alkyl, Phenyl oder durch 1 bis 3 Reste A₁ substituiertes Phenyl bedeutet, wobei die Reste A₁ unabhängig voneinander C₁-C₁₂-Alkyl, Halogen, Hydroxy, Methoxy oder Ethoxy bedeuten, oder R₂₂ ferner C₅-C₈-Cycloalkyl darstellt,
- R₂₃, R₂₄: unabhängig voneinander Wasserstoff oder Methyl bedeuten mit der Massgabe, dass R₂₃ und R₂₄ nicht gleichzeitig Methyl sind;
- R₂₅: Wasserstoff oder C₁-C₂₄-Alkyl bedeutet, oder
wenn n = 2 ist,
- R₁₇: einen zweiwertigen Rest einer Hexose, einen zweiwertigen Rest eines Hexitols, 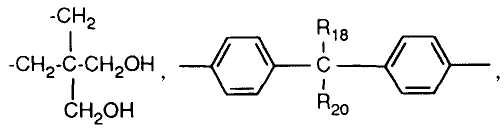 -CᵣH₂ᵣ-, 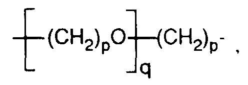 wobei p und q die obige Bedeutung haben, -CH₂CH₂-T₄-CH₂CH₂-, -CH₂-CH=CH-CH₂-,-CH₂-C≡C-CH₂-, 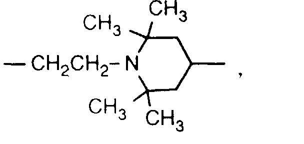 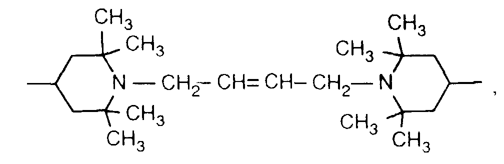 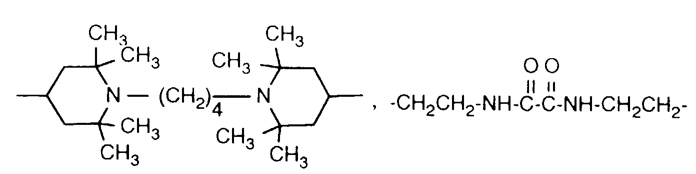 oder 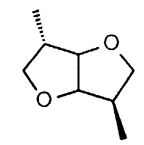 darstellt, wobei
- R₁₈, R₂₀: unabhängig voneinander Wasserstoff oder C₁-C₁₂-Alkyl bedeuten oder zusammen den Rest -CH₂CH₂CH₂CH₂CH₂- bilden,
- r: eine ganze Zahl aus dem Bereich von 2 bis 10 darstellt,
- T₄: Schwefel, 〉N-R₂₆ oder 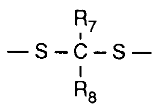 bedeutet, wobei R₇ und R₈ die obige Bedeutung haben, und
- R₂₆: Wasserstoff, C₁-C₁₈-Alkyl, Phenyl oder durch 1 bis 3 Reste A₁ substituiertes Phenyl, wobei die Reste A₁ die obige für die Formel I beschriebene Bedeutung haben, oder R₂₆ ferner C₅-C₈-Cycloalkyl oder 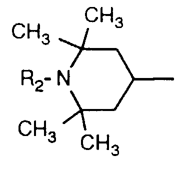 bedeutet, wobei R₂ die obige Bedeutung hat, oder
wenn n = 3 ist,
- R₁₇: einen dreiwertigen Rest einer Hexose, einen dreiwertigen Rest eines Hexitols, 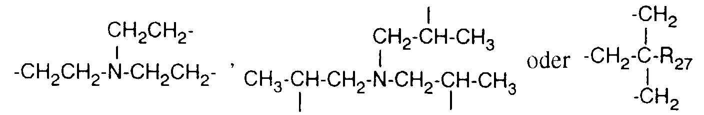 darstellt, wobei
- R₂₇: Wasserstoff, -CH₂OH, C₁-C₄-Alkyl, C₁-C₁₈-Alkylamido oder 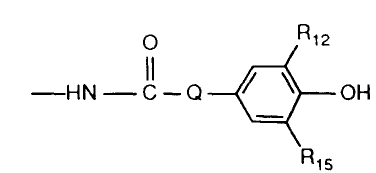 bedeutet, wobei Q, R₁₂ und R₁₅ die obige Bedeutung haben, oder
wenn n = 4 ist,
- R₁₇: einen vierwertigen Rest einer Hexose, einen vierwertigen Rest eines Hexitols, C₄-C₁₀-Alkantetrayl, 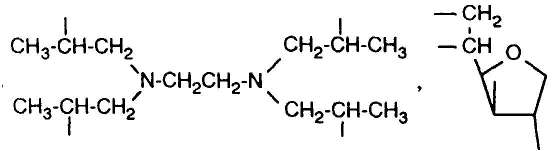 oder 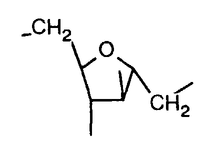 darstellt, oder
wenn n = 5 ist,
- R₁₇: einen fünfwertigen Rest einer Hexose oder einen fünfwertigen Rest eines Hexitols darstellt, oder
wenn n = 6 ist,
- R₁₇: einen sechswertigen Rest eines Hexitols oder 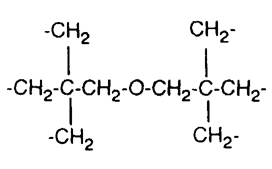 bedeutet,
in der Verbindung der Formel IV
- D: Schwefel, R₅₀-SH, 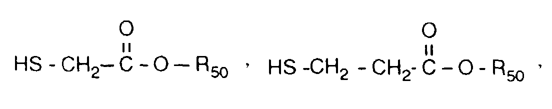 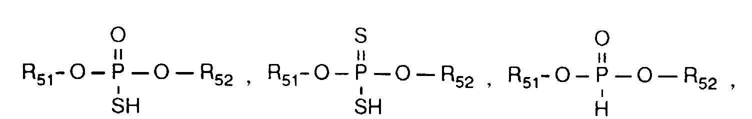 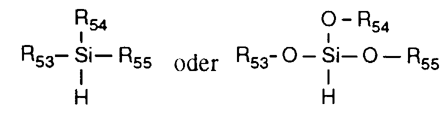 darstellt,
- R₅₀: C₁-C₁₈-Alkyl, unsubstituiertes oder durch C₁-C₄-Alkyl substituiertes C₅-C₁₂-Cycloalkyl bedeutet,
- R₅₁ und R₅₂: unabhängig voneinander C₁-C₁₈-Alkyl, unsubstituiertes oder durch C₁-C₁₈-Alkyl substituiertes Phenyl darstellt, und
- R₅₃, R₅₄ und R₅₅: unabhängig voneinander C₁-C₁₈-Alkyl oder Phenyl bedeutet.

Die flüssigen und schwerflüchtigen Produkte gemäss vorliegender Erfindung zeichnen sich durch eine sehr gute Stabilisierung von organischen Materialien, wie z.B. Polymeren oder Oelen sowohl gegen oxidativen, thermischen als auch lichtinduzierten Abbau aus.

Alkyl mit bis zu 45 C-Atomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl oder Pentacosyl. Eine der bevorzugten Bedeutungen von R₁, R₄ und R₁₆ ist beispielweise C₁-C₄-Alkyl, von R₂ Methyl, von R₁₁ C₁-C₂₀-Alkyl, von R₁₂ und R₁₅ C₁-C₄-Alkyl, insbesondere tert-Butyl, und von R₁₇ C₁-C₁₈-Alkyl; von R₅₁ und R₅₂ C₃-C₈-Alkyl.

Cycloalkyl mit bis zu 12 C-Atomen bedeutet beispielsweise Cyclopentyl, Cyclohexyl, Cycloheptyl, Cyclooctyl, Cyclodecyl oder Cyclododecyl. Eine der bevorzugten Bedeutungen von R₁, R₁₁, R₁₂ und R₁₅ ist C₅-C₇-Cycloalkyl. Besonders bevorzugt ist Cyclohexyl.

Unsubstituiertes oder durch C₁-C₄-Alkyl substituiertes C₅-C₁₂-Cycloalkyl bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist C₅-C₈-Cycloalkyl, insbesondere Cyclohexyl und tert-Butylcyclohexyl.

Alkenyl mit bis zu 30 C-Atomen bedeutet beispielsweise Vinyl, Propenyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, Oleyl, n-Octadec-2-enyl oder n-Octadec-4-enyl. Bedeuten R₁, R₂ und X C₃-C₆-Alkenyl, so ist das C-Atom, welches an den Stickstoff gebunden ist, zweckmässigerweise gesättigt.

Phenylalkyl mit 7 bis 9 C-Atomen bedeutet beispielsweise Benzyl, α-Methylbenzyl, α,α-Dimethylbenzyl oder 2-Phenylethyl. Benzyl ist bevorzugt.

Beispiele für Phenyl, welches durch 1 bis 3 Reste A₁ substituiert ist, sind o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 2-Methyl-4-tert-butylphenyl, 2-Ethylphenyl, 2,6-Diethylphenyl, 2,6-Diethyl-4-methylphenyl, 2,6-Diisopropylphenyl, 4-tert-Butylphenyl, p-Nonylphenyl, o-, m- oder p-Chlorphenyl, 2,3-Dichlorphenyl, 2,4-Dichlorphenyl, 2,5-Dichlorphenyl, 2,6-Dichlorphenyl, 3,4-Dichlorphenyl, 2,4,5-Trichlorphenyl, 2,4,6-Trichlorphenyl, o-, m- oder p-Hydroxyphenyl, o-, m- oder p-Methoxyphenyl, o- oder p-Ethoxyphenyl, 2,4-Dimethoxyphenyl, 2,5-Di-methoxyphenyl, 2,5-Diethoxyphenyl, o-, m- oder p-Methoxycarbonyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, 2-Hydroxy-4-methylphenyl, 3-Hydroxy-4-methylphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-dimethoxyphenyl und 4-Chlor-2,5-dimethoxyphenyl. Bevorzugt ist dabei mit 1 oder 2, insbesondere 1 Rest(en) A₁ substituiertes Phenyl, wobei A₁ insbesondere Alkyl ist.

Durch C₁-C₁₈-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-Butylphenyl, 2-Ethylphenyl, 2,6-Diethylphenyl, Butylphenyl, Pentylphenyl, Hexylphenyl, Heptylphenyl, Oktylphenyl, Nonylphenyl, Decylphenyl, Undecylphenyl oder Dodecylphenyl. Bevorzugt ist C₁-C₁₂-Alkyl substituiertes Phenyl, insbesondere C₄-C₈-Alkyl substituiertes Phenyl.

Ein C₅-C₁₂-Cycloalkylidenring bedeutet beispielsweise Cyclopentyliden, Cyclohexyliden, Cycloheptyliden, Cyclooktyliden oder Cyclononyliden. Bevorzugt ist Cyclohexyliden.

Alkoxy mit 1 bis 18 C-Atomen bedeutet beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Eine der bevorzugten Bedeutungen von R₂ ist C₆-C₁₂-Alkoxy. Besonders bevorzugt sind Heptoxy und Octoxy.

Cycloalkoxy mit 5 bis 12 C-Atomen bedeutet beispielsweise Cyclopentoxy, Cyclohexoxy, Cycloheptoxy, Cyclooctoxy, Cyclodecyloxy oder Cyclododecyloxy. Eine der bevorzugten Bedeutungen von R₂ ist C₅-C₈-Cycloalkoxy. Besonders bevorzugt sind Cyclopentoxy und Cyclohexoxy.

Beispiele für C₇-C₉-Phenylalkyl, welches am Phenylring mit C₁-C₄-Alkyl mono-, di- oder tri-substituiert ist, sind Methylbenzyl, Dimethylbenzyl, Trimethylbenzyl oder tert-Butylbenzyl.

Acyl mit 1 bis 8 C-Atomen bedeutet beispielsweise Formyl, Acetyl, Propionyl, Butyryl, Pentanoyl, Hexanoyl, Heptanoyl, Octanoyl, Benzoyl, Acryloyl oder Crotonyl. Bevorzugt sind C₁-C₈-Alkanoyl, C₃-C₈-Alkenoyl oder Benzoyl, insbesondere Acetyl.

Alkantriyl mit 3 bis 10 C-Atomen bedeutet beispielsweise 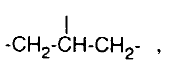 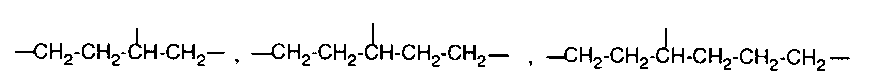 oder 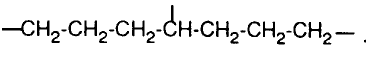 Bevorzugt ist Glyceryl.

Alkantetrayl mit 4 bis 10 C-Atomen bedeutet beispielsweise 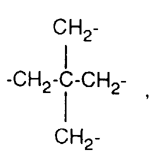 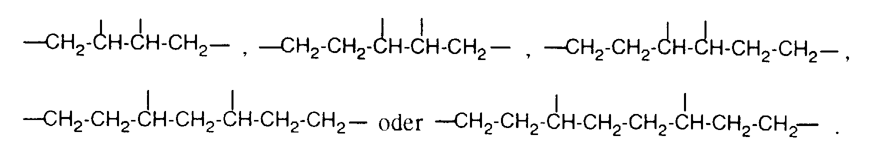 Bevorzugt ist Pentaerythrityl.

Alkanhexayl mit 6 bis 10 C-Atomen bedeutet beispielsweise 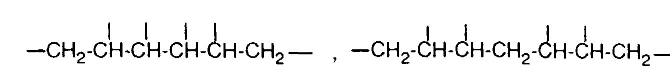 oder

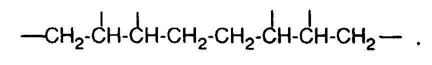

Bedeutet R₁₇ für n = 1 bis 6 einen n-wertigen Rest einer Hexose, so leitet sich dieser z.B. von Allose, Altrose, Glucose, Mannose, Gulose, Idose, Galaktose oder Talose ab, d.h. um die entsprechenden Verbindungen der Formel III zu erhalten, müssen eine, zwei, drei, vier, fünf oder sechs -OH Gruppen durch die Estergruppe E-1, 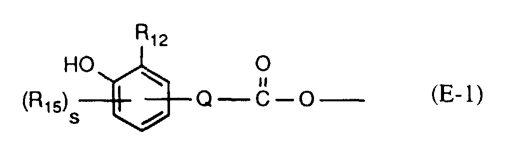 worin R₁₂, R₁₅, s und Q die oben angegebenen Bedeutungen haben, ersetzt werden. So kann R₁₇ z.B. für n = 5 eine Gruppe 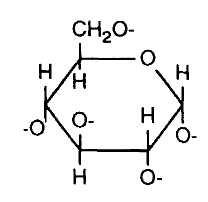 bedeuten.

Stellt R₁₇ den n-wertigen Rest eines Hexitols dar, so erhält man die entsprechenden Verbindungen der Formel III, indem n -OH Gruppen durch die oben angegebene Estergruppe E-1 ersetzt werden. R₁₇ kann als sechswertiger Rest eines Hexitols z.B. 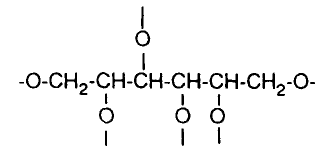 sein. Diese Gruppe leitet sich von dem D-Sorbitol ab.

Alkylamido mit 1 bis 18 C-Atomen bedeutet beispielsweise CH₃-CO-NH-, CH₃CH₂-CO-NH-, C₆H₁₃-CO-NH- oder C₁₈H₃₇-CO-NH-. 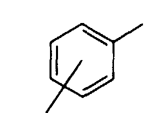 bedeutet, dass der Phenylring ortho, meta oder para substituiert sein kann.

Die Umsetzung der vier Komponenten a), b), c) und d) miteinander zu den Produkten der vorliegenden Erfindung kann in einer beliebigen Reihenfolge erfolgen.

Bevorzugt wird zuerst Komponente a) mit Komponente b) umgesetzt und anschliessend wird Komponente c) und dann die Komponente d) zugegeben.

Zweckmässigerweise wird die Reaktion in Gegenwart eines Katalysators oder Radikalstarters durchgeführt. Als Katalysatoren kommen insbesondere Lewis-Säuren oder Basen in Frage.

Geeignete basische Katalysatoren sind beispielsweise Metallhydride, Metallalkylide, Metallarylide, Metallhydroxide, Metallalkoholate, Metallphenolate, Metallamide oder Metallcarboxylate.

Bevorzugte Metallhydride sind beispielsweise Lithiumhydrid, Natriumhydrid oder Kaliumhydrid.

Bevorzugte Metallalkylide sind beispielsweise Butyllithium oder Methyllithium.

Ein bevorzugtes Metallarylid ist beispielsweise Phenyllithium.

Bevorzugte Metallhydroxide sind beispielsweise Lithiumhydroxid, Natriumhydroxid, Kaliumhydroxid, Cäsiumhydroxid, Rubidiumhydroxid, Magnesiumhydroxid, Calciumhydroxid, Bariumhydroxid oder Aluminiumhydroxid.

Bevorzugte Metallalkoholate sind beispielsweise Natriummethanolat, Natriumethanolat, Kaliummethanolat, Kaliumethanolat, Natriumisopropylat oder Kalium tert-butylat.

Bevorzugte Metallphenolate sind beispielweise Natriumphenolat oder Kaliumphenolat.

Bevorzugte Metallamide sind beispielsweise Natriumamid oder Lithiumamid.

Ein bevorzugtes Metallcarboxylat ist beispielsweise Calciumacetat.

Geeignete Lewis-Säuren-Katalysatoren sind beispielsweise 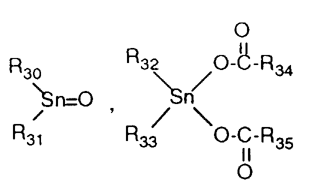 oder 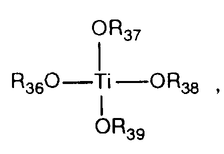 wobei die Reste R₃₀, R₃₁, R₃₂, R₃₃, R₃₄, R₃₅, R₃₆, R₃₇, R₃₈ und R₃₉ z.B. unabhängig voneinander C₁-C₁₈-Alkyl oder Phenyl bedeuten. Bevorzugt ist C₁-C₈-Alkyl. Ein besonders bevorzugter Lewis-Säure-Katalysator ist Dibutylzinnoxid.

Geeignete Radikalstarter sind beispielsweise Azoverbindungen wie z.B. 2,2'-Azoisobuttersäurenitril; Peroxide wie z.B. Benzoylperoxid; oder Hydroperoxide wie z.B. α,α-Dimethyl-benzylhydroperoxid. Radikalstarter werden bevorzugt dann eingesetzt, wenn die Komponente d) als Reaktionspartner beteiligt ist.

Der Katalysator oder Radikalstarter wird zu den Komponenten a), b), c) und d) z.B. in einer Menge von 0,05 bis 10 Gewichtspromille zugegeben, bevorzugt in einer Menge von 0,1 bis 5 Gewichtspromille. Besonders bevorzugt ist die Zugabe von 1 bis 2 Gewichtspromille Dibutylzinnoxid.

Die Umsetzung der Komponenten a), b), c) und d) kann in einem Lösungsmittel, wie beispielsweise Xylol, oder ohne Lösungsmittel durchgeführt werden. Bevorzugt wird die Umsetzung ohne Lösungsmittel durchgeführt.

Die Reaktionstemperatur liegt z.B. zwischen 130 und 250°C. Bevorzugt wird die Umsetzung in einem Temperaturbereich von 130 bis 190°C durchgeführt.

Ein weiterer Anmeldungsgegenstand ist auch ein Verfahren zur Herstellung der erfindungsgemässen Produkte, dadurch gekennzeichnet, dass die Komponenten a), b), c) und d) in einem molaren Mengenverhältnis von 0,1:1:0,1:0,1 bis 15:1:30:10 umgesetzt werden.

Die Komponenten a), b), c) und d) können, sofern sie nicht im Handel erhältlich sind, nach bekannten Verfahren bzw. in Analogie zu solchen hergestellt werden. Mögliche Herstellungsverfahren für die Verbindungen der Formel III sind z.B. in folgenden Veröffentlichungen zu finden: GB-A-996 502, US-A-3 330 859, US-A-3 944 594, US-A-4 593 057, EP-A-154 518 oder US-A-3 960 928.

Ein bevorzugter Gegenstand der Erfindung sind Produkte, wobei in der Verbindung der Formel III s die Zahl 1 oder 2 bedeutet.

Ein ebenfalls bevorzugter Gegenstand der Erfindung sind Produkte,
wobei in der Verbindung der Formel I die Reste
- Y: unabhängig voneinander OH, (HOCH₂CH₂)₂N- oder -HNR₁ bedeutet und
- R₁: Wasserstoff, C₁-C₁₀-Alkyl, C₅-C₇-Cycloalkyl, 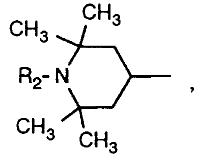 C₃-C₆-Alkenyl, Benzyl oder Phenyl bedeutet, wobei
- R₂: Wasserstoff, C₁-C₄-Alkyl, OH, -CH₂CN, C₆-C₁₂-Alkoxy, C₅-C₈-Cycloalkoxy, Allyl, Benzyl, Acetyl oder HOCH₂CH₂- darstellt, und
- a: die Zahl 1,2,3,4 oder 6 bedeutet, wobei
wenn Y = OH und a = 1 ist,
- X: C₁-C₃₀-Alkyl, C₃-C₁₈-Alkenyl, -CH₂CH₂T₁(CH₂CH₂O)_{b}R₄ oder 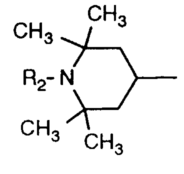 bedeutet, wobei R₂ die obige Bedeutung hat, und
- T₁: Sauerstoff, Schwefel oder 〉N-R₅,
- R₄: C₁-C₁₀-Alkyl,
- b: eine ganze Zahl aus dem Bereich von 0 bis 10 und
- R₅: Wasserstoff, C₁-C₁₀-Alkyl oder Phenyl darstellen, oder
wenn Y = OH und a = 2 ist,
- X: -CH₂CH₂T₂(CH₂CH₂O)_{b}CH₂CH₂-, wobei b die obige Bedeutung hat, 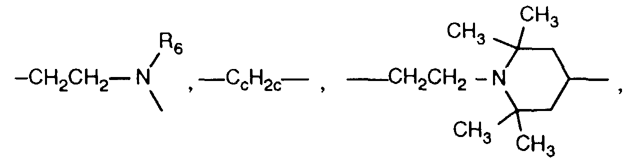 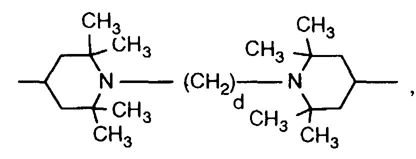 ―CH₂―CH=CH―CH₂― 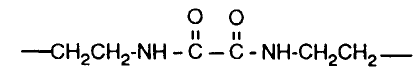 oder 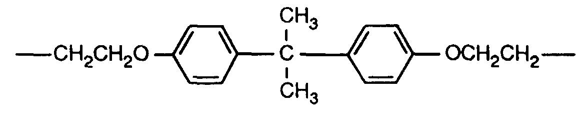 bedeutet, wobei
- T₂: Sauerstoff, Schwefel, 〉N-R₅ oder 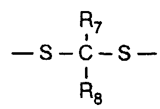 ist und R₅ die obige Bedeutung hat,
- R₆: Wasserstoff, C₁-C₁₀-Alkyl oder Phenyl,
- c: eine ganze Zahl aus dem Bereich von 2 bis 10,
- d: eine ganze Zahl aus dem Bereich von 2 bis 6 und
- R₇, R₈: unabhängig voneinander Wasserstoff, C₁-C₁₀-Alkyl oder Phenyl sind, oder R₇ und R₈ mit dem C-Atom, an das sie gebunden sind, einen C₅-C₇-Cycloalkyl-Ring bilden, oder
wenn Y = -HNR₁ und a = 1 ist,
- X: C₁-C₁₀-Alkyl, C₃-C₁₈-Alkenyl, C₅-C₇-Cycloalkyl, Benzyl, Phenyl, 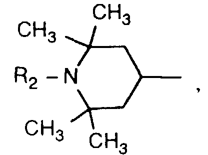 wobei R₂ die obige Bedeutung hat, oder X ferner 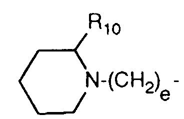 ist, oder X mit R₁ gemeinsam eine Gruppe der Formel -CH₂CH₂CH₂CH₂CH₂- oder -CH₂CH₂OCH₂CH₂- bildet, wobei
- R₁₀: Wasserstoff oder Methyl ist, und
- e: 2 oder 3 bedeutet, und
in der Verbindung der Formel II die Reste
- Z: Wasserstoff oder eine Gruppe der Formel 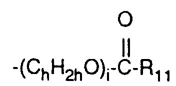 bedeuten, und
- k: eine ganze Zahl aus dem Bereich von 0 bis 4 darstellt, wobei
- h: 2 oder 3,
- i: eine ganze Zahl aus dem Bereich von 0 bis 6 und
- R₁₁: C₈-C₂₀-Alkenyl bedeutet, mit der Bedingung, dass die Verbindung der Formel II eine Gruppe 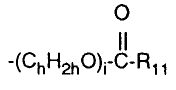 enthält;
in der Verbindung der Formel III
- R₁₂: C₁-C₆-Alkyl, C₅-C₇-Cycloalkyl, Phenyl oder Benzyl bedeutet,
- R₁₅: Wasserstoff, C₁-C₆-Alkyl, C₅-C₇-Cycloalkyl, Phenyl oder Benzyl darstellt,
- s: 1 oder 2 bedeutet,
- Q: -CₘH₂ₘ-, 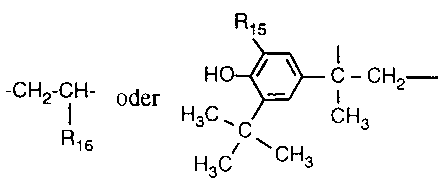 darstellt, wobei R₁₅ die obige Bedeutung hat,
- m: eine ganze Zahl aus dem Bereich von 0 bis 3 bedeutet,
- R₁₆: C₁-C₄-Alkyl darstellt und
- n: eine ganze Zahl aus dem Bereich von 1 bis 6 bedeutet, wobei
wenn n = 1 ist,
- R₁₇: Wasserstoff, C₁-C₃₀-Alkyl, C₅-C₇-Cycloalkyl, C₂-C₁₈-Alkenyl, einen einwertigen Rest einer Hexose, einen einwertigen Rest eines Hexitols, 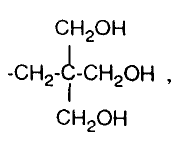 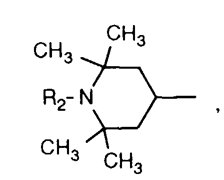 wobei R₂ die obige Bedeutung hat, oder ferner R₁₇ -CH₂CH₂-T₃-R₁₉ oder 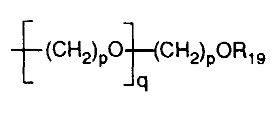 bedeutet, wobei
- T₃: Sauerstoff, Schwefel oder 〉N-R₂₂ darstellt,
- R₁₉: 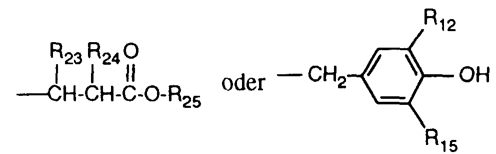 ist, wobei R₁₂ und R₁₅ die obige Bedeutung haben, oder R₁₉ ferner Wasserstoff, C₁-C₁₈-Alkyl, Phenyl, C₅-C₇-Cycloalkyl oder 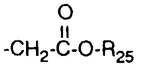 bedeutet, wobei
- p: eine ganze Zahl aus dem Bereich von 2 bis 4 darstellt,
- q: eine ganze Zahl aus dem Bereich von 2 bis 20 bedeutet,
- R₂₂: C₁-C₁₀-Alkyl, Phenyl oder C₅-C₈-Cycloalkyl darstellt,
- R₂₃, R₂₄: unabhängig voneinander Wasserstoff oder Methyl bedeuten, mit der Massgabe, dass R₂₃ und R₂₄ nicht gleichzeitig Methyl sind;
- R₂₅: Wasserstoff oder C₁-C₁₈-Alkyl bedeutet, oder
wenn n = 2 ist,
- R₁₇: einen zweiwertigen Rest einer Hexose, einen zweiwertigen Rest eines Hexitols, 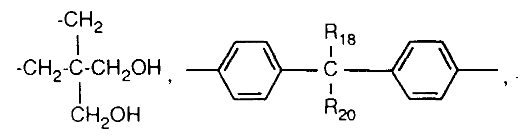 -CᵣH₂ᵣ-, 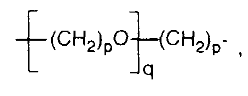 wobei p und q die obige Bedeutung haben, -CH₂CH₂-T₄-CH₂CH₂-, -CH₂-CH=CH-CH₂-, -CH₂-C≡C-CH₂- , 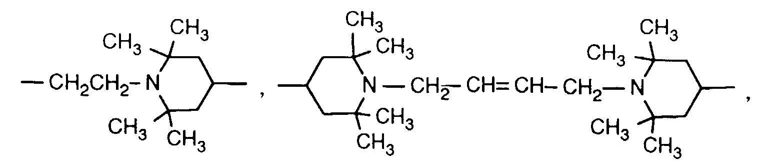 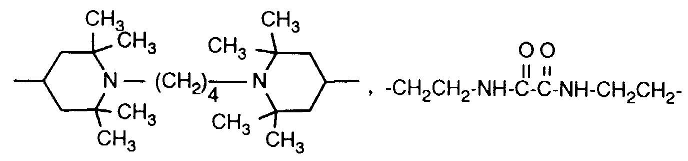 oder 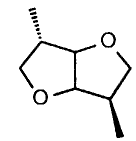 darstellt, wobei
- R₁₈, R₂₀: unabhängig voneinander Wasserstoff oder C₁-C₆-Alkyl bedeuten oder zusammen den Rest -CH₂CH₂CH₂CH₂CH₂- bilden,
- r: eine ganze Zahl aus dem Bereich von 2 bis 10 darstellt,
- T₄: Schwefel, 〉N-R₂₆ oder 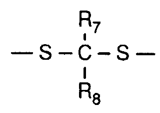 bedeutet, wobei R₇ und R₈ die obige Bedeutung haben und
- R₂₆: Wasserstoff, C₁-C₁₀-Alkyl, Phenyl, C₅-C₈-Cycloalkyl oder 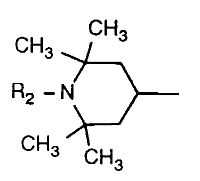 bedeutet, wobei R₂ die obige Bedeutung hat,
in der Verbindung der Formel IV
- D: Schwefel, R₅₀-SH, 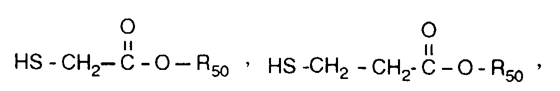 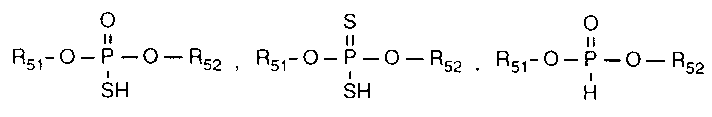 oder 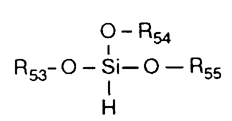 darstellt,
- R₅₀: C₁-C₁₈-Alkyl oder C₅-C₁₂-Cycloalkyl bedeutet,
- R₅₁ und R₅₂: unabhängig voneinander C₁-C₁₂-Alkyl, unsubstituiertes oder durch C₁-C₁₂-Alkyl substituiertes Phenyl darstellt, und
- R₅₃, R₅₄ und R₅₅: unabhängig voneinander C₁-C₁₂-Alkyl oder Phenyl bedeutet.

Ein besonders bevorzugter Gegenstand der Erfindung sind Produkte,
wobei in der Verbindung der Formel I die Reste
- Y: unabhängig voneinander OH, (HOCH₂CH₂)₂N- oder -HNR₁ darstellt und
- R₁: Wasserstoff, C₁-C₄-Alkyl oder 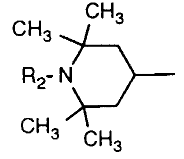 bedeutet, wobei
- R₂: Wasserstoff, C₁-C₄-Alkyl, OH, Allyl, Benzyl, Acetyl oder HOCH₂CH₂- darstellt, und
- a: die Zahl 1,2,3,4 oder 6 bedeutet, wobei
wenn Y = OH und a = 1 ist,
- X: C₁-C₁₈-Alkyl, C₃-C₁₈-Alkenyl, -CH₂CH₂T₁(CH₂CH₂O)_{b}R₄ oder 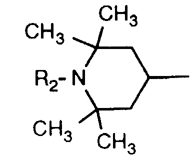 bedeutet, wobei R₂ die obige Bedeutung hat, und
- T₁: Sauerstoff,
- R₄: C₁-C₄-Alkyl und
- b: eine ganze Zahl aus dem Bereich von 0 bis 10 darstellt, oder
wenn Y = OH und a = 2 ist,
- X: -CH₂CH₂T₂(CH₂CH₂O)_{b}CH₂CH₂-, wobei b die obige Bedeutung hat, oder ferner X ―C_{c}H_{2c}―, 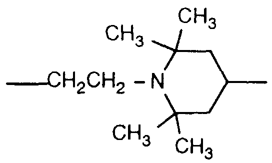 oder ―CH₂-CH=CH-CH₂― bedeutet, wobei
- T₂: Sauerstoff, Schwefel oder 〉N-R₅,
- R₅: Wasserstoff,
- b: die Zahl 0 oder 1, und
- c: eine ganze Zahl aus dem Bereich von 2 bis 8 darstellen, oder
wenn a = 3 ist,
- X: 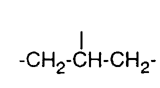 oder N(CH₂CH₂-)₃ bedeutet, oder
wenn Y = OH und a = 4 ist,
- X: 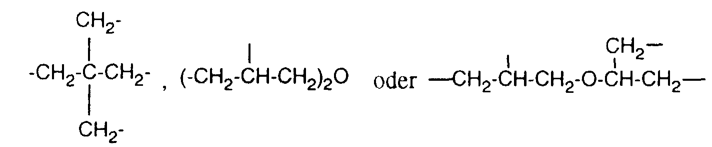 darstellt, oder
wenn Y = OH und a = 6 ist,
- X: 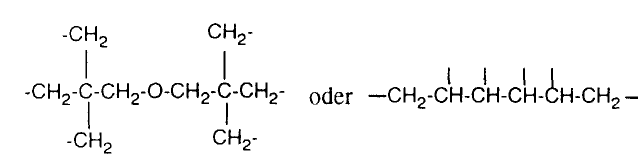 darstellt, oder
wenn Y = -HNR₁ und a = 1 ist,
- X: C₁-C₁₀-Alkyl, C₃-C₁₈-Alkenyl, C₅-C₇-Cycloalkyl oder 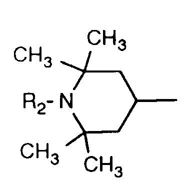 bedeutet, wobei R₂ die obige Bedeutung hat, oder
wenn Y = -HNR₁ und a = 2 ist,
- X: -C_{f}H_{2f}- darstellt, wobei
- f: eine ganze Zahl aus dem Bereich von 2 bis 10 bedeutet, und
in der Verbindung der Formel II die Reste
- Z: Wasserstoff oder eine Gruppe der Formel 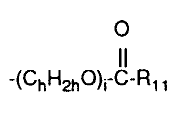 bedeutet, und
- k: 1, 2 oder 3 darstellt,
- h: 2 oder 3 bedeutet,
- i: eine ganze Zahl aus dem Bereich von 0 bis 4 ist und
- R₁₁: C₈-C₂₀-Alkenyl bedeutet, mit der Bedingung, dass die Verbindung der Formel II eine Gruppe 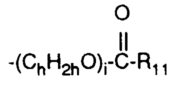 enthält;
in der Verbindung der Formel III
- R₁₂: C₁-C₆-Alkyl oder C₅-C₇-Cycloalkyl bedeutet,
- R₁₅: Wasserstoff, C₁-C₆-Alkyl oder C₅-C₇-Cycloalkyl darstellt,
- s: 1 oder 2 bedeutet,
- Q: -CₘH₂ₘ- oder 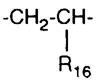 darstellt,
- m: eine ganze Zahl aus dem Bereich von 0 bis 3 bedeutet,
- R₁₆: C₁-C₄-Alkyl darstellt und
- n: eine ganze Zahl aus dem Bereich von 1 bis 6 bedeutet, wobei
wenn n = 1 ist,
- R₁₇: Wasserstoff, C₁-C₁₈-Alkyl, C₅-C₇-Cycloalkyl, C₂-C₁₈-Alkenyl, einen einwertigen Rest einer Hexose, einen einwertigen Rest eines Hexitols, 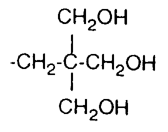 oder 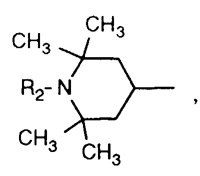 wobei R₂ die obige Bedeutung hat, oder ferner R₁₇ 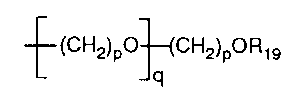 bedeutet, wobei
- R₁₉: Wasserstoff, C₁-C₁₈-Alkyl oder C₅-C₇-Cycloalkyl bedeutet, wobei
- p: eine ganze Zahl aus dem Bereich von 2 bis 4 darstellt,
- q: eine ganze Zahl aus dem Bereich von 2 bis 10 bedeutet, oder
wenn n = 2 ist,
- R₁₇: einen zweiwertigen Rest einer Hexose, einen zweiwertigen Rest eines Hexitols, 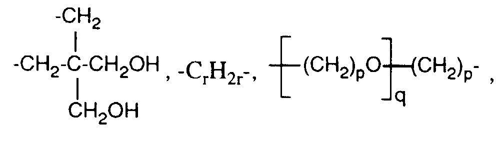 wobei p und q die obige Bedeutung haben, -CH₂CH₂-T₄-CH₂CH₂- oder 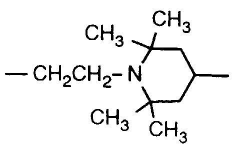 darstellt, wobei
- r: eine ganze Zahl aus dem Bereich von 2 bis 10 darstellt,
- T₄: Schwefel oder 〉N-R₂₆ bedeutet und
- R₂₆: Wasserstoff, C₁-C₁₀-Alkyl oder C₅-C₈-Cycloalkyl bedeutet, oder
wenn n = 3 ist,
- R₁₇: einen dreiwertigen Rest einer Hexose, einen dreiwertigen Rest eines Hexitols, 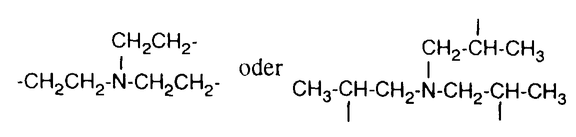 darstellt, oder
wenn n = 4 ist,
- R₁₇: einen vierwertigen Rest einer Hexose, einen vierwertigen Rest eines Hexitols, 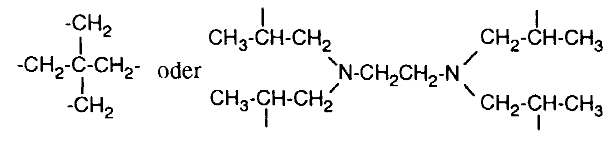 darstellt,
in der Verbindung der Formel IV
- D: Schwefel, 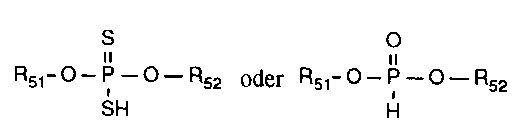 bedeutet, und
- R₅₁ und R₅₂: unabhängig voneinander C₁-C₁₂-Alkyl, unsubstituiertes oder durch C₁-C₈-Alkyl substituiertes Phenyl darstellt.

Einen bevorzugten Gegenstand der Erfindung bilden weiterhin Produkte,
wobei in der Verbindung der Formel I die Reste
- Y: unabhängig voneinander Hydroxy oder -NH₂ bedeutet und
- a: eine ganze Zahl aus dem Bereich von 1 bis 4 darstellt, wobei
wenn a = 1 ist,
- X =: 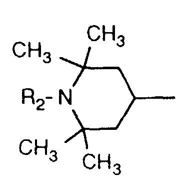 bedeutet, und
- R₂: Wasserstoff, Methyl oder HOCH₂CH₂- darstellt, oder
wenn Y = OH und a = 2 ist,
- X: -CH₂CH₂T₂(CH₂CH₂O)_{b}CH₂CH₂-, ―C_{c}H_{2c}― oder 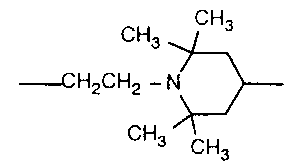 bedeutet, wobei
- T₂: Sauerstoff, Schwefel oder 〉N-R₅,
- R₅: Wasserstoff,
- b: die Zahl 0 oder 1 und
- c: die Zahl 2, 3 oder 4 darstellen, oder
wenn Y = OH und a = 3 ist,
- X: 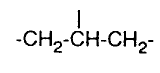 bedeutet, oder
wenn Y = OH und a = 4 ist,
- X: 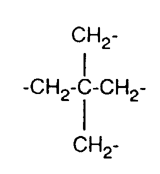 darstellt, und
in der Verbindung der Formel II die Reste
- Z: Wasserstoff oder eine Gruppe der Formel 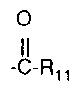 bedeuten,
- k: die Zahl 1 darstellt, und
- R₁₁: C₈-C₂₀-Alkenyl bedeutet, mit der Bedingung, dass die Verbindung der Formel II eine Gruppe 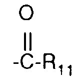 enthält, und
in der Verbindung der Formel III
- R₁₂: tert-Butyl bedeutet,
- R₁₅: C₁-C₄-Alkyl darstellt und in ortho Stellung zur OH-Gruppe gebunden ist,
- s: die Zahl 1 bedeutet,
- Q: -CₘH₂ₘ- darstellt und para zur OH-Gruppe gebunden ist, wobei
- m: die Zahl 2 bedeutet,
- n: 1 darstellt, und
- R₁₇: C₁-C₄-Alkyl bedeutet,
in der Verbindung der Formel IV
- D: Schwefel oder 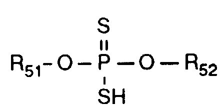 darstellt, und
- R₅₁ und R₅₂: unabhängig voneinander C₃-C₈-Alkyl bedeutet.

Von Interesse sind Produkte, wobei in der Verbindung der Formel IV
- D: Schwefel oder 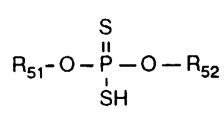 darstellt, und
- R₅₁ und R₅₂: unabhängig voneinander C₁-C₁₂-Alkyl, unsubstituiertes oder durch C₁-C₈-Alkyl substituiertes Phenyl bedeutet.

Bevorzugte Verbindungen der Formel I sind beispielsweise Pentaerythrit, Thiodiethylenglykol, 1,4-Butandiol, 1,2-Propandiol, Diethylenglykol, Triethylenglykol, Diethanolamin, Glycerin,

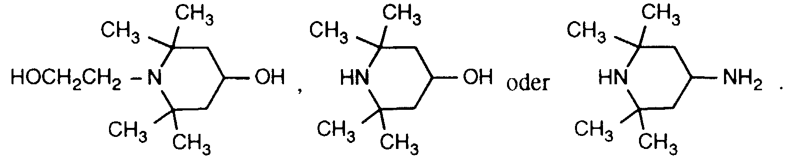

Besonders bevorzugt ist Glycerin oder Thiodiethylenglykol.

Bevorzugte Verbindungen der Formel II sind in der Natur vorkommende pflanzliche Oele, Fette und Wachse, tierische Oele und Fette sowie künstlich erzeugte Polyol-Derivate.

Bevorzugte pflanzliche Oele, Fette und Wachse sind beispielsweise Sonnenblumenöl, Kokosfett, Rapsöl, Sojabohnenöl, Maiskeimöl, Distelöl, Olivenöl, Erdnussöl, Baumwollsamenöl, Sesamöl, Safloröl, Rizinusöl, Talgöl, Kürbiskernenöl oder Leinsamenöl.

Bevorzugte tierische Oele und Fette sind beispielsweise Butterfett, Schweinefett, Fischöl, Spermöl, Klauenöl oder Trane.

Bevorzugte künstlich erzeugte Polyol-Derivate sind beispielsweise Radiamuls (Glycerin Tri C₈/C₁₀) oder Sorbitan-Derivate. Die Sorbitan-Derivate sind z.B. unter den Namen Span®20, Span®40, Span®60, Span®65, Span®80, Span®85, Tween 20®, Tween 40®, Tween 60®, Tween 65®, Tween 80® oder Tween 85® im Handel erhältlich.

Besonders bevorzugt ist Sonnenblumenöl, Kokosfett oder Rapsöl.

Einen bevorzugten Gegenstand der Erfindung bilden ebenfalls Produkte,
wobei in der Verbindung der Formel III
- R₁₂: C₁-C₄-Alkyl oder Cyclohexyl bedeutet,
- R₁₅: C₁-C₄-Alkyl oder Cyclohexyl darstellt und in ortho Stellung zur OH-Gruppe gebunden ist,
- s: die Zahl 1 bedeutet,
- Q: -CₘH₂ₘ- darstellt und para zur OH-Gruppe gebunden ist, wobei
- m: eine ganze Zahl aus dem Bereich von 0 bis 3, und
- n: eine ganze Zahl aus dem Bereich von 1 bis 4 bedeuten, wobei
wenn n = 1 ist,
- R₁₇: Wasserstoff, C₁-C₁₀-Alkyl, Cyclohexyl, C₂-C₁₈-Alkenyl oder 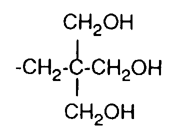 darstellt, oder
wenn n = 2 ist,
- R₁₇: 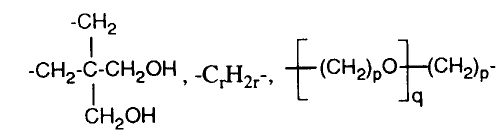 oder -CH₂CH₂-T₄-CH₂CH₂- bedeutet, wobei
- p: eine ganze Zahl aus dem Bereich von 2 bis 4 darstellt,
- q: eine ganze Zahl aus dem bereich von 2 bis 10 bedeutet,
- r: eine ganze Zahl aus dem Bereich von 2 bis 6 darstellt,
- T₄: Schwefel oder 〉N-R₂₆ bedeutet und
- R₂₆: Wasserstoff oder C₁-C₄-Alkyl bedeutet, oder
wenn n = 3 ist,
- R₁₇: 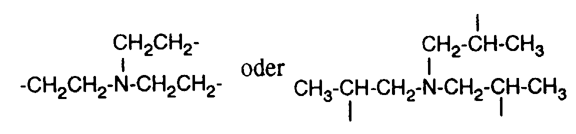 darstellt, oder
wenn n = 4 ist,
- R₁₇: 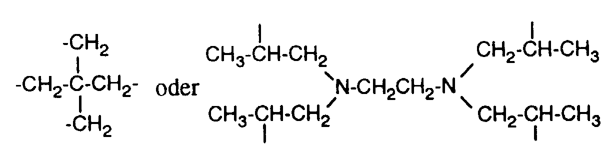 darstellt.

Ein besonders bevorzugter Gegenstand der Erfindung sind ferner Produkte,
wobei in der Verbindung der Formel III
- R₁₂: tert-Butyl bedeutet,
- R₁₅: C₁-C₄-Alkyl darstellt und in ortho Stellung zur OH-Gruppe gebunden ist,
- s: die Zahl 1 bedeutet,
- Q: -CₘH₂ₘ- darstellt und para zur OH-Gruppe gebunden ist, wobei
- m: die Zahl 2 bedeutet und
- n: eine ganze Zahl 1,2 oder 4 bedeutet, wobei
wenn n = 1 ist,
- R₁₇: C₁-C₄-Alkyl bedeutet, oder
wenn n = 2 ist,
- R₁₇: 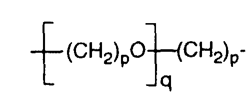 oder -CH₂CH₂-T₄-CH₂CH₂- bedeutet, wobei
- p: die Zahl 2,
- q: die Zahl 2 und
- T₄: Schwefel bedeuten, oder
wenn n = 4 ist,
- R₁₇: 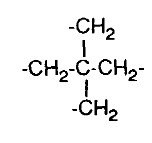 darstellt.

Bevorzugte Verbindungen der Formel III sind beispielsweise auch

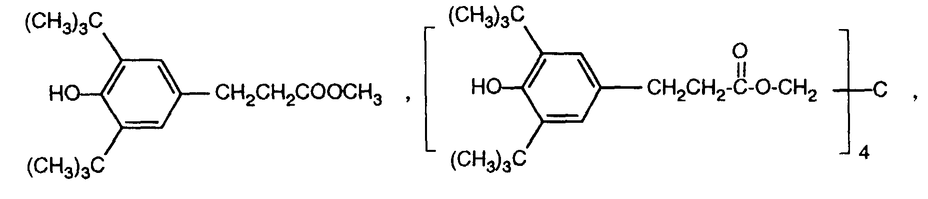

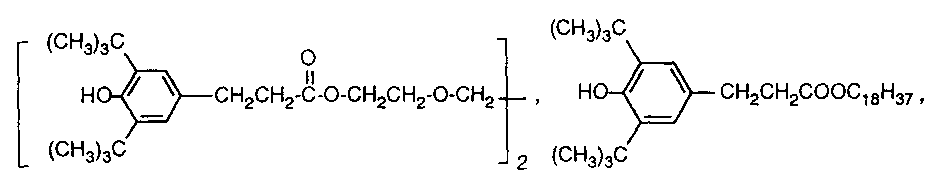

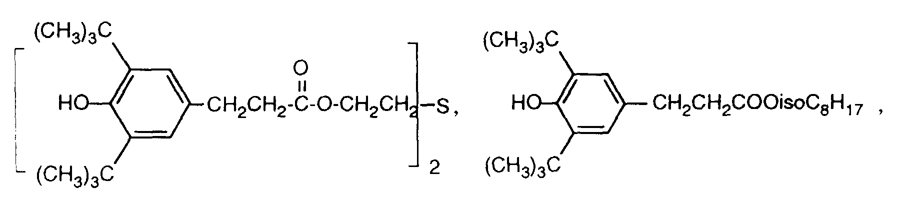

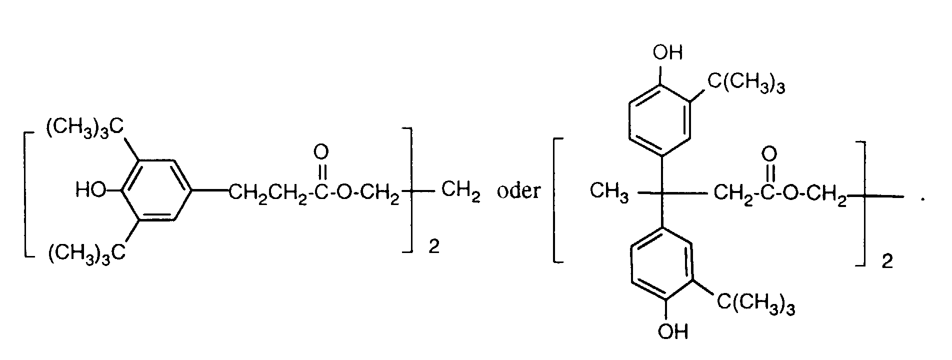

Besonders bevorzugte Verbindungen der Formel III sind 3-(3',5'-Di-tert-butyl-4'-hydroxyphenyl)propionsäuremethylester und 3-(3'-tert-Butyl-4'-hydroxy-5'-methylphenyl)propionsäuremethylester.

Einen speziell bevorzugten Gegenstand der Erfindung bilden Produkte, erhältlich durch Umsetzung der Komponenten a), b), c) und d), wobei die Komponente a) eine Verbindung der Formel I, insbesondere Pentaerythrit, Thiodiethylenglykol, 1,4-Butandiol, 1,2-Propandiol, Diethylenglykol, Triethylenglykol, Diethanolamin oder Glycerin bedeutet oder ein Gemisch davon, die Komponente b) eine Verbindung der Formel II, insbesondere Sonnenblumenöl, Kokosfett, Rapsöl, Maiskeimöl, Distelöl, Olivenöl, Erdnussöl oder Radiamuls darstellt oder ein Gemisch davon, die Komponente c) eine Verbindung der Formel III, insbesondere 3-(3',5'-Di-tert-butyl-4'-hydroxyphenyl)propionsäuremethylester oder 3-(3'-tert-Butyl-4'-hydroxy-5'-methylphenyl)propionsäuremethylester bedeutet oder ein Gemisch davon, und die Komponente d) eine Verbindung der Formel IV, insbesondere Schwefel oder Dithiophosphorsäurediisopropylester darstellt oder ein Gemisch davon.

Die vorliegende Erfindung betrifft ferner Produkte, erhältlich durch Umsetzung der Komponenten a), b), c) und d) in einem molaren Mengenverhältnis von 0,1:1:0,1:0,1 bis 15:1:30:10. Bevorzugt ist ein molares Mengenverhältnis von 1:1:1:0,5 bis 10:1:20:10. Besonders bevorzugt ist ein molares Mengenverhältnis von 1:1:2:2 bis 10:1:20:10.

Die erfindungsgemässen Produkte können z.B. 30 bis 80 Gew.-%, bevorzugt 35 bis 80 Gew.-%, insbesondere 50 bis 80 Gew.-% der Wirkgruppe E-2 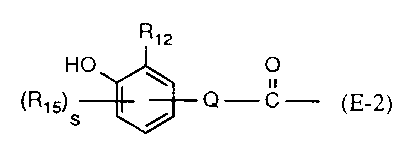 enthalten.

Wie bereits erwähnt, eignen sich die vorliegenden Produkte zum Stabilisieren von organischen Materialien gegen oxidativen, thermischen oder lichtinduzierten Abbau. Insbesondere wird auf ihre hervorragende Wirkung als Antioxidantien bei der Stabilisierung organischer Materialien hingewiesen.

Beispiele für derartige Materialien sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
30. Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher Zusammensetzungen enthaltend ein gegen oxidativen, thermischen oder lichtinduzierten Abbau empfindliches organisches Material und mindestens ein Produkt, erhältlich durch Umsetzung der Komponenten a), b), c) und d), sowie die Verwendung dieser Produkte zum Stabilisieren von organischem Material gegen oxidativen, thermischen oder lichtinduzierten Abbau.

Die Erfindung umfasst daher auch ein Verfahren zum Stabilisieren von organischem Material gegen thermischen, oxidativen oder lichtinduzierten Abbau, dadurch gekennzeichnet, dass man diesem Material mindestens ein Produkt, erhältlich durch Umsetzung der Komponenten a), b), c) und d), zusetzt.

Von besonderem Interesse ist die Verwendung dieser Produkte als Antioxidantien in organischen Materialien.

Bevorzugte organische Materialien sind Polymere, z.B. synthetische Polymere, insbesondere thermoplastische Polymere. Besonders bevorzugte organische Materialien sind Polyolefine und Styrolcopolymere, z.B. die oben unter den Punkten 1 bis 3 und unter den Punkten 6 und 7 angegebenen, insbesondere Polyethylen und Polypropylen sowie ABS und Styrol-Butadien-Copolymere. Bevorzugter Gegenstand der Erfindung sind daher Zusammensetzungen, worin das organische Material ein synthetisches organisches Polymer bzw. ein Gemisch solcher Polymere, insbesondere ein Polyolefin oder ein Styrolcopolymer ist.

Im allgemeinen werden die Produkte dem zu stabilisierenden Material in Mengen von 0,01 bis 10 %, bevorzugt 0,01 bis 5 %, insbesondere 0,01 bis 2 %, bezogen auf das Gesamtgewicht deszu stabilisierenden Materials, zugesetzt. Besonders bevorzugt ist der Einsatz der erfindungsgemässen Produkte in Mengen von 0,01 bis 0,5 %, vor allem 0,05 bis 0,3 %.

Neben dem Produkt können die erfindunsgemässen Zusammensetzungen zusätzlich herkömmliche Additive enthalten, wie beispielsweise die unten angegebenen.
1. Antioxidantien
   1.1. Alkylierte Monophenole, z.B. 2,6-Di-tert-butyl-4-methylphenol, 2-butyl-4,6-di-methylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
   1.4. Tocopherole, z.B. α-Tocopherol, β-Tocopherol, γ-Tocopherol, δ-Tocopherol und Mischungen davon (Vitamin E).
   1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
   1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-di-methylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
   1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.
   1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.13. Ester der β-(3,5-Di-tert-butyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der β-(3,5-Dicyclohexyl-4-hydroxyphenyl)-propionsäure mit ein- oder mehr wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis- (hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.17. Amide der β-(3,5-Di-tert-butyl-4-hydroxyphenyl)-propionsäure, wie z.B. N,N'-Bis- (3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
2. UV-Absorber und Lichtschutzmittel
   2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; [R-CH₂CH₂-COO(CH₂)₃ 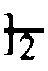 mit R = 3'-tert-Butyl-4'-hydroxy-5'-2H-benzotriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
   2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-piva loyloxy-phenyl)-5,7-di-tert-butyl-benzofuran-2-on.

Die herkömmlichen Additive werden beispielsweise in Konzentrationen von 0,01 bis 10 %, bezogen auf das Gesamtgewicht des zu stabilisierenden Materials, zugesetzt.

Die Einarbeitung der Produkte sowie gegebenenfalls weiterer Additive in das organische Material erfolgt nach bekannten Methoden. Die Einarbeitung in die Materialien kann beispielsweise durch Einmischen oder Aufbringen der Produkte und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbrindung der gelösten oder dispergierten Produkte auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Produkte in Polymere besteht in deren Zugabe vor, während oder unmittelbar nach der Polymerisation der entsprechenden Monomeren bzw. vor der Vernetzung. Die erfindungsgemässen Produkte können dabei als solche, aber auch in verkapselter Form (z.B. in Wachsen, Oelen oder Polymeren) zugesetzt werden. Im Falle der Zugabe vor oder während der Polymerisation können die erfindungsgemässen Produkte auch als Regulatoren für die Kettenlänge der Polymeren (Kettenabbrecher) wirken. Die erfindungsgemässen Produkte können auch in Form eines Masterbatches, der diese beispielsweise in einer Konzentration von 2,5 bis 25 Gew.-% enthält, den zu stabilisierenden Materialien zugesetzt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Weitere Gegenstände der Erfindung sind Zusammensetzungen, enthaltend eine funktionelle Flüssigkeit, bevorzugt aus der Reihe der Schmierstoffe, der Hydraulikflüssigkeiten und der Metallbearbeitungsflüssigkeiten sowie Kraftstoffe zum Antrieb von Motoren des Typs 4-Takt-Otto-, 2-Takt-, Diesel-, Wankel- sowie Orbital und mindestens ein Produkt, erhältlich durch Umsetzung der Komponenten a), b), c) und d).

Besonders bevorzugt als Schmierstoff sind die Mineralöle, die synthetischen Oele oder Mischungen davon.

Als funktionelle Flüssigkeiten aus der Reihe der Schmierstoffe, der Hydraulikflüssigkeiten und der Metallbearbeitungsflüssigkeiten kommen die an sich bekannten Produkte zum Einsatz.

Die in Frage kommenden Schmierstoffe und Hydraulikflüssigkeiten sind dem Fachmann geläufig und z.B. in Dieter Klamann "Schmierstoffe und verwandte Produkte", Verlag Chemie, Weinheim, 1982, in Schewe-Kobek, "Das Schmiermittel-Taschenbuch", Dr. Alfred Hüthig-Verlag, Heidelberg, 1974, oder in "Ullmanns Encyklopädie der technischen Chemie", Band 13, Seiten 85-94 (Verlag Chemie, Weinheim, 1977) beschrieben.

Beispiele hierfür sind Schmierstoffe und Hydraulikflüssigkeiten auf Mineralöl-Basis oder synthetische Schmierstoffe oder Hydraulikflüssigkeiten, insbesondere solche, die Carbonsäure-Esterderivate darstellen und bei Temperaturen von 200°C und höher verwendet werden.

Beispiele von synthetischen Schmierstoffen umfassen Schmierstoffe auf der Basis eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropan-tripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrittetracaprylat, oder eines komplexen Esters von einwertigen und zweiwertigen Säuren mit mehrwertigen Alkoholen, wie z.B. ein komplexer Ester von Trimethylolpropan mit Capryl- und Sebacinsäure oder von einem Gemisch davon.

Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Die erfindungsgemässen Produkte sind Oele und gut in Schmierstoffen löslich und deshalb als Zusätze zu Schmierstoffen besonders geeignet und es ist auf ihre überraschend gute antioxidative und antikorrosive Wirkung hinzuweisen.

Beispielsweise in Schmierstoffen für Verbrennungsmotoren, wie z.B. in Verbrennungsmotoren nach dem Otto-Prinzip, vermögen die erfindungsgemässen Produkte ihre überraschenden Eigenschaften zu entfalten. So verhindern dabei die erfindungsgemässen Produkte in Schmierölen die Bildung von Ablagerungen (Schlamm) oder reduzieren diese in überraschendem Masse.

Es ist auch möglich, sogenannte Masterbatches herzustellen.

Die erfindungsgemässen Produkte wirken schon in sehr geringen Mengen als Additive in Schmierstoffen. Sie werden den Schmierstoffen zweckmässig in einer Menge von 0,01 bis 5 Gew.-%, vorzugsweise in einer Menge von 0,05 bis 3 Gew.-% und ganz besonders bevorzugt in einer Menge von 0,1 bis 2 Gew.-%, jeweils bezogen auf den Schmierstoff, beigemischt.

Die Schmierstoffe können zusätzlich andere Additive enthalten, die zugegeben werden, um die Grundeigenschaften von Schmierstoffen noch weiter zu verbessern; dazu gehören: Antioxidantien, Metallpassivatoren, Rostinhibitoren, Viskositätsindex-Verbesserer, Stockpunkterniedriger, Dispergiermittel, Detergentien, Hochdruck-Zusätze, Reibungsverbesserer und Antiverschleiss-Additive.

Beispielsweise ist eine Reihe solcher Verbindungen obiger Auflistung "1. Antioxidantien", insbesondere Punkte 1.1 bis 1.16 zu entnehmen. Zusätzlich sind weitere Additive beispielhalft zu nennen:

### Beispiele für aminische Antioxidantien:

N,N'-Di-isopropyl-p-phenylendiamin, N,N'-Di-sec-butyl-p-phenylendiamin, N,N'-Bis-(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diaminodiphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

### Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy-3,7,10,14-tetrathiahexadecan.

### Beispiele für Metall-Desaktivatoren, z.B. für Kupfer, sind:

a) Benztriazole und deren Derivate, z.B. 4- oder 5-Alkylbenztriazole (z.B. Tolutriazol) und deren Derivate, 4,5,6,7-Tetrahydrobenztriazol, 5,5'-Methylenbis-benztriazol; Mannich-Basen von Benztriazol oder Tolutriazol wie 1-[Di(2-ethylhexyl)aminomethyl)-tolutriazol und 1-[Di(2-ethylhexyl)aminomethyl)-benztriazol; Alkoxyalkylbenztriazole wie 1-(Nonyloxymethyl)-benztriazol, 1-(1-Butoxyethyl)-benztriazol und 1-(1-Cyclohexyloxybutyl)-tolutriazol.
b) 1,2,4-Triazole und deren Derivate, z.B. 3-Alkyl (oder Aryl)- 1,2,4-Triazole, Mannich-Basen von 1,2,4-Triazolen wie 1-[Di(2-ethylhexyl)aminomethyl-1,2,4-triazol; Alkoxyalkyl-1,2,4-triazole wie 1-(1-Butoxyethyl-1,2,4-triazol; acylierte 3-Amino-1,2,4-triazole.
c) Imidazolderivate, z.B. 4,4'-Methylenbis(2-undecyl-5-methylimidazol, Bis[(N-methyl)imidazol-2-yl]carbinol-octylether.
d) Schwefelhaltige heterocyclische Verbindungen, z.B. 2-Mercaptobenzthiazol, 2,5-Dimercapto-1,3,4-thiadiazol und deren Derivate; 3,5-Bis[di(2-ethylhexyl)amino-methyl]-1,3,4-thiadiazolin-2-on.
e) Aminoverbindungen, z.B. Salicyliden-propylendiamin, Salicylaminoguanidin und deren Salze.

### Beispiele für Rost-Inhibitoren sind:

a) Organische Säuren, ihre Ester, Metallsalze, Aminsalze und Anhydride, z.B. Alkyl- und Alkenylbernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-Carboxymethyl-1-dodecyl-3-methylglycerin und dessen Aminsalze.
b) Stickstoffhaltige Verbindungen, z.B.:
   I. Primäre, sekundäre oder tertiäre aliphatische oder cycloaliphatische Amine und Aminsalze von organischen und anorganischen Säuren, z.B. öllösliche Alkylammoniumcarboxylate, ferner 1-[N,N-bis-(2-hydroxyethyl)amino]-3-(4-nonylphenoxy)-propan-2-ol.
   II. Heterocyclische Verbindungen, z.B.:
      Substituierte Imidazoline und Oxazoline, 2-Heptadecenyl-1-(2-hydroxyethyl)-imidazolin.
c) Phosphorhaltige Verbindungen, z.B.:
   Aminsalze von Phosphorsäurepartialestern oder Phosphonsäurepartialestern, Zinkdialkyldithiophosphate.
d) Schwefelhaltige Verbindungen, z.B.:
   Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.
e) Glycerinderivate, z.B.:
   Glycerin-monooleat, 1-(Alkylphenoxy)-3-(2-hydroxyethyl)glycerine, 1-(Alkylphenoxy)-3-(2,3-dihydroxypropyl)glycerine, 2-Carboxyalkyl-1,3-dialkylglycerine.

### Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

### Beispiele für Stockpunkterniedriger sind:

Polymethacrylat, alkylierte Naphthalinderivate.

### Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

### Beispiele für Verschleissschutz-Additive sind:

Schwefel und/oder Phosphor und/oder Halogen enthaltende Verbindungen, wie geschwefelte Olefine und pflanzliche Oele, Zinkdialkyldithiophosphate, alkylierte Triphenylphosphate, Tritolylphosphat, Tricresylphosphat, chlorierte Paraffine, Alkyl- und Aryldi- und tri-sulfide, Aminsalze von Mono- und Dialkylphosphaten, Aminsalze der Methylphosphonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)-thio]-propionsäure-ethylester, Triphenylthiophosphat (Triphenylphosphorothioat), Tris-(alkylphenyl)phosphorothioate und deren Gemische, (z.B. Tris(isononylphenyl)phosphorothioat), Diphenyl-monononylphenyl-phosphorothioat, Isobutylphenyl-diphenylphosphorothioat, Dodecylaminsalz des 3-Hydroxy-1,3-thiaphosphetan-3-oxids, Trithiophosphorsäure-5,5,5-tris[isooctylacetat(2)], Derivate von 2-Mercaptobenzthiazol wie 1-[N,N-Bis(2-ethylhexyl)aminomethyl-2-mercapto-1H-1,3-benzthiazol, Ethoxycarbonyl-5-octyl-dithiocarbamat.

Speziell bevorzugte zusätzliche Additive in Schmierstoffen sind aminische Antioxidantien, insbesondere Gemische aus mono- und dialkylierten tert-Butyl/tert-Octyldiphenylaminen.

Die folgenden Beispiele erläutern die Erfindung weiter. Teil- und Prozentangaben beziehen sich darin, soweit nichts anderes angegeben ist, auf das Gewicht.

### Beispiel 1: Herstellung der Sonnenblumenöl-Derivate mit Glycerin, 3-(3',5'-Di-tert-butyl-4'-hydroxyphenyl)propionsäuremethylester und Schwefel.

Eine Mischung von 41,5 g (∼50 mMol) Sonnenblumenöl, 4,60 g (50 mMol) Glycerin und 51 mg (0,20 mMol) Dibutylzinnoxid wird in einem Sulfierkolben mit Rückflusskühler und mechanischem Rührer unter Stickstoff während 3 Stunden bei 180-185°C gehalten. Anschliessend werden 29,2 g (100 mMol) 3-(3',5'-Di-tert-butyl-4'-hydroxyphenyl)propionsäuremethylester und nochmals 51 mg (0,20 mMol) Dibutylzinnoxid zugegeben. Das Reaktionsgemisch wird 8 Stunden bei 180-185°C weiter gerührt. Nach dem Abkühlen auf ca. 100°C werden 4,8 g (150 mMol) Schwefel zugegeben und unter Stickstoff während einer Stunde bei 180-190°C gehalten. Nach dem Abkühlen werden 70,1 g (99 %) Produkt als dunkles Oel mit einem Schwefelgehalt von 6,24 % erhalten.

### Beispiel 2: Herstellung der Sonnenblumenöl-Derivate mit Glycerin, 3-(3',5'-Di-tert-butyl-4'-hydroxyphenyl)propionsäuremethylester und Schwefel.

Eine Mischung von 41,5 g (∼50 mMol) Sonnenblumenöl, 4,60 g (50 mMol) Glycerin und 51 mg (0,20 mMol) Dibutylzinnoxid wird in einem Sulfierkolben mit Rückflusskühler und mechanischem Rührer unter Stickstoff während 3 Stunden bei 180-185°C gehalten. Anschliessend werden 29,2 g (100 mMol) 3-(3',5'-Di-tert-butyl-4'-hydroxyphenyl)propionsäuremethylester und nochmals 51 mg (0,20 mMol) Dibutylzinnoxid zugegeben. Das Reaktionsgemisch wird 8 Stunden bei 180-185°C weiter gerührt. Nach dem Abkühlen auf ca. 100°C werden 9,6 g (300 mMol) Schwefel zugegeben und unter Stickstoff während einer Stunde bei 180-190°C gehalten. Nach dem Abkühlen werden 71,1 g (99 %) Produkt als dunkles Oel mit einem Schwefelgehalt von 9,67 % erhalten.

### Beispiel 3: Herstellung der Sonnenblumenöl-Derivate mit Glycerin, 3-(3',5'-Di-tert-butyl-4'-hydroxyphenyl)propionsäuremethylester und Dithiophosphorsäurediisopropylester.

Eine Mischung von 30 g (∼34 mMol) Sonnenblumenöl, 14,22 g (154 mMol) Glycerin und 100 mg (0,40 mMol) Dibutylzinnoxid wird in einem Sulfierkolben mit Rückflusskühler und mechanischem Rührer unter Stickstoff während 7 Stunden bei 180-190°C gehalten. Anschliessend werden 87,73 g (300 mMol) 3-(3',5'-Di-tert-butyl-4'-hydroxyphenyl)propionsäuremethylester und nochmals 100 mg (0,40 mMol) Dibutylzinnoxid zugegeben. Das Reaktionsgemisch wird 15 Stunden bei 180-190°C weiter gerührt. Nach dem Abkühlen auf ca. 100°C werden 21,4 g (100 mMol) Dithiophosphorsäurediisopropylester zugegeben und unter Stickstoff während 8 Stunden bei 100-110°C gehalten. Nach dem Abkühlen werden 143,4 g (97 %) Produkt als helles Oel mit einem Schwefelgehalt von 3,4 % und einem Phosphorgehalt von 2,13 % erhalten.

### Beispiel 4: Herstellung der Sonnenblumenöl-Derivate mit Glycerin, 3-(3',5'-Di-tert-butyl-4'-hydroxyphenyl)propionsäuremethylester und Dithiophosphorsäurediisopropylester.

Eine Mischung von 30 g (∼34 mMol) Sonnenblumenöl, 14,22 g (154 mMol) Glycerin und 100 mg (0,40 mMol) Dibutylzinnoxid wird in einem Sulfierkolben mit Rückflusskühler und mechanischem Rührer unter Stickstoff während 7 Stunden bei 180-190°C gehalten. Anschliessend werden 87,73 g (300 mMol) 3-(3',5'-Di-tert-butyl-4'-hydroxyphenyl)pro pionsäuremethylester und nochmals 100 mg (0,40 mMol) Dibutylzinnoxid zugegeben. Das Reaktionsgemisch wird 15 Stunden bei 180-190°C weiter gerührt. Nach dem Abkühlen auf ca. 100°C werden 42,8 g (200 mMol) Dithiophosphorsäurediisopropylester zugegeben und unter Stickstoff während 8 Stunden bei 100-110°C gehalten. Nach dem Abkühlen werden 164,3 g (97 %) Produkt als helles Oel mit einem Schwefelgehalt von 6,0 % und einem Phosphorgehalt von 3,72 % erhalten.

### Beispiel 5: Herstellung der Sonnenblumenöl-Derivate mit Glycerin, 3-(3',5'-Di-tert-butyl-4'-hydroxyphenyl)propionsäuremethylester und Schwefel.

Eine Mischung von 30 g (∼34 mMol) Sonnenblumenöl, 14,22 g (154 mMol) Glycerin und 100 mg (0,40 mMol) Dibutylzinnoxid wird in einem Sulfierkolben mit Rückflusskühler und mechanischem Rührer unter Stickstoff während 7 Stunden bei 180-190°C gehalten. Anschliessend werden 87,73 g (300 mMol) 3-(3',5'-Di-tert-butyl-4'-hydroxyphenyl)propionsäuremethylester und nochmals 100 mg (0,40 mMol) Dibutylzinnoxid zugegeben. Das Reaktionsgemisch wird 15 Stunden bei 180-190°C weiter gerührt. Nach dem Abkühlen auf ca. 100°C werden 3,2 g (100 mMol) Schwefel zugegeben und unter Stickstoff während einer Stunde bei 180-190°C gehalten. Nach dem Abkühlen werden 129,5 g (99 %) Produkt als dunkles Oel mit einem Schwefelgehalt von 2,4 % erhalten.

### Beispiel 6: Herstellung der Sonnenblumenöl-Derivate mit Glycerin, 3-(3',5'-Di-tert-butyl-4'-hydroxyphenyl)propionsäuremethylester und Schwefel.

Eine Mischung von 30 g (∼34 mMol) Sonnenblumenöl, 14,22 g (154 mMol) Glycerin und 100 mg (0,40 mMol) Dibutylzinnoxid wird in einem Sulfierkolben mit Rückflusskühler und mechanischem Rührer unter Stickstoff während 7 Stunden bei 180-190°C gehalten. Anschliessend werden 87,73 g (300 mMol) 3-(3',5'-Di-tert-butyl-4'-hydroxyphenyl)propionsäuremethylester und nochmals 100 mg (0,40 mMol) Dibutylzinnoxid zugegeben. Das Reaktionsgemisch wird 15 Stunden bei 180-190°C weiter gerührt. Nach dem Abkühlen auf ca. 100°C werden 6,4 g (200 mMol) Schwefel zugegeben und unter Stickstoff während einer Stunde bei 180-190°C gehalten. Nach dem Abkühlen werden 132,3 g (99 %) Produkt als dunkles Oel mit einem Schwefelgehalt von 4,56 % erhalten.

### Beispiel 7: Herstellung der Sonnenblumenöl-Derivate mit Thiodiethylenglykol, 3-(3',5'-Di-tert-butyl-4'-hydroxyphenyl)propionsäuremethylester und Schwefel.

Eine Mischung von 41,5 g (∼50 mMol) Sonnenblumenöl, 6,1 g (50 mMol) Thiodiethylenglykol und 50 mg (0,20 mMol) Dibutylzinnoxid wird in einem Sulfierkolben mit Rückflusskühler und mechanischem Rührer unter Stickstoff während 10 Stunden bei 180-190°C gehalten. Anschliessend werden 29,2 g (50 mMol) 3-(3',5'-Di-tert-butyl-4'-hydroxyphenyl)propionsäuremethylester und nochmals 50 mg (0,20 mMol) Dibutylzinnoxid zugegeben. Das Reaktionsgemisch wird 15 Stunden bei 180-190°C weiter gerührt. Nach dem Abkühlen auf ca. 100°C werden 4,8 g (150 mMol) Schwefel zugegeben und unter Stickstoff während einer Stunde bei 180-190°C gehalten. Nach dem Abkühlen werden 71,9 g (99 %) Produkt als dunkles Oel mit einem Schwefelgehalt von 7,11 % erhalten.

### Beispiel 8: "Deposit and Oxidation Panel Test" (DOPT).

Beim "Deposit and Oxidation Panel Test" (DOPT) handelt es sich um eine Variante einer Prüfmethode für Motorenöle, insbesondere für Dieselmotorenöle, welche durch G. Abellaneda et al, IIIè Symposium CEC, 1989, 61, New Cavendish Street, London WIM8AR, England beschrieben wurde. Dabei wird die Eignung der Oele mit Stabilisator zur Verhinderung von Ablagerungen auf den Kolben geprüft.

Die Testdauer beträgt 20 Stunden, die Panel-Temperatur 260°C und der Oelfluss 1 ml/Minute. Die feuchte Luft-Atmosphäre wird mit 260 ppm NO₂ und 26 ppm SO₂ angereichert. Nach dem Test wird die Metallplatte (Panel), auf die das Oel tropft, gewogen und visuell bewertet. Je kleiner die Zahlen sind, desto besser. Als Schmieroel wird ein handelsübliches CD-Oel, welches mit dem Grundoel STANCO 150® verdünnt wird, verwendet. Diesem vorbereiteten Oel werden nun die in Tabelle 1 angegebenen Stabilisatoren in einer Menge von 0,6 Gew-% bezogen auf das Oel, zugemischt und einer "DOPT-Prüfung" unterzogen. Je kleiner die Werte, desto besser ist die Stabilisatorwirkung.

**Tabelle 1:**

| "Deposit and Oxidation Panel Test" (DOPT) | | |
|---|---|---|
| Produkt gemäss Beispiel | Konz. in Gew.-% | Ablagerungen auf Panel Gewicht (mg) |
| ― | ― | 72 |
| Beispiel 1 | 0,6 | 38 |
| Beispiel 2 | 0,6 | 29 |

### Beispiel 9: Test auf Verschleiss-Schutz.

Zur Prüfung der erfindungsgemässen Stabilisatoren auf die Eignung auf Verschleiss-Schutz wird die ASTM-Standard-Methode D 2783-81 unter Verwendung des Shell-Vierkugel-Apparates herangezogen. Als Basisöl wird Oel BB [Mobil Stock, Kohlenstoff (aromatisch) 6,5 %; Kohlenstoff (aliphatisch) 72 %; Kohlenstoff (naphthylisch) 21,5 %] verwendet. Gemessen wird der mittlere Verschleiss-Durchmesser WSD ("Wear Scar Diameter") in mm bei einer Last von 40 kg während einer Stunde. Diesem Oel werden die erfindungsgemässen Stabilisatoren in einer Menge von 1,0 Gew.-% zugesetzt. Die Resultate sind in Tabelle 2 zusammensgefasst. Je kleiner die Werte, desto besser ist die Stabilisatorwirkung.

**Tabelle 2:**

| Test auf Verschleiss-Schutz | | |
|---|---|---|
| Produkt gemäss Beispiel | Konz. in Gew.-% | Verschleiss-Durchmesser WSD (mm) |
| ― | ― | 0,91 |
| Beispiel 1 | 1,0 | 0,35 |
| Beispiel 2 | 1,0 | 0,37 |
| Beispiel 4 | 1,0 | 0,36 |

## Patentansprüche

1. Produkt, erhältlich durch Umsetzung der Komponenten a), b), c) und d), wobei die Komponente a) eine Verbindung der Formel I oder ein Gemisch von Verbindungen der Formel I, die Komponente b) eine Verbindung der Formel II oder ein Gemisch von Verbindungen der Formel II, die Komponente c) eine Verbindung der Formel III oder ein Gemisch von Verbindungen der Formel III, und die Komponente d) eine Verbindung der Formel IV oder ein Gemisch von Verbindungen der Formel IV sind, 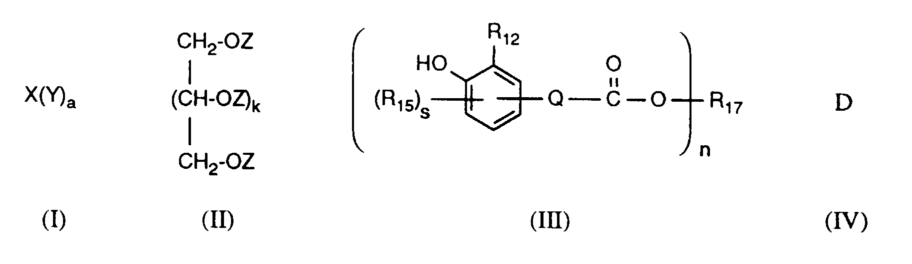 wobei in der Verbindung der Formel I die Reste
Y unabhängig voneinander OH, (HOCH₂CH₂)₂N- oder -HNR₁ darstellt und
R₁ Wasserstoff, C₁-C₁₈-Alkyl, C₅-C₁₂-Cycloalkyl,C₅-C₁₂-Cycloalkyl, 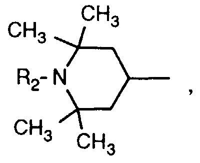 C₃-C₆-Alkenyl, C₇-C₉-Phenylalkyl, Phenyl oder durch 1 bis 3 Reste A₁ substituiertes Phenyl bedeutet, wobei die Reste A₁ unabhängig voneinander C₁-C₁₂-Alkyl, Halogen, Hydroxy, Methoxy oder Ethoxy bedeuten, wobei
R₂ Wasserstoff, C₁-C₈-Alkyl, O^{•}, OH, NO, -CH₂CN, C₁-C₁₈-Alkoxy, C₅-C₁₂-Cycloalkoxy, C₃-C₆-Alkenyl, C₇-C₉-Phenylalkyl oder C₇-C₉-Phenylalkyl, welches am Phenylring mit C₁-C₄-Alkyl mono-, di- oder tri-substituiert ist, oder R₂ ferner C₁-C₈-Acyl oder HOCH₂CH₂- darstellt, und
a die Zahl 1,2,3,4 oder 6 bedeutet, wobei
wenn Y = OH und a = 1 ist,
X C₁-C₄₅-Alkyl, C₃-C₁₈-Alkenyl, -CH₂CH₂T₁(CH₂CH₂O)_{b}R₄ oder 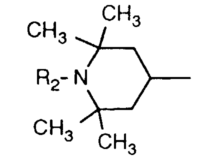 bedeutet, wobei R₂ die obige Bedeutung hat, und
T₁ Sauerstoff, Schwefel oder 〉N-R₅,
R₄ C₁-C₂₀-Alkyl,
b eine ganze Zahl aus dem Bereich von 0 bis 10 und
R₅ Wasserstoff, C₁-C₁₈-Alkyl oder Phenyl darstellt, oder
wenn Y = OH und a = 2 ist,
X -CH₂CH₂T₂(CH₂CH₂O)_{b}CH₂CH₂-, wobei b die obige Bedeutung hat, 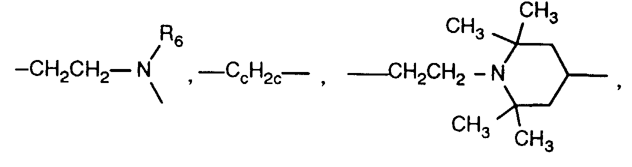 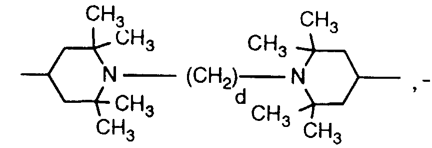
―CH₂-CH=CH-CH₂―, 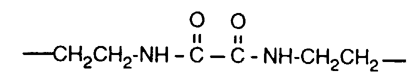
oder 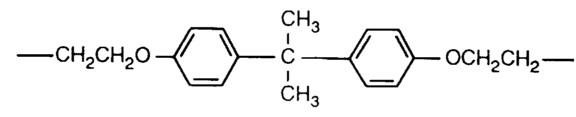 bedeutet, wobei
T₂ Sauerstoff, Schwefel, 〉N-R₅ oder 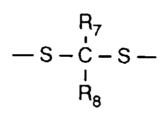 ist und R₅ die obige Bedeutung hat,
R₆ Wasserstoff, C₁-C₁₈-Alkyl oder Phenyl,
c eine ganze Zahl aus dem Bereich von 2 bis 10,
d eine ganze Zahl aus dem Bereich von 2 bis 6 und
R₇, R₈ unabhängig voneinander Wasserstoff, C₁-C₁₈-Alkyl oder Phenyl sind, oder R₇ und R₈ mit dem C-Atom, an das sie gebunden sind, einen C₅-C₁₂-Cycloalkylidenring bilden, oder
wenn a = 3 ist,
X C₃-C₁₀-Alkantriyl oder N(CH₂CH₂-)₃ bedeutet, oder
wenn Y = OH und a = 4 ist,
X C₄-C₁₀-Alkantetrayl, 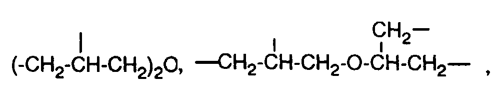 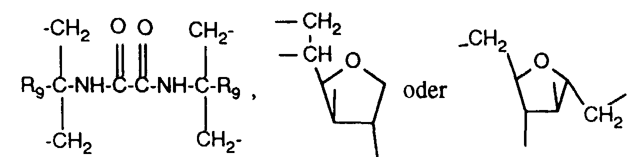 darstellt, wobei
R₉ C₁-C₄-Alkyl bedeutet, oder
wenn Y = OH und a = 6 ist,
X 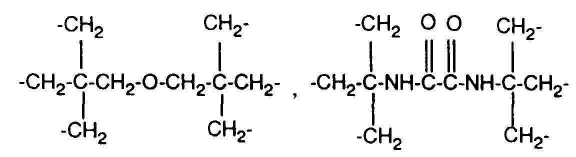 oder C₆-C₁₀-Alkanhexayl darstellt, oder
wenn Y = -HNR₁ und a = 1 ist,
X C₁-C₁₈-Alkyl, C₃-C₁₈-Alkenyl, C₅-C₁₂-Cycloalkyl, C₇-C₉-Phenylalkyl, Phenyl, 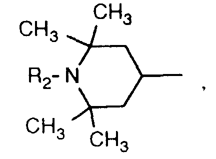 wobei R₂ die obige Bedeutung hat, oder X ferner 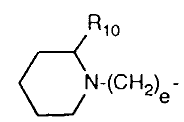 ist, oder X mit R₁ gemeinsam eine Gruppe der Formel -CH₂CH₂CH₂CH₂CH₂- oder -CH₂CH₂OCH₂CH₂- bildet, wobei
R₁₀ Wasserstoff oder Methyl, und
e 2 oder 3 bedeutet, oder
wenn Y = -HNR₁ und a = 2 ist,
X -C_{f}H_{2f}-, 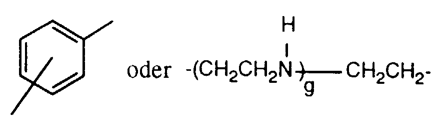 darstellt, wobei
f eine ganze Zahl aus dem Bereich von 2 bis 10 und
g eine ganze Zahl aus dem Bereich von 1 bis 6 bedeutet, und
in der Verbindung der Formel II die Reste
Z Wasserstoff oder eine Gruppe der Formel 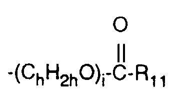 bedeutet, und
k eine ganze Zahl aus dem Bereich von 0 bis 6 darstellt, wobei
h 2 oder 3,
i eine ganze Zahl aus dem Bereich von 0 bis 12 und
R₁₁ C₈-C₃₀-Alkenyl bedeutet, mit der Bedingung, dass die Verbindung der Formel II eine Gruppe 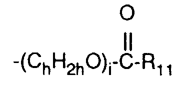 enthält;
in der Verbindung der Formel III
R₁₂ C₁-C₁₈-Alkyl, C₅-C₁₂-Cycloalkyl, Phenyl oder C₇-C₉-Phenylalkyl bedeutet,
R₁₅ Wasserstoff, C₁-C₁₈-Alkyl, C₅-C₁₂-Cycloalkyl, Phenyl oder C₇-C₉-Phenylalkyl darstellt,
s 0, 1 oder 2 bedeutet,
Q -CₘH₂ₘ-, 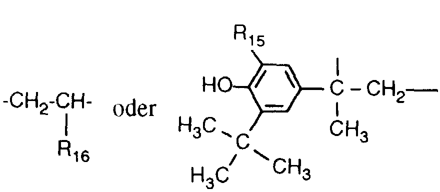 darstellt, wobei R₁₅ die obige Bedeutung hat,
m eine ganze Zahl aus dem Bereich von 0 bis 3 bedeutet,
R₁₆ C₁-C₈-Alkyl darstellt und
n eine ganze Zahl aus dem Bereich von 1 bis 6 bedeutet, wobei
wenn n = 1 ist,
R₁₇ Wasserstoff, C₁-C₄₅-Alkyl, C₅-C₁₂-Cycloalkyl, C₂-C₁₈-Alkenyl, einen einwertigen Rest einer Hexose, einen einwertigen Rest eines Hexitols, 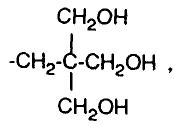 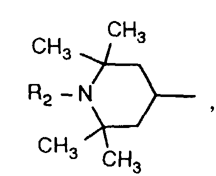 wobei R₂ die obige Bedeutung hat, oder ferner R₁₇ -CH₂CH₂-T₃-R₁₉ oder 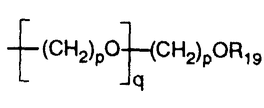 bedeutet, wobei
T₃ Sauerstoff, Schwefel oder 〉N-R₂₂ darstellt,
R₁₉ 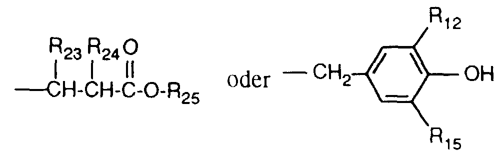 ist, wobei R₁₂ und R₁₅ die obige Bedeutung haben, oder R₁₉ ferner Wasserstoff, C₁-C₂₄-Alkyl, Phenyl, C₅-C₁₂-Cycloalkyl oder 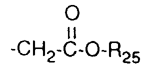 bedeutet, wobei
p eine ganze Zahl aus dem Bereich von 2 bis 4 darstellt,
q eine ganze Zahl aus dem Bereich von 2 bis 20 bedeutet,
R₂₂ C₁-C₁₈-Alkyl, Phenyl oder durch 1 bis 3 Reste A₁ substituiertes Phenyl bedeutet, wobei die Reste A₁ unabhängig voneinander C₁-C₁₂-Alkyl, Halogen, Hydroxy, Methoxy oder Ethoxy bedeuten, oder R₂₂ ferner C₅-C₈-Cycloalkyl darstellt,
R₂₃, R₂₄ unabhängig voneinander Wasserstoff oder Methyl bedeuten mit der Massgabe, dass R₂₃ und R₂₄ nicht gleichzeitig Methyl sind;
R₂₅ Wasserstoff oder C₁-C₂₄-Alkyl bedeutet, oder
wenn n = 2 ist,
R₁₇ einen zweiwertigen Rest einer Hexose, einen zweiwertigen Rest eines Hexitols, 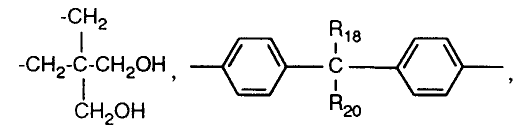 -CᵣH₂ᵣ-, 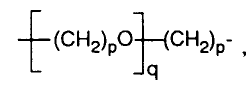 wobei p und q die obige Bedeutung haben, -CH₂CH₂-T₄-CH₂CH₂-, -CH₂-CH=CH-CH₂-, -CH₂-C≡C-CH₂-, 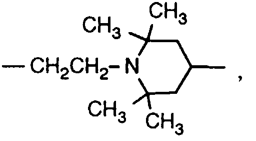 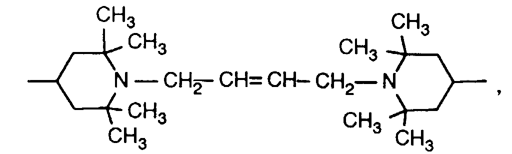 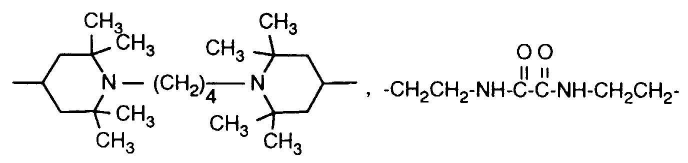 oder 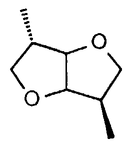 darstellt, wobei
R₁₈, R₂₀ unabhängig voneinander Wasserstoff oder C₁-C₁₂-Alkyl bedeuten oder zusammen den Rest -CH₂CH₂CH₂CH₂CH₂- bilden,
r eine ganze Zahl aus dem Bereich von 2 bis 10 darstellt,
T₄ Schwefel, 〉N-R₂₆ oder 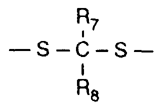 bedeutet, wobei R₇ und R₈ die obige Bedeutung haben, und
R₂₆ Wasserstoff, C₁-C₁₈-Alkyl, Phenyl oder durch 1 bis 3 Reste A₁ substituiertes Phenyl, wobei die Reste A₁ die obige für die Formel I beschriebene Bedeutung haben, oder R₂₆ ferner C₅-C₈-Cycloalkyl oder 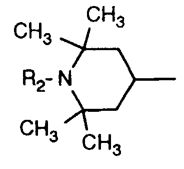 bedeutet, wobei R₂ die obige Bedeutung hat, oder
wenn n = 3 ist,
R₁₇ einen dreiwertigen Rest einer Hexose, einen dreiwertigen Rest eines Hexitols, 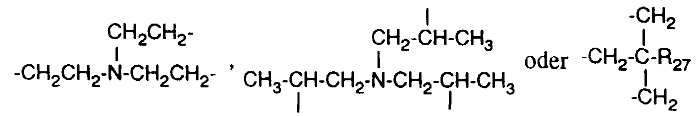 darstellt, wobei
R₂₇ Wasserstoff, -CH₂OH, C₁-C₄-Alkyl, C₁-C₁₈-Alkylamido oder 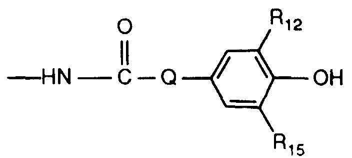 bedeutet, wobei Q, R₁₂ und R₁₅ die obige Bedeutung haben, oder
wenn n = 4 ist,
R₁₇ einen vierwertigen Rest einer Hexose, einen vierwertigen Rest eines Hexitols, C₄-C₁₀-Alkantetrayl, 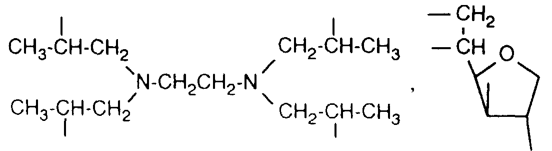 oder 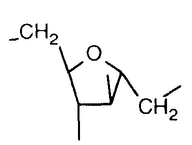 darstellt, oder
wenn n = 5 ist,
R₁₇ einen fünfwertigen Rest einer Hexose oder einen fünfwertigen Rest eines Hexitols darstellt, oder
wenn n = 6 ist,
R₁₇ einen sechswertigen Rest eines Hexitols oder 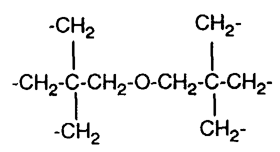 bedeutet,
in der Verbindung der Formel IV
D Schwefel, R₅₀-SH, 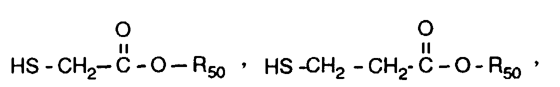 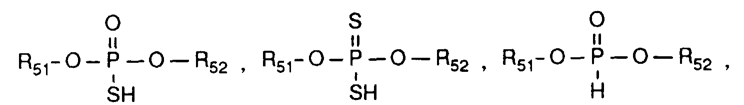 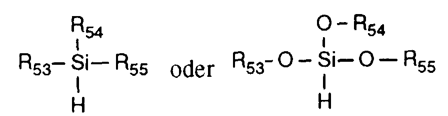 darstellt,
R₅₀ C₁-C₁₈-Alkyl, unsubstituiertes oder durch C₁-C₄-Alkyl substituiertes C₅-C₁₂-Cycloalkyl bedeutet,
R₅₁ und R₅₂ unabhängig voneinander C₁-C₁₈-Alkyl, unsubstituiertes oder durch C₁-C₁₈-Alkyl substituiertes Phenyl darstellt, und
R₅₃, R₅₄ und R₅₅ unabhängig voneinander C₁-C₁₈-Alkyl oder Phenyl bedeutet.

2. Produkt gemäss Anspruch 1, wobei in der Verbindung der Formel III s die Zahl 1 oder 2 bedeutet.

3. Produkt gemäss Anspruch 1,
wobei in der Verbindung der Formel I die Reste
Y unabhängig voneinander OH, (HOCH₂CH₂)₂N- oder -HNR₁ bedeutet und
R₁ Wasserstoff, C₁-C₁₀-Alkyl, C₅-C₇-Cycloalkyl, 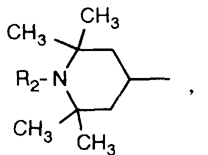 C₃-C₆-Alkenyl, Benzyl oder Phenyl bedeutet, wobei
R₂ Wasserstoff, C₁-C₄-Alkyl, OH, -CH₂CN, C₆-C₁₂-Alkoxy, C₅-C₈-Cycloalkoxy, Allyl, Benzyl, Acetyl oder HOCH₂CH₂- darstellt, und
a die Zahl 1,2,3,4 oder 6 bedeutet, wobei
wenn Y = OH und a = 1 ist,
X C₁-C₃₀-Alkyl, C₃-C₁₈-Alkenyl, -CH₂CH₂T₁(CH₂CH₂O)_{b}R₄ oder 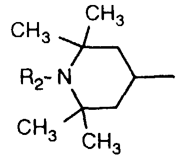 bedeutet, wobei R₂ die obige Bedeutung hat, und
T₁ Sauerstoff, Schwefel oder 〉N-R₅,
R₄ C₁-C₁₀-Alkyl,
b eine ganze Zahl aus dem Bereich von 0 bis 10 und
R₅ Wasserstoff, C₁-C₁₀-Alkyl oder Phenyl darstellen, oder
wenn Y = OH und a = 2 ist,
X -CH₂CH₂T₂(CH₂CH₂O)_{b}CH₂CH₂-, wobei b die obige Bedeutung hat, 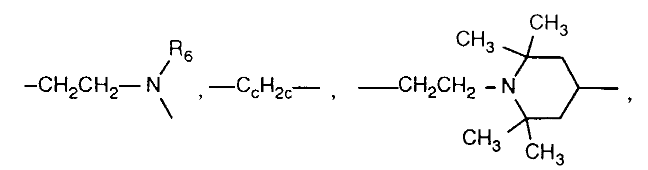 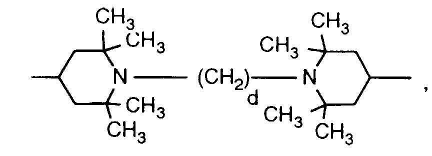 ―CH₂-CH=CH-CH₂―, 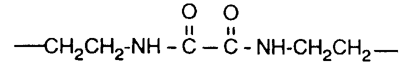 oder 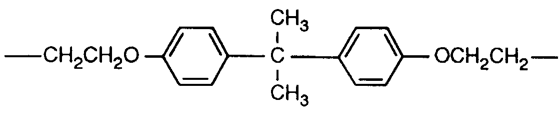 bedeutet, wobei
T₂ Sauerstoff, Schwefel, 〉N-R₅ oder 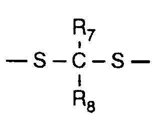 ist und R₅ die obige Bedeutung hat,
R₆ Wasserstoff, C₁-C₁₀-Alkyl oder Phenyl,
c eine ganze Zahl aus dem Bereich von 2 bis 10,
d eine ganze Zahl aus dem Bereich von 2 bis 6 und
R₇, R₈ unabhängig voneinander Wasserstoff, C₁-C₁₀-Alkyl oder Phenyl sind, oder R₇ und R₈ mit dem C-Atom, an das sie gebunden sind, einen C₅-C₇-Cycloalkyl-Ring bilden, oder
wenn Y = -HNR₁ und a = 1 ist,
X C₁-C₁₀-Alkyl, C₃-C1₈-Alkenyl, C₅-C₇-Cycloalkyl, Benzyl, Phenyl, 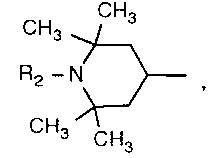 wobei R₂ die obige Bedeutung hat, oder X ferner 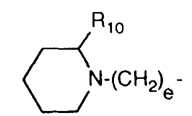 ist, oder X mit R₁ gemeinsam eine Gruppe der Formel -CH₂CH₂CH₂CH₂CH₂- oder -CH₂CH₂OCH₂CH₂- bildet, wobei
R₁₀ Wasserstoff oder Methyl ist, und
e 2 oder 3 bedeutet, und
in der Verbindung der Formel II die Reste
Z Wasserstoff oder eine Gruppe der Formel 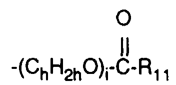 bedeuten, und
k eine ganze Zahl aus dem Bereich von 0 bis 4 darstellt, wobei
h 2 oder 3,
i eine ganze Zahl aus dem Bereich von 0 bis 6 und
R₁₁ C₈-C₂₀-Alkenyl bedeutet, mit der Bedingung, dass die Verbindung der Formel II eine Gruppe 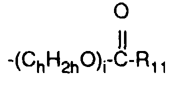 enthält;
in der Verbindung der Formel III
R₁₂ C₁-C₆-Alkyl, C₅-C₇-Cycloalkyl, Phenyl oder Benzyl bedeutet,
R₁₅ Wasserstoff, C₁-C₆-Alkyl, C₅-C₇-Cycloalkyl, Phenyl oder Benzyl darstellt,
s 1 oder 2 bedeutet,
Q -CₘH₂ₘ-, 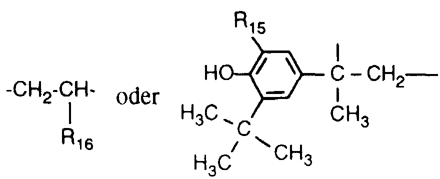 darstellt, wobei R₁₅ die obige Bedeutung hat,
m eine ganze Zahl aus dem Bereich von 0 bis 3 bedeutet,
R₁₆ C₁-C₄-Alkyl darstellt und
n eine ganze Zahl aus dem Bereich von 1 bis 6 bedeutet, wobei
wenn n = 1 ist,
R₁₇ Wasserstoff, C₁-C₃₀-Alkyl, C₅-C₇-Cycloalkyl, C₂-C₁₈-Alkenyl, einen einwertigen Rest einer Hexose, einen einwertigen Rest eines Hexitols, 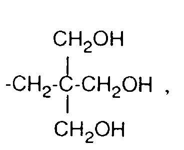 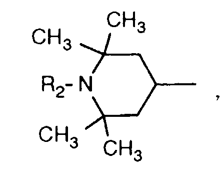 wobei R₂ die obige Bedeutung hat, oder ferner R₁₇ -CH₂CH₂-T₃-R₁₉ oder 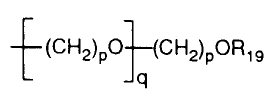 bedeutet, wobei
T₃ Sauerstoff, Schwefel oder 〉N-R₂₂ darstellt,
R₁₉ 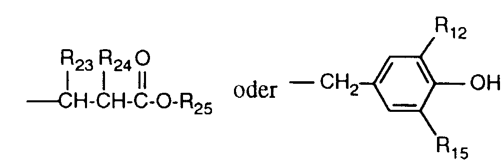 ist, wobei R₁₂ und R₁₅ die obige Bedeutung haben, oder R₁₉ ferner Wasserstoff, C₁-C₁₈-Alkyl, Phenyl, C₅-C₇-Cycloalkyl oder 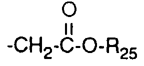 bedeutet, wobei
p eine ganze Zahl aus dem Bereich von 2 bis 4 darstellt,
q eine ganze Zahl aus dem Bereich von 2 bis 20 bedeutet,
R₂₂ C₁-C₁₀-Alkyl, Phenyl oder C₅-C₈-Cycloalkyl darstellt,
R₂₃, R₂₄ unabhängig voneinander Wasserstoff oder Methyl bedeuten, mit der Massgabe, dass R₂₃ und R₂₄ nicht gleichzeitig Methyl sind;
R₂₅ Wasserstoff oder C₁-C₁₈-Alkyl bedeutet, oder
wenn n = 2 ist,
R₁₇ einen zweiwertigen Rest einer Hexose, einen zweiwertigen Rest eines Hexitols, 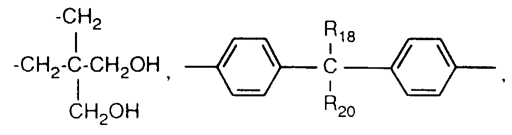 -CᵣH₂ᵣ-, 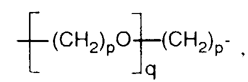 wobei p und q die obige Bedeutung haben, -CH₂CH₂-T₄-CH₂CH₂-, -CH₂-CH=CH-CH₂-, -CH₂-C≡C-CH₂- , 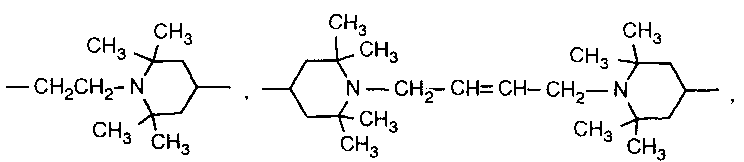 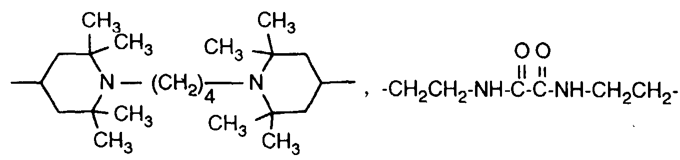 oder 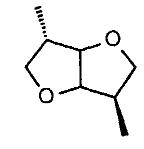 darstellt, wobei
R₁₈, R₂₀ unabhängig voneinander Wasserstoff oder C₁-C₆-Alkyl bedeuten oder zusammen den Rest -CH₂CH₂CH₂CH₂CH₂- bilden,
r eine ganze Zahl aus dem Bereich von 2 bis 10 darstellt,
T₄ Schwefel, 〉N-R₂₆ oder 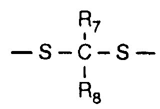 bedeutet, wobei R₇ und R₈ die obige Bedeutung haben und
R₂₆ Wasserstoff, C₁-C₁₀-Alkyl, Phenyl, C₅-C₈-Cycloalkyl oder 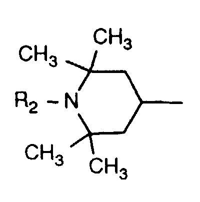 bedeutet, wobei R₂ die obige Bedeutung hat,
in der Verbindung der Formel IV
D Schwefel, R₅₀-SH, 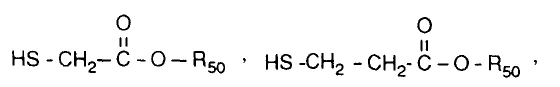 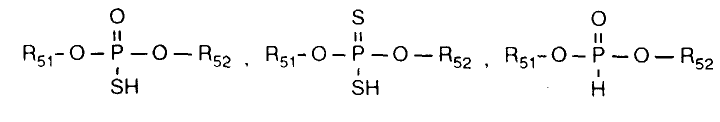 oder 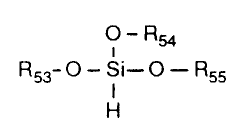 darstellt,
R₅₀ C₁-C₁₈-Alkyl oder C₅-C₁₂-Cycloalkyl bedeutet,
R₅₁ und R₅₂ unabhängig voneinander C₁-C₁₂-Alkyl, unsubstituiertes oder durch C₁-C₁₂-Alkyl substituiertes Phenyl darstellt, und
R₅₃, R₅₄ und R₅₅ unabhängig voneinander C₁-C₁₂-Alkyl oder Phenyl bedeutet.

4. Produkt gemäss Anspruch 1,
wobei in der Verbindung der Formel I die Reste
Y unabhängig voneinander OH, (HOCH₂CH₂)₂N- oder -HNR₁ darstellt und
R₁ Wasserstoff, C₁-C₄-Alkyl oder 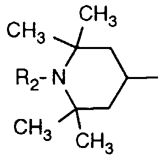 bedeutet, wobei
R₂ Wasserstoff, C₁-C₄-Alkyl, OH, Allyl, Benzyl, Acetyl oder HOCH₂CH₂- darstellt, und
a die Zahl 1,2,3,4 oder 6 bedeutet, wobei
wenn Y = OH und a = 1 ist,
X C₁-C₁₈-Alkyl, C₃-C₁₈-Alkenyl, -CH₂CH₂T₁(CH₂CH₂O)_{b}R₄ oder 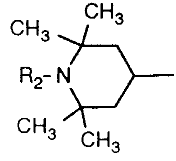 bedeutet, wobei R₂ die obige Bedeutung hat, und
T₁ Sauerstoff,
R₄ C₁-C₄-Alkyl und
b eine ganze Zahl aus dem Bereich von 0 bis 10 darstellt, oder
wenn Y = OH und a = 2 ist,
X -CH₂CH₂T₂(CH₂CH₂O)_{b}CH₂CH₂-, wobei b die obige Bedeutung hat, oder ferner X ―C_{c}H_{2c}―, 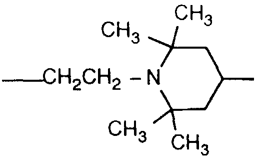 oder ―CH₂-CH=CH-CH₂― bedeutet, wobei
T₂ Sauerstoff, Schwefel oder 〉N-R₅,
R₅ Wasserstoff,
b die Zahl 0 oder 1, und
c eine ganze Zahl aus dem Bereich von 2 bis 8 darstellen, oder
wenn a = 3 ist,
X 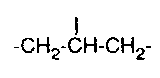 oder N(CH₂CH₂-)₃ bedeutet, oder
wenn Y = OH und a = 4 ist,
X 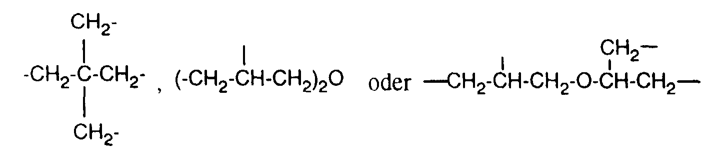 darstellt, oder
wenn Y = OH und a = 6 ist,
X 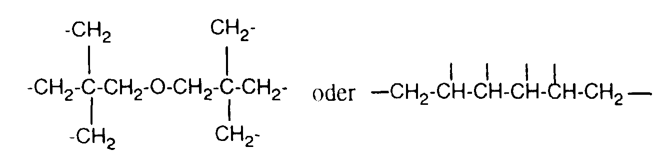 darstellt, oder
wenn Y = -HNR₁ und a = 1 ist,
X C₁-C₁₀-Alkyl, C₃-C₁₈-Alkenyl, C₅-C₇-Cycloalkyl oder 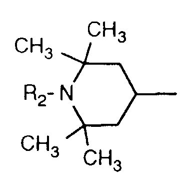 bedeutet, wobei R₂ die obige Bedeutung hat, oder
wenn Y = -HNR₁ und a = 2 ist,
X -C_{f}H_{2f}- darstellt, wobei
f eine ganze Zahl aus dem Bereich von 2 bis 10 bedeutet, und
in der Verbindung der Formel II die Reste
Z Wasserstoff oder eine Gruppe der Formel 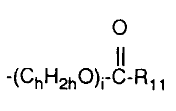 bedeutet, und
k 1, 2 oder 3 darstellt,
h 2 oder 3 bedeutet,
i eine ganze Zahl aus dem Bereich von 0 bis 4 ist und
R₁₁ C₈-C₂₀-Alkenyl bedeutet, mit der Bedingung, dass die Verbindung der Formel II eine Gruppe 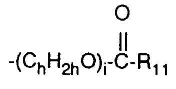 enthält;
in der Verbindung der Formel III
R₁₂ C₁-C₆-Alkyl oder C₅-C₇-Cycloalkyl bedeutet,
R₁₅ Wasserstoff, C₁-C₆-Alkyl oder C₅-C₇-Cycloalkyl darstellt,
s 1 oder 2 bedeutet,
Q -CₘH₂ₘ- oder 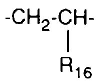 darstellt,
m eine ganze Zahl aus dem Bereich von 0 bis 3 bedeutet,
R₁₆ C₁-C₄-Alkyl darstellt und
n eine ganze Zahl aus dem Bereich von 1 bis 6 bedeutet, wobei
wenn n = 1 ist,
R₁₇ Wasserstoff, C₁-C₁₈-Alkyl, C₅-C₇-Cycloalkyl, C₂-C₁₈-Alkenyl, einen einwertigen Rest einer Hexose, einen einwertigen Rest eines Hexitols, 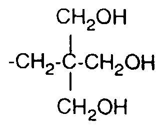 oder 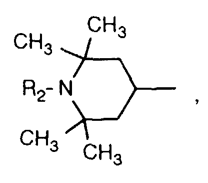 wobei R₂ die obige Bedeutung hat, oder ferner R₁₇ 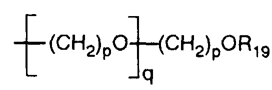 bedeutet, wobei
R₁₉ Wasserstoff, C₁-C₁₈-Alkyl oder C₅-C₇-Cycloalkyl bedeutet, wobei
p eine ganze Zahl aus dem Bereich von 2 bis 4 darstellt,
q eine ganze Zahl aus dem Bereich von 2 bis 10 bedeutet, oder
wenn n = 2 ist,
R₁₇ einen zweiwertigen Rest einer Hexose, einen zweiwertigen Rest eines Hexitols, 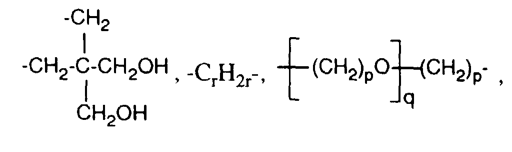 wobei p und q die obige Bedeutung haben, -CH₂CH₂-T₄-CH₂CH₂- oder 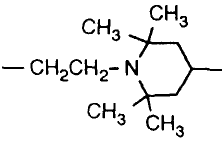 darstellt, wobei
r eine ganze Zahl aus dem Bereich von 2 bis 10 darstellt,
T₄ Schwefel oder 〉N-R₂₆ bedeutet und
R₂₆ Wasserstoff, C₁-C₁₀-Alkyl oder C₅-C₈-Cycloalkyl bedeutet, oder
wenn n = 3 ist,
R₁₇ einen dreiwertigen Rest einer Hexose, einen dreiwertigen Rest eines Hexitols, 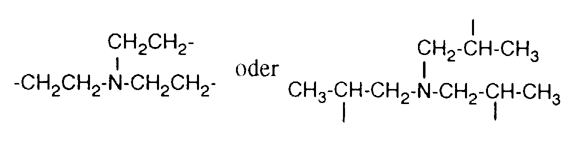 darstellt, oder
wenn n = 4 ist,
R₁₇ einen vierwertigen Rest einer Hexose, einen vierwertigen Rest eines Hexitols, 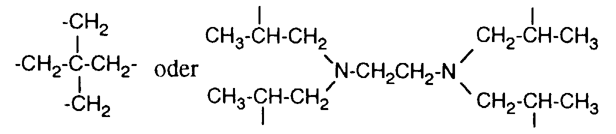 darstellt,
in der Verbindung der Formel IV
D Schwefel, 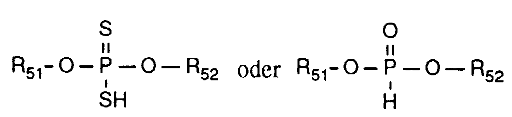 bedeutet, und
R₅₁ und R₅₂ unabhängig voneinander C₁-C₁₂-Alkyl, unsubstituiertes oder durch C₁-C₈-Alkyl substituiertes Phenyl darstellt.

5. Produkt gemäss Anspruch 1,
wobei in der Verbindung der Formel I die Reste
Y unabhängig voneinander Hydroxy oder -NH₂ bedeutet und
a eine ganze Zahl aus dem Bereich von 1 bis 4 darstellt, wobei
wenn a = 1 ist,
X = 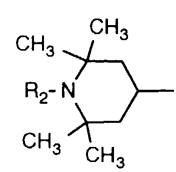 bedeutet, und
R₂ Wasserstoff, Methyl oder HOCH₂CH₂- darstellt, oder
wenn Y = OH und a = 2 ist,
X -CH₂CH₂T₂(CH₂CH₂O)_{b}CH₂CH₂-, ―C_{c}H_{2c}― oder 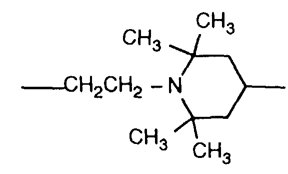 bedeutet, wobei
T₂ Sauerstoff, Schwefel oder 〉N-R₅,
R₅ Wasserstoff,
b die Zahl 0 oder 1 und
c die Zahl 2, 3 oder 4 darstellen, oder
wenn Y = OH und a = 3 ist,
X 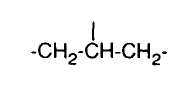 bedeutet, oder
wenn Y = OH und a = 4 ist,
X 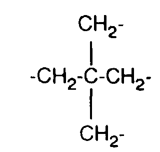 darstellt, und
in der Verbindung der Formel II die Reste
Z Wasserstoff oder eine Gruppe der Formel 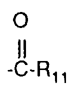 bedeuten,
k die Zahl 1 darstellt, und
R₁₁ C₈-C₂₀-Alkenyl bedeutet, mit der Bedingung, dass die Verbindung der Formel II eine Gruppe 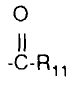 enthält, und
in der Verbindung der Formel III
R₁₂ tert-Butyl bedeutet,
R₁₅ C₁-C₄-Alkyl darstellt und in ortho Stellung zur OH-Gruppe gebunden ist,
s die Zahl 1 bedeutet,
Q -CₘH₂ₘ- darstellt und para zur OH-Gruppe gebunden ist, wobei
m die Zahl 2 bedeutet,
n 1 darstellt, und
R₁₇ C₁-C₄-Alkyl bedeutet,
in der Verbindung der Formel IV
D Schwefel oder 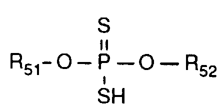 darstellt, und
R₅₁ und R₅₂ unabhängig voneinander C₃-C₈-Alkyl bedeutet.

6. Produkt gemäss Anspruch 1, wobei in der Verbindung der Formel IV
D Schwefel oder 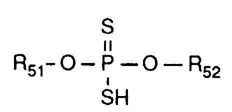 darstellt, und
R₅₁ und R₅₂ unabhängig voneinander C₁-C₁₂-Alkyl, unsubstituiertes oder durch C₁-C₈-Alkyl substituiertes Phenyl bedeutet.

7. Produkt gemäss Anspruch 1,
wobei in der Verbindung der Formel III
R₁₂ C₁-C₄-Alkyl oder Cyclohexyl bedeutet,
R₁₅ C₁-C₄-Alkyl oder Cyclohexyl darstellt und in ortho Stellung zur OH-Gruppe gebunden ist,
s die Zahl 1 bedeutet,
Q -CₘH₂ₘ- darstellt und para zur OH-Gruppe gebunden ist, wobei
m eine ganze Zahl aus dem Bereich von 0 bis 3, und
n eine ganze Zahl aus dem Bereich von 1 bis 4 bedeuten, wobei
wenn n = 1 ist,
R₁₇ Wasserstoff, C₁-C₁₀-Alkyl, Cyclohexyl, C₂-C₁₈-Alkenyl oder 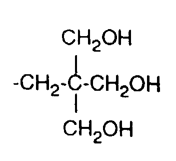 darstellt, oder
wenn n = 2 ist,
R₁₇ 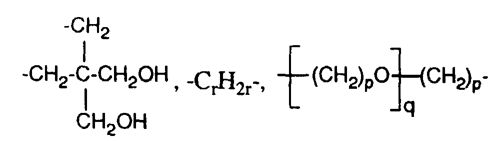 oder -CH₂CH₂-T₄-CH₂CH₂- bedeutet, wobei
p eine ganze Zahl aus dem Bereich von 2 bis 4 darstellt,
q eine ganze Zahl aus dem bereich von 2 bis 10 bedeutet,
r eine ganze Zahl aus dem Bereich von 2 bis 6 darstellt,
T₄ Schwefel oder 〉N-R₂₆ bedeutet und
R₂₆ Wasserstoff oder C₁-C₄-Alkyl bedeutet, oder
wenn n = 3 ist,
R₁₇ 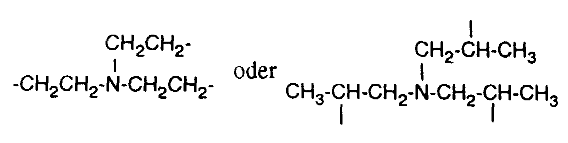 darstellt, oder
wenn n = 4 ist,
R₁₇ 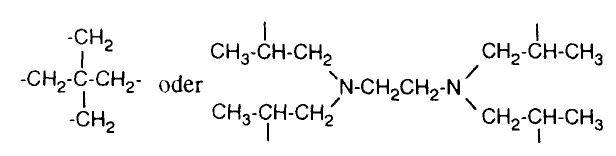 darstellt.

8. Produkt gemäss Anspruch 1,
wobei in der Verbindung der Formel III
R₁₂ tert-Butyl bedeutet,
R₁₅ C₁-C₄-Alkyl darstellt und in ortho Stellung zur OH-Gruppe gebunden ist,
s die Zahl 1 bedeutet,
Q -CₘH₂ₘ- darstellt und para zur OH-Gruppe gebunden ist, wobei
m die Zahl 2 bedeutet und
n eine ganze Zahl 1,2 oder 4 bedeutet, wobei
wenn n = 1 ist,
R₁₇ C₁-C₄-Alkyl bedeutet, oder
wenn n = 2 ist,
R₁₇ 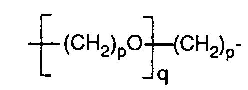 oder -CH₂CH₂-T₄-CH₂CH₂- bedeutet, wobei
p die Zahl 2,
q die Zahl 2 und
T₄ Schwefel bedeuten, oder
wenn n = 4 ist,
R₁₇ 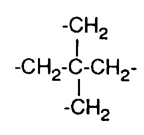 darstellt.

9. Produkt gemäss Anspruch 1, wobei die Verbindung der Formel I Pentaerythrit, Thiodiethylenglykol, 1,4-Butandiol, 1,2-Propandiol, Diethylenglykol, Triethylenglykol, Diethanolamin oder Glycerin bedeutet, die Verbindung der Formel II Sonnenblumenöl, Kokosfett, Rapsöl, Maiskeimöl, Distelöl, Olivenöl, Erdnussöl oder Radiamuls darstellt, die Verbindung der Formel III 3-(3',5'-Di-tert-butyl-4'-hydroxyphenyl)propionsäuremethylester oder 3-(3'-tert-Butyl-4'-hydroxy-5'-methylphenyl)-propionsäuremethylester bedeutet, und die Verbindung der Formel IV Schwefel oder Dithiophosphorsäurediisopropylester darstellt.

10. Produkt gemäss Anspruch 1, worin das molare Mengenverhältnis der Komponenten a), b), c) und d) 0,1:1:0,1:0,1 bis 15:1:30:10 beträgt.

11. Produkt gemäss Anspruch 1, worin der Gewichtsanteil der Wirkgruppe E-2 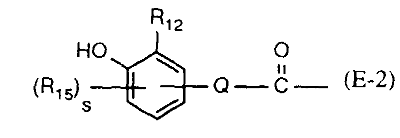 30 bis 80 Gew.-% beträgt.

12. Produkt gemäss Anspruch 1, wobei zunächst die Komponenten a) und b) miteinander umgesetzt werden und anschliessend das so erhaltene Zwischenprodukt mit der Komponente c) und dann mit der Komponente d) zur Reaktion gebracht wird.

13. Zusammensetzung, enthaltend
α) ein dem oxidativen, thermischen oder lichtinduzierten Abbau unterworfenes organisches Material und
β) mindestens ein Produkt gemäss Anspruch 1.

14. Zusammensetzung gemäss Anspruch 13, worin die Komponente α) ein Schmierstoff, eine Hydraulikflüssigkeit, eine Metallbearbeitungsflüssigkeit oder ein synthetisches Polymer ist.

15. Zusammensetzung gemäss Anspruch 13, worin die Komponente α) ein Schmierstoff aus der Reihe der Mineralöle, der synthetischen Oele oder einer Mischung davon ist.

16. Zusammensetzung gemäss Anspruch 13, worin die Komponente α) ein synthetisches Polymer ist.

17. Zusammensetzung gemäss Anspruch 13, worin die Komponente α) ein Polyolefin oder Styrolcopolymer ist.

18. Zusammensetzung gemäss Anspruch 13, enthaltend neben den Komponenten α) und β) zusätzlich weitere Additive.

19. Zusammensetzung gemäss Anspruch 18, enthaltend als weitere Additive aminische Antioxidantien.

20. Verwendung von Produkten gemäss Anspruch 1 zum Stabilisieren von Polymeren und Oelen gegen oxidativen, thermischen oder lichtinduzierten Abbau.

21. Verfahren zum Stabilisieren von Polymeren oder Oelen gegen oxidativen, thermischen oder lichtinduzierten Abbau, dadurch gekennzeichnet, dass man diesen ein Produkt gemäss Anspruch 1 einverleibt.

22. Verfahren zur Herstellung der Produkte gemäss Anspruch 1, dadurch gekennzeichnet, dass die im Anspruch 1 definierten Komponenten a), b), c) und d) in einem molaren Mengenverhältnis von 0,1:1:0,1:0,1 bis 15:1:30:10 umgesetzt werden.

## Claims

1. A product obtainable by reacting components a), b), c) and d), where component a) is a compound of the formula I or a mixture of compounds of the formula I, component b) is a compound of the formula II or a mixture of compounds of the formula II, component c) is a compound of the formula III or a mixture of compounds of the formula III and component d) is a compound of the formula IV or a mixture of compounds of the formula IV, 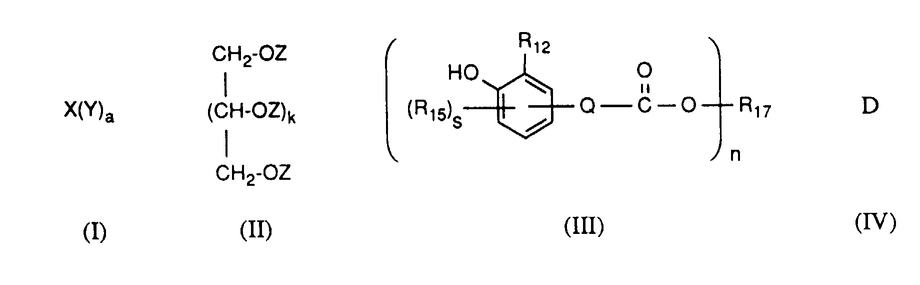 in which, in the compound of the formula I, the radicals
Y independently of one another are OH, (HOCH₂CH₂)₂N- or -HNR₁ and
R₁ is hydrogen, C₁-C₁₈alkyl, C₅-C₁₂cycloalkyl, 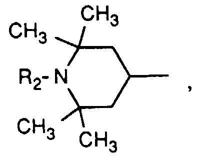 C₃-C₆alkenyl, C₇-C₉phenylalkyl, phenyl, or phenyl which is substituted by 1 to 3 radicals A₁, the radicals A₁ independently of one another being C₁-C₁₂alkyl, halogen, hydroxyl, methoxy or ethoxy, in which
R₂ is hydrogen, C₁-C₈alkyl, O^{•}, OH, NO, -CH₂CN, C₁-C₁₈alkoxy, C₅-C₁₂cycloalkoxy, C₃-C₆alkenyl, C₇-C₉phenylalkyl or C₇-C₉phenylalkyl which is mono-, di- or trisubstituted on the phenyl ring by C₁-C₄alkyl, or R₂ is furthermore C₁-C₈acyl or HOCH₂CH₂-, and a is the number 1, 2, 3, 4 or 6, where,
if Y is OH and a is 1,
X is C₁-C₄₅alkyl, C₃-C₁₈alkenyl, -CH₂CH₂T₁(CH₂CH₂O)_{b}R₄ or 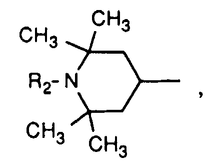 in which R₂ is as defined above, and
T₁ is oxygen, sulfur or 〉N-R₅,
R₄ is C₁-C₂₀alkyl,
b is an integer ranging from 0 to 10 and
R₅ is hydrogen, C₁-C₁₈alkyl or phenyl, or,
if Y is OH and a is 2,
X is -CH₂CH₂T₂(CH₂CH₂O)_{b}CH₂CH₂-, in which b is as defined above, 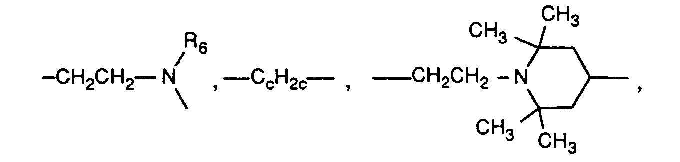 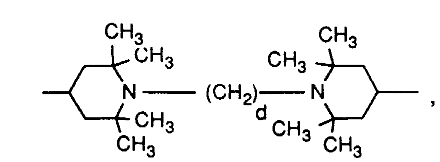 -CH₂-CH=CH-CH₂-, 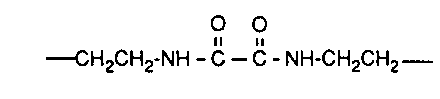 or 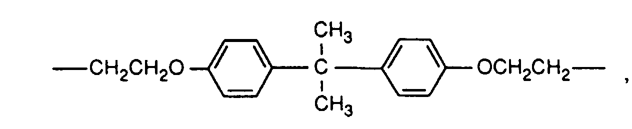 in which
T₂ is oxygen, sulfur, 〉N-R₅ or 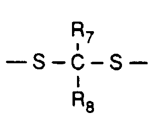 and R₅ is as defined above,
R₆ is hydrogen, C₁-C₁₈alkyl or phenyl,
c is an integer ranging from 2 to 10,
d is an integer ranging from 2 to 6 and
R₇ and R₈ independently of one another are hydrogen, C₁-C₁₈alkyl or phenyl, or R₇ and R₈ together with the C atom to which they are bonded form a C₅-C₁₂cycloalkylidene ring, or
if a is 3,
X is C₃-C₁₀alkanetdyl or N(CH₂CH₂-)₃, or
if Y is OH and a is 4,
X is C₄-C₁₀alkanetetrayl, 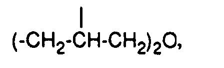 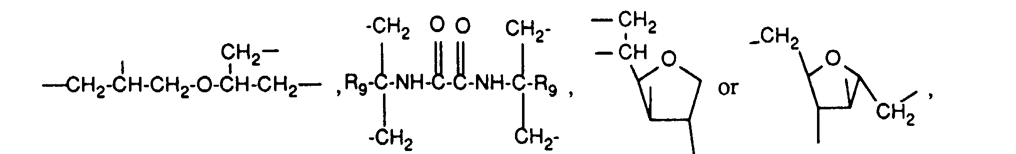 in which
R₉ is C₁-C₄alkyl, or
if Y is OH and a is 6,
X is 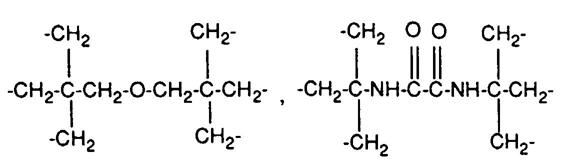 or C₆-C₁₀alkanehexayl, or
if Y is HNR₁ and a is 1,
X is C₁-C₁₈alkyl, C₃-C₁₈alkenyl, C₅-C₁₂cycloalkyl, C₇-C₉phenylalkyl, phenyl, 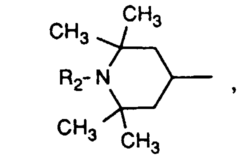 in which R₂ is as defined above or X is furthermore 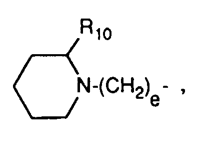 or X together with R₁ is a group of the formula -CH₂CH₂CH₂CH₂CH₂- or -CH₂CH₂OCH₂CH₂-, in which
R₁₀ is hydrogen or methyl and
e is 2 or 3, or
if Y is -HNR₁ and a is 2,
X is -C_{f}H_{2f}-, 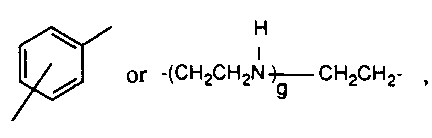 in which
f is an integer ranging from 2 to 10 and
g is an integer ranging from 1 to 6, and,
in the compound of the formula II,
the radicals Z are hydrogen or a group of the formula 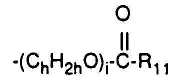 and
k is an integer ranging from 0 to 6, in which
h is 2 or 3,
i is an integer ranging from 0 to 12 and
R₁₁ is C₈-C₃₀alkenyl, with the proviso that the compound of the formula II has a group 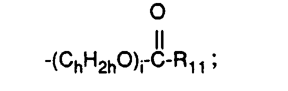 in the compound of the formula III,
R₁₂ is C₁-C₁₈alkyl, C₅-C₁₂cycloalkyl, phenyl or C₇-C₉phenylalkyl,
R₁₅ is hydrogen, C₁-C₁₈alkyl, C₅-C₁₂cycloalkyl, phenyl or C₇-C₉phenylalkyl,
s is 0, 1 or 2,
Q is -CₘH₂ₘ-, 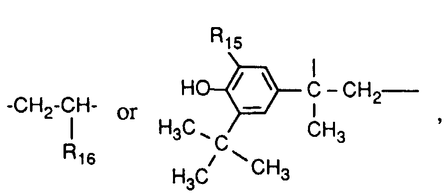 in which R₁₅
is as defined above,
m is an integer ranging from 0 to 3,
R₁₆ is C₁-C₈alkyl and
n is an integer ranging from 1 to 6, where,
if n is 1,
R₁₇ is hydrogen, C₁-C₄₅alkyl, C₅-C₁₂cycloalkyl, C₂-C₁₈alkenyl, a monovalent radical of a hexose, a monovalent radical of a hexitol, 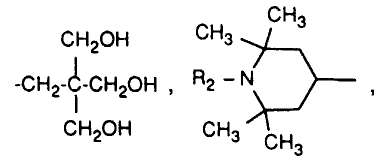 in which
R₂ is as defined above, or furthermore R₁₇ is -CH₂CH₂-T₃-R₁₉ or 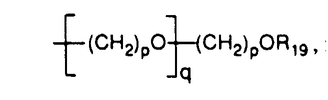 in which
T₃ is oxygen, sulfur or 〉N-R₂₂,
R₁₉ is 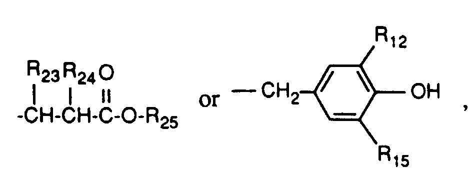 in which R₁₂ and R₁₅ are as defined above, or R₁₉ is furthermore hydrogen, C₁-C₂₄alkyl, phenyl, C₅-C₁₂cycloalkyl or 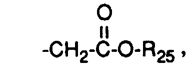 in which
p is an integer ranging from 2 to 4,
q is an integer ranging from 2 to 20,
R₂₂ is C₁-C₁₈alkyl, phenyl or phenyl which is substituted by 1 to 3 radicals A₁, in which the radicals A₁ independently of one another are C₁-C₁₂alkyl, halogen, hydroxyl, methoxy or ethoxy, or R₂₂ is furthermore C₅-C₈cycloalkyl,
R₂₃ and R₂₄ independently of one another are hydrogen or methyl, with the proviso that
R₂₃ and R₂₄ are not simultaneously methyl;
R₂₅ is hydrogen or C₁-C₂₄alkyl, or
if n is 2,
R₁₇ is a divalent radical of a hexose, a divalent radical of a hexitol, 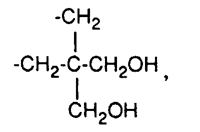 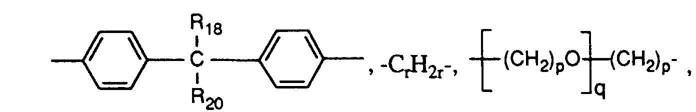 ,in which p and q are as defined above,
-CH₂CH₂-T₄-CH₂CH₂-, -CH₂-CH=CH-CH₂-, CH₂-C≡C-CH2-, 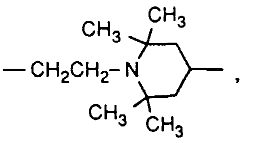 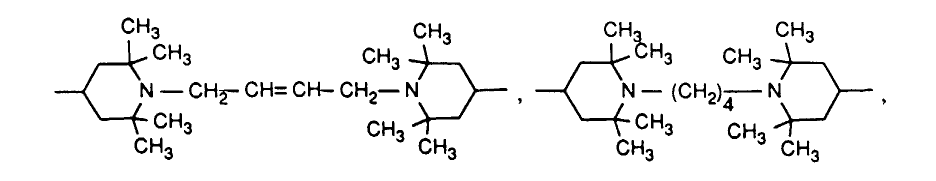 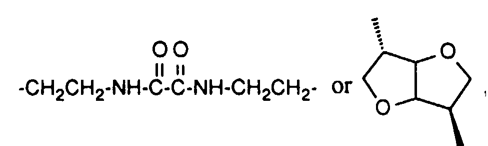 in which
R₁₈ and R₂₀ independently of one another are hydrogen or C₁-C₁₂alkyl or together are the radical -CH₂CH₂CH₂CH₂CH₂-,
r is an integer ranging from 2 to 10,
T₄ is sulfur, 〉N-R₂₆ or 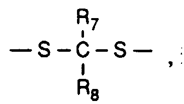 in which R₇ and R₈ are as defined above, and
R₂₆ is hydrogen, C₁-C₁₈alkyl, phenyl or phenyl which is substituted by 1 to 3 radicals A₁, in which the radicals A₁ are as defined above in formula I, or R₂₆ is furthermore C₅-C₈cycloalkyl or 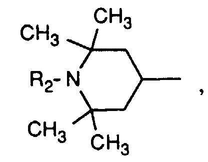 in which R₂ is as defined above, or,
if n is 3,
R₁₇ is a trivalent radical of a hexose, a trivalent radical of a hexitol, 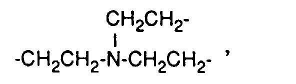 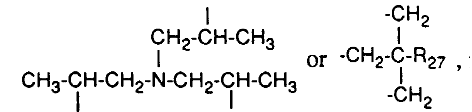 in which
R₂₇ is hydrogen, -CH₂OH, C₁-C₄alkyl, C₁-C₁₈alkylamido or 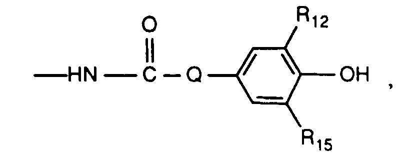 in which Q, R₁₂ and R₁₅ are as defined above, or,
if n is 4,
R₁₇ is a tetravalent radical of a hexose, a tetravalent radical of a hexitol, C₄-C₁₀alkanetetrayl, 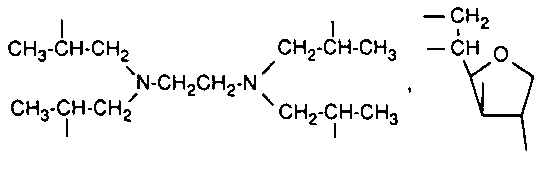 or 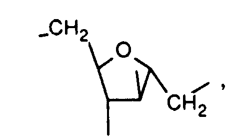
if n is 5,
R₁₇ is a pentavalent radical of a hexose or a pentavalent radical of a hexitol, or,
if n is 6,
R₁₇ is a hexavalent radical of a hexitol or 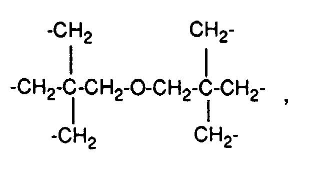 in the compound of the formula IV,
D is sulfur, R₅₀-SH, 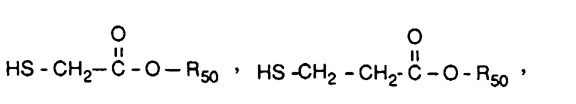 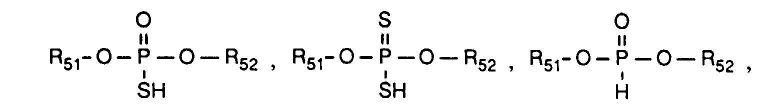 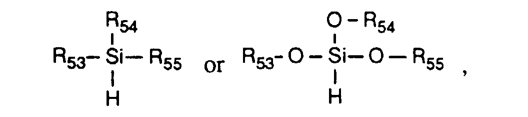
R₅₀ is C₁-C₁₈alkyl, C₅-C₁₂cycloalkyl which is unsubstituted or substituted by C₁-C₄alkyl,
R₅₁ and R₅₂, independently of one another are C₁-C₁₈alkyl, phenyl which is unsubstituted or substituted by C₁-C₁₈alkyl, and
R₅₃, R₅₄ and R₅₅ independently of one another are C₁-C₁₈alkyl or phenyl.

2. A product according to claim 1, in which, in the compound of the formula III, s is the number 1 or 2.

3. A product according to claim 1,
in which, in the compound of the formula I, the radicals
Y independently of one another are OH, (HOCH₂CH₂)₂N- or -HNR₁ and
R₁ is hydrogen, C₁-C₁₀alkyl, C₅-C₇cycloalkyl, 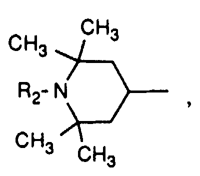 C₃-C₆alkenyl, benzyl or phenyl, in which
R₂ is hydrogen, C₁-C₄alkyl, OH, -CH₂CN, C₆-C₁₂alkoxy, C₅-C₈cycloalkoxy, allyl, benzyl, acetyl or HOCH₂CH₂- and
a is the number 1, 2, 3, 4 or 6, where,
if Y is OH and a is 1,
X is C₁-C₃₀alkyl, C₃-C₁₈alkenyl, -CH₂CH₂T₁(CH₂CH₂O)_{b}R₄ or 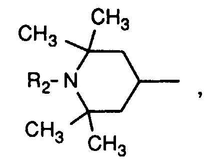 in which R₂ is as defined above, and
T₁ is oxygen, sulfur or 〉N-R₅,
R₄ is C₁-C₁₀alkyl,
b is an integer ranging from 0 to 10 and
R₅ is hydrogen, C₁-C₁₀alkyl or phenyl, or,
if Y is OH and a is 2,
X is -CH₂CH₂T₂(CH₂CH₂O)_{b}CH₂CH₂-, in which b is as defined above, 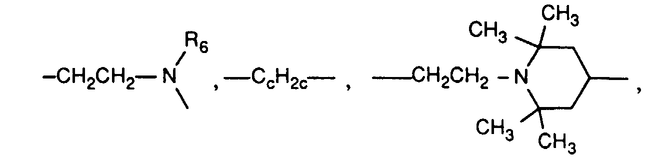 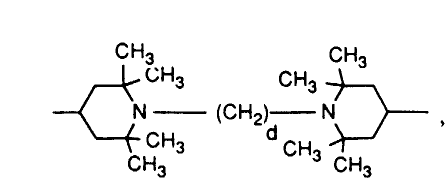 -CH₂-CH=CH-CH₂-, 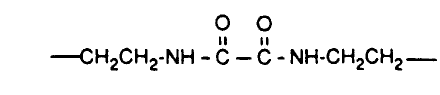 or 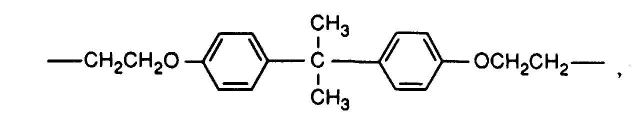 in which
T₂ is oxygen, sulfur, 〉N-R₅ or 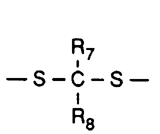 and R₅ is as defined above,
R₆ is hydrogen, C₁-C₁₀alkyl or phenyl,
c is an integer ranging from 2 to 10,
d is an integer ranging from 2 to 6 and
R₇ and R₈ independently of one another are hydrogen, C₁-C₁₀alkyl or phenyl, or R₇ and R₈ together with the C atom to which they are bonded form a C₅-C₇cycloalkyl ring, or,
if Y is -HNR₁ and a is 1,
X is C₁-C₁₀alkyl, C₃-C₁₈alkenyl, C₅-C₇cycloalkyl, benzyl, phenyl, 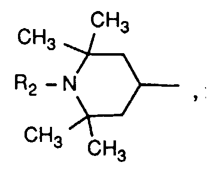 in which R₂ is as defined above, or X is furthermore 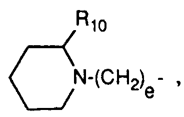 or X together with R₁ is a group of the formula -CH₂CH₂CH₂CH₂CH₂- or -CH₂CH₂OCH₂CH₂-, in which
R₁₀ is hydrogen or methyl and
e is 2 or 3, and
in the compound of the formula II, the radicals
Z are hydrogen or a group of the formula 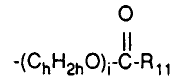 and
k is an integer ranging from 0 to 4, in which
h is 2 or 3,
i is an integer ranging from 0 to 6 and
R₁₁ is C₈-C₂₀alkenyl, with the proviso that the compound of the formula II comprises a group 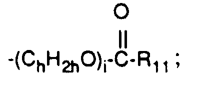 in the compound of the formula III
R₁₂ is C₁-C₆alkyl, C₅-C₇cycloalkyl, phenyl or benzyl
R₁₅ is hydrogen, C₁-C₆alkyl, C₅-C₇cycloalkyl, phenyl or benzyl,
s is 1 or 2,
Q is -CₘH₂ₘ-, 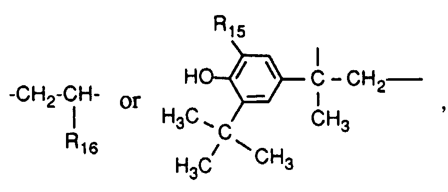 in which R₁₅ is as defined above,
m is an integer ranging from 0 to 3,
R₁₆ is C₁-C₄alkyl and
n is an integer ranging from 1 to 6, where,
if n is 1,
R₁₇ is hydrogen, C₁-C₃₀alkyl, C₅-C₇cycloalkyl, C₂-C₁₈alkenyl, a monovalent radical of a hexose, a monovalent radical of a hexitol, 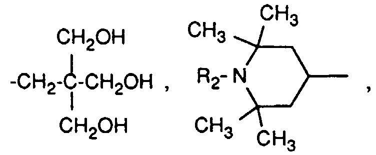 in which
R₂ is as defined above, or furthermore R₁₇ is -CH₂CH₂-T₃-R₁₉ or 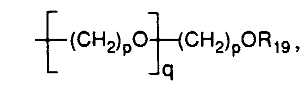 in which
T₃ is oxygen, sulfur or 〉N-R₂₂,
R₁₉ is 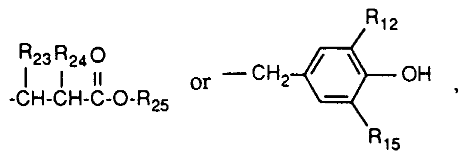 in which R₁₂ and R₁₅ are as defined above, or R₁₉ is furthermore hydrogen, C₁-C₁₈alkyl, phenyl, C₅-C₇cycloalkyl or 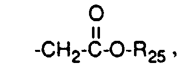 in which
p is an integer ranging from 2 to 4,
q is an integer ranging from 2 to 20,
R₂₂ is C₁-C₁₀alkyl, phenyl or C₅-C₈cycloalkyl,
R₂₃ and R₂₄ independently of one another are hydrogen or methyl with the proviso that
R₂₃ and R₂₄ are not simultaneously methyl;
R₂₅ is hydrogen or C₁-C₁₈alkyl, or,
if n is 2,
R₁₇ is a divalent radical of a hexose, a divalent radical of a hexitol, 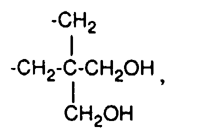 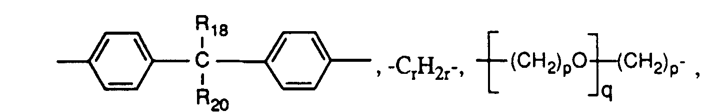 in which p and q are as defined above, -CH₂CH₂-T₄-CH₂CH₂-, -CH₂-CH=CH-CH₂-, -CH₂-C≡C-CH₂-, 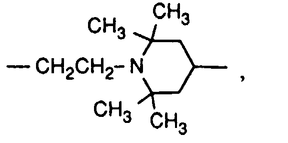 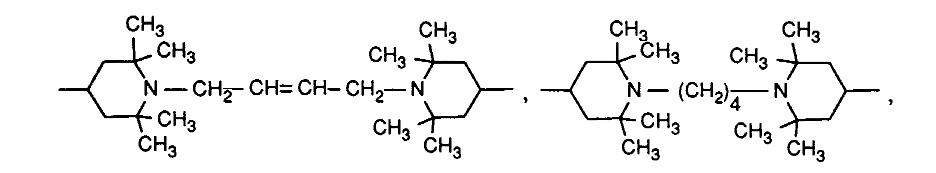 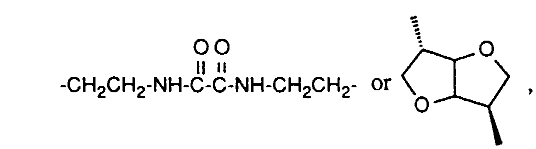 in which
R₁₈ and R₂₀ independently of one another are hydrogen or C₁-C₆alkyl or together are the radical -CH₂CH₂CH₂CH₂CH₂-,
r is an integer ranging from 2 to 10,
T₄ is sulfur, 〉N-R₂₆ or 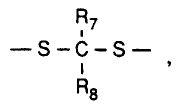 in which R₇ and R₈ are as defined above and
R₂₆ is hydrogen, C₁-C₁₀alkyl, phenyl, C₅-C₈cycloalkyl or 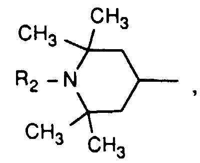 in which R₂ is as defined above,
in the compound of the formula IV,
D is sulfur, R₅₀-SH, 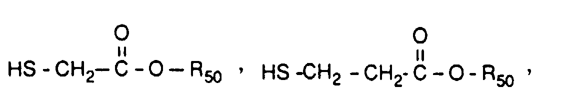 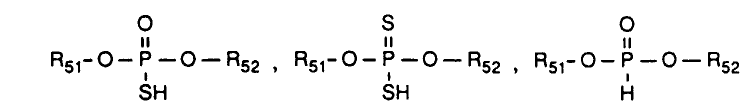 or 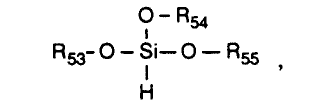
R₅₀ is C₁-C₁₈alkyl or C₅-C₁₂cycloalkyl,
R₅₁ and R₅₂ independently of one another are C₁-C₁₂alkyl, phenyl which is unsubstituted or substituted by C₁-C₁₂alkyl, and
R₅₃, R₅₄ and R₅₅ independently of one another are C₁-C₁₂alkyl or phenyl.

4. A product according to claim 1,
in which, in the compound of the formula I, the radicals
Y independently of one another are OH, (HOCH₂CH₂)₂N- or -HNR₁ and
R₁ is hydrogen, C₁-C₄alkyl or 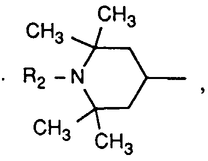 in which
R₂ is hydrogen, C₁-C₄alkyl, OH, allyl, benzyl, acetyl or HOCH₂CH₂- and
a is the number 1, 2, 3, 4 or 6, where
if Y is OH and a is 1,
X is C₁-C₁₈alkyl, C₃-C₁₈alkenyl, -CH₂CH₂T₁(CH₂CH₂O)_{b}R₄ or 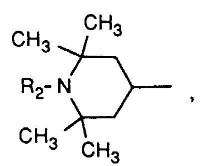 in which R₂ is as defined above, and
T₁ is oxygen,
R₄ is C₁-C₄alkyl and
b is an integer ranging from 0 to 10, or
if Y is OH and a is 2,
X is -CH₂CH₂T₂(CH₂CH₂O)_{b}CH₂CH₂-, in which b is as defined above, or furthermore X is -C_{c}H_{2c}-, 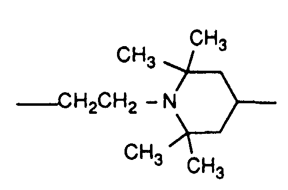 or -CH₂-CH=CH-CH₂-, in which
T₂ is oxygen, sulfur or 〉N-R₅,
R₅ is hydrogen,
b is the number 0 or 1 and
c is an integer ranging from 2 to 8, or,
if a is 3,
X is 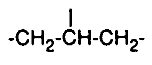 or N(CH₂CH₂-)₃, or,
if Y is OH and a is 4,
X is 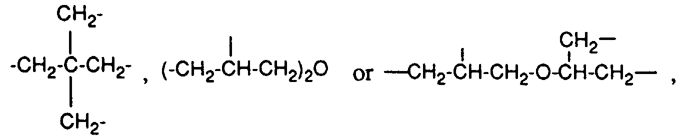 or
if Y is OH and a is 6,
X is 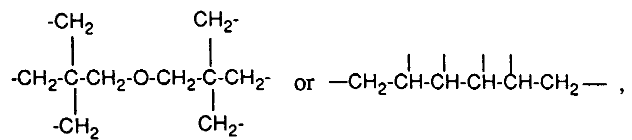 or,
if Y is -HNR₁ and a is 1,
X is C₁-C₁₀alkyl, C₃-C₁₈alkenyl, C₅-C₇cydoalkyl or 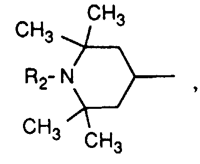 where R₂ is as defined above, or,
if Y is -HNR₁ and a is 2,
X is -C_{f}H_{2f}- in which
f is an integer ranging from 2 to 10 and,
in the compound of the formula II, the radicals
Z are hydrogen or a group of the formula 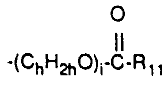 and
k is 1, 2 or 3,
h is 2 or 3,
i is an integer ranging from 0 to 4 and
R₁₁ is C₈-C₂₀alkenyl, with the proviso that the compound of the formula II comprises a group 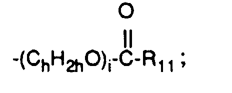 in the compound of the formula III,
R₁₂ is C₁-C₆alkyl or C₅-C₇cycloalkyl,
R₁₅ is hydrogen, C₁-C₆alkyl or C₅-C₇cycloalkyl,
s is 1 or 2,
Q is -CₘH₂ₘ- or 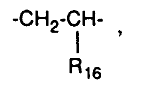
m is an integer ranging from 0 to 3,
R₁₆ is C₁-C₄alkyl and
n is an integer ranging from 1 to 6, where,
if n is 1,
R₁₇ is hydrogen, C₁-C₁₈alkyl, C₅-C₇cycloalkyl, C₂-C₁₈alkenyl, a monovalent radical of a hexose, a monovalent radical of a hexitol, 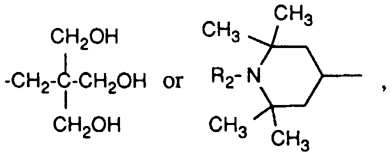 in which
R₂ is as defined above,
or furthermore R₁₇ is 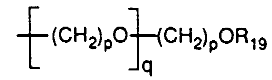 in which
R₁₉ is hydrogen, C₁-C₁₈alkyl or C₅-C₇cycloalkyl, in which
p is an integer ranging from 2 to 4,
q is an integer ranging from 2 to 10, or,
if n is 2,
R₁₇ is a divalent radical of a hexose, a divalent radical of a hexitol, 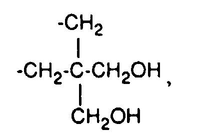 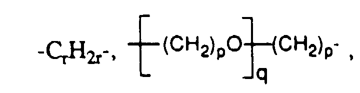 in which p and q are as defined above, -CH₂CH₂-T₄-CH₂CH₂-, or 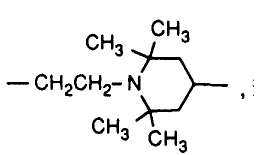 in which
r is an integer ranging from 2 to 10,
T₄ is sulfur or 〉N-R₂₆ and
R₂₆ is hydrogen, C₁-C₁₀alkyl or C₅-C₈cycloalkyl, or,
if n is 3,
R₁₇ is a trivalent radical of a hexose, a trivalent radical of a hexitol, 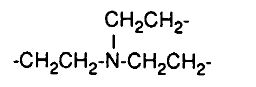 or 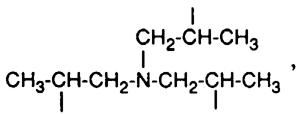 or,
if n is 4,
R₁₇ is a tetravalent radical of a hexose, a tetravalent radical of a hexitol, 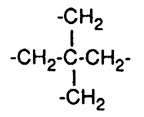 or 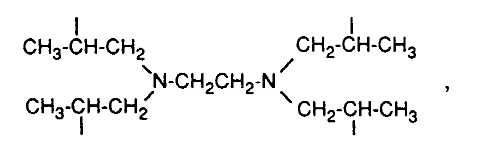 in the compound of the formula IV,
D is sulfur, 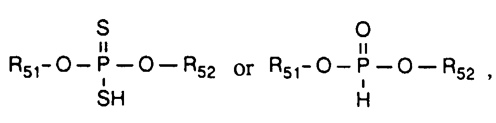 and
R₅₁ and R₅₂ independently of one another are C₁-C₁₂alkyl, phenyl which is unsubstituted or substituted by C₁-C₈alkyl.

5. A product according to claim 1,
in which, in the compound of the formula I, the radicals
Y independently of one another are hydroxyl or -NH₂ and
a is an integer ranging from 1 to 4, where
if a is 1,
X is 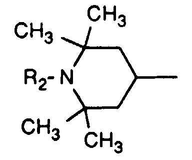 and
R₂ is hydrogen, methyl or HOCH₂CH₂-, or
if Y is OH and a is 2,
X is -CH₂CH₂T₂(CH₂CH₂O)_{b}CH₂CH₂-, -C_{c}H_{2c}- or 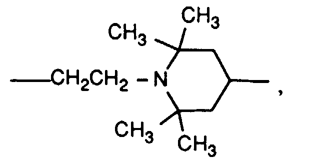 in which
T₂ is oxygen, sulfur or 〉N-R₅,
R₅ is hydrogen,
b is the number 0 or 1 and
c is the number 2, 3 or 4, or,
if Y is OH and a is 3,
X is 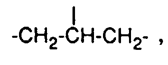 or,
if Y is OH and a is 4,
X is 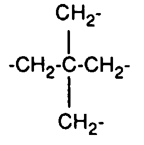 and,
in the compound of the formula II, the radicals
Z are hydrogen or a group of the formula 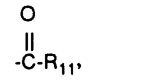
k is the number 1 and
R₁₁ is C₈-C₂₀alkenyl, with the proviso that the compound of the formula II comprises a group 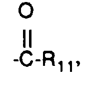 and,
in the compound of the formula III,
R₁₂ is tert-butyl,
R₁₅ is C₁-C₄alkyl and is bonded in the ortho-position relative to the OH group,
s is the number 1,
Q is -CₘH₂ₘ- and is bonded in the para-position relative to the OH group, where,
m is the number 2,
n is 1 and
R₁₇ is C₁-C₄alkyl,
in the compound of the formula IV,
D is sulfur or 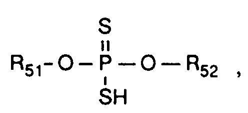 and
R₅₁ and R₅₂ independently of one another are C₃-C₈alkyl.

6. A product according to claim 1, in which in the formula IV,
D is sulfur or 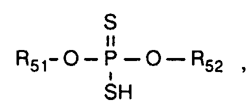 and
R₅₁ and R₅₂ independently of one another are C₁-C₁₂alkyl, phenyl which is unsubstituted or substituted by C₁-C₈alkyl.

7. A product according to claim 1,
in which, in the compound of the formula III,
R₁₂ is C₁-C₄alkyl or cyclohexyl,
R₁₅ is C₁-C₄alkyl or cyclohexyl and is bonded in the ortho-position relative to the OH group,
s is the number 1,
Q is -CₘH₂ₘ- and is bonded in the para-position relative to the OH group, where
m is an integer ranging from 0 to 3 and
n is an integer ranging from 1 to 4, where
if n is 1,
R₁₇ is hydrogen, C₁-C₁₀alkyl, cyclohexyl, C₂-C₁₈alkenyl or 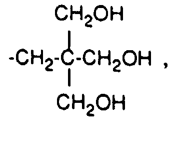 or,
if n is 2,
R₁₇ is 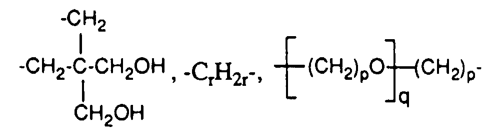 or -CH₂CH₂-T₄-CH₂CH₂- in which
p is an integer ranging from 2 to 4,
q is an integer ranging from 2 to 10,
r is an integer ranging from 2 to 6,
T₄ is sulfur or 〉N-R₂₆ and
R₂₆ is hydrogen or C₁-C₄alkyl, or,
if n is 3,
R₁₇ is 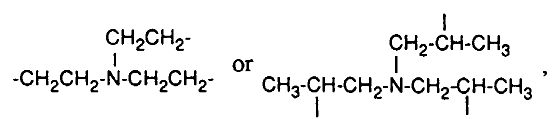 or,
if n is 4,
R₁₇ is

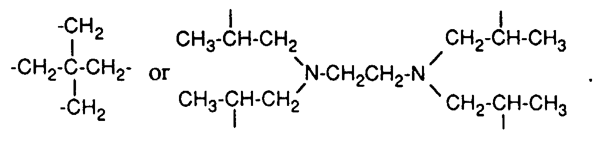

8. A product according to claim 1,
in which, in the compound of the formula III,
R₁₂ is tert-butyl,
R₁₅ is C₁-C₄alkyl and is bonded in the ortho-position relative to the OH group,
s is the number 1,
Q is -CₘH₂ₘ- and is bonded in the para-position relative to the OH group, in which
n is the number 2 and
n is an integer 1, 2 or 4, where,
if n is 1,
R₁₇ is C₁-C₄alkyl, or,
if n is 2,
R₁₇ is 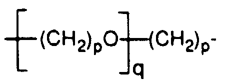 or -CH₂CH₂-T₄-CH₂CH₂-, in which
p is the number 2,
q is the number 2 and
T₄ is sulfur, or,
if n is 4,
R₁₇ is

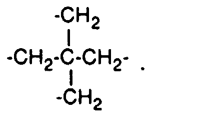

9. A product according to claim 1, in which the compound of the formula I is pentaerythritol, thiodiethylene glycol, 1,4-butanediol, 1,2-propanediol, diethylene glycol, triethylene glycol, diethanolamine or glycerol, the compound of the formula II is sunflower oil, coconut fat, rapeseed oil, maize germ oil, safflower oil, olive oil, groundnut oil or Radiamuls, and the compound of the formula III is methyl 3-(3',5'-di-tert-butyl-4'-hydroxyphenyl)propionate or methyl 3-(3'-tert-butyl-4'-hydroxy-5'-methylphenyl)propionate, and the compound of the formula IV is sulfur or diisopropyl dithiophosphate.

10. A product according to claim 1, in which the molar quantitative ratio of components a), b), c) and d) is 0.1:1:0.1:0.1 to 15:1:30:10.

11. A product according to claim 1, in which the amount by weight of active group E-2 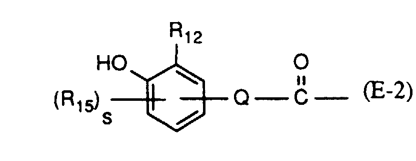 is 30 to 80 % by weight.

12. A product according to claim 1, in which first components a) and b) are reacted with each other and the resulting intermediate is subsequently reacted with component c) and then with component d).

13. A composition comprising α) an organic material subjected to oxidative, thermal or light-induced degradation and β) at least one product according to claim 1.

14. A composition according to claim 13, in which component α) is a lubricant, a hydraulic fluid, a metal-working fluid or a synthetic polymer.

15. A composition according to claim 13, in which component α) is a lubricant from the series of the mineral oils, the synthetic oils or a mixture of these.

16. A composition according to claim 13, in which component α) is a synthetic polymer.

17. A composition according to claim 13, in which component α) is a polyolefin or a styrene copolymer.

18. A composition according to claim 13 which contains, in addition to components α) and β), additional further additives.

19. A composition according to claim 18 which contains amine antioxidants as further additives.

20. The use of a product according to claim 1 for stabilising polymers and oils against oxidative, thermal or light-induced degradation..

21. A process for stabilising polymers or oils against oxidative, thermal or light-induced degradation, which comprises incorporating a product according to claim 1 into these polymers or oils.

22. A process for the preparation of a product according to claim 1, which comprises reacting the components a), b), c) and d) which have been defined in claim 1 in a molar quantitative ratio of 0.1:1:0.1:0.1 to 15:1:30:10.

## Revendications

1. Produit, qu'on peut obtenir par réaction des composants a), b), c) et d), dans lequel le composant a) est un composé de formule I ou un mélange de composés de formule I, le composant b) un composé de formule II ou un mélange de composés de formule II, le composant c) un composé de formule III ou un mélange de composés de formule III, et le composant d) un composé de formule IV ou un mélange de composés de formule IV, 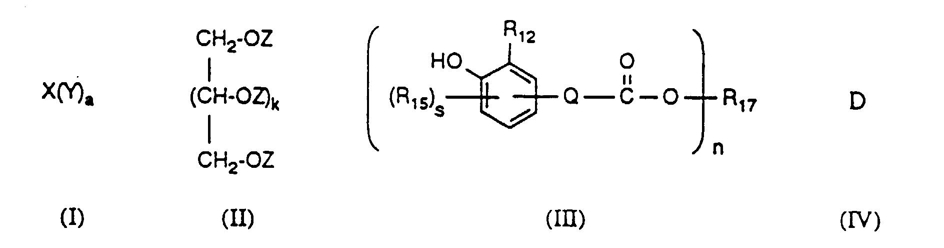 dans lesquelles dans le composé de formule I les groupes
Y représentent indépendamment les uns des autres OH, (HOCH₂CH₂)₂N- ou -HNR₁ et
R₁ représente un atome d'hydrogène, un groupe alkyle en C₁-C₁₈, cycloalkyle en C₅-C₁₂, 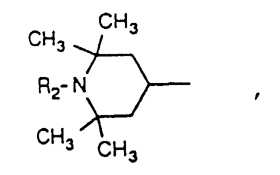 alcényle en C₃-C₆, phénylalkyle en C₇-C₉, phényle ou phényle substitué par 1 à 3 groupes A₁, les groupes A₁ représentant indépendamment les uns des autres un groupe alkyle en C₁-C₁₂, un atome d'halogène, un groupe hydroxy, méthoxy ou éthoxy, dans laquelle
R₂ représente un atome d'hydrogène, un groupe alkyle en C₁-C₈, O^{•}, OH, NO, -CH₂CN, un groupe alcoxy en C₁-C₁₈, cycloalcoxy en C₅-C₁₂, alcényle en C₃-C₆, phénylalkyle en C₇-C₉, ou phénylalkyle en C₇-C₉ qui est mono, di ou trisubstitué sur le noyau phényle avec un groupe alkyle en C₁-C₄ ou R₂ représente en outre un groupe acyle en C₁-C₈ ou le groupe HOCH₂CH₂-, et
a représente le nombre 1, 2, 3, 4 ou 6, dans laquelle lorsque Y = OH et a = 1,
X représente un groupe alkyle en C₁-C₄₅, alcényle en C₃-C₁₈, -CH₂CH₂T₁(CH₂CH₂O)_{b}R₄ ou 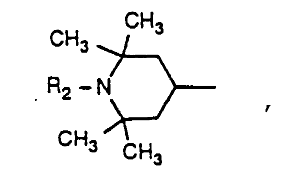 dans laquelle R₂ a la signification ci-dessus, et
T₁ réprésente un atome d'oxygène, de soufre ou 〉N-R₅,
R₄ représente un groupe alkyle en C₁-C₂₀,
b un nombre entier dans l'intervalle de 0 à 10 et
R₅ représente un atome d'hydrogène, un groupe alkyle en C₁-C₁₈ ou phényle, ou
lorsque Y = OH et a = 2,
X représente un groupe -CH₂CH₂T₂(CH₂CH₂O)_{b}CH₂CH₂-, dans lequel b a la signification ci-dessus, 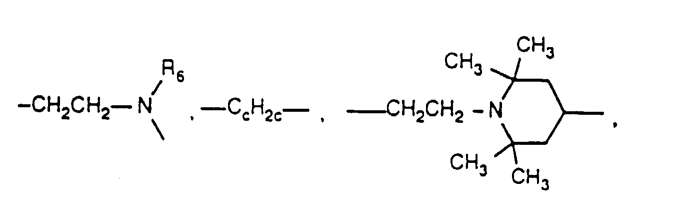 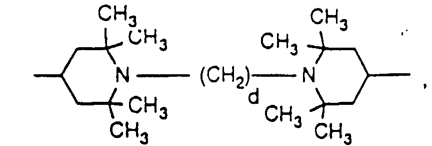 -CH₂-CH=CH-CH₂-, 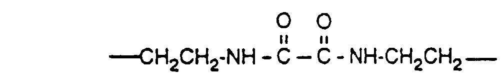 ou 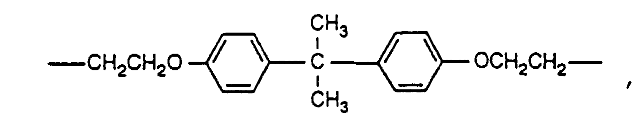 dans lesquelles
T₂ représente un atome d'oxygène, de soufre, 〉N-R₅, ou 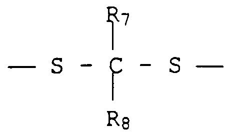 et R₅ a la signification ci-dessus,
R₆ représente un atome d'hydrogène, un groupe alkyle en C₁-C₁₈ ou phényle,
c représente un nombre entier dans l'intervalle de 2 à 10,
d un un nombre entier dans l'intervalle de 2 à 6 et
R₇, R₈ représentent indépendamment l'un de l'autre un atome d'hydrogène, un groupe alkyle en C₁-C₁₈ ou phényle, ou R₇ et R₈ forment avec l'atome de carbone auquel ils sont liés, un cycle cycloalkylidène en C₅-C₁₂, ou
lorsque a = 3,
X représente un groupe alcanetriyle en C₃-C₁₀ ou N(CH₂CH₂-)₃, ou
lorsque Y = OH et a = 4,
X représente un groupe alcanetétrayle en C₄-C₁₀, 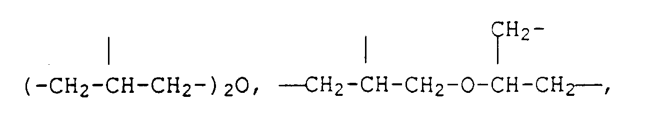 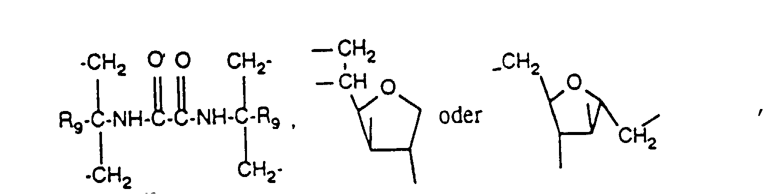
dans lequel
R₉ représente un groupe alkyle en C₁-C₄, ou
lorsque Y = OH et a = 6,
X représente 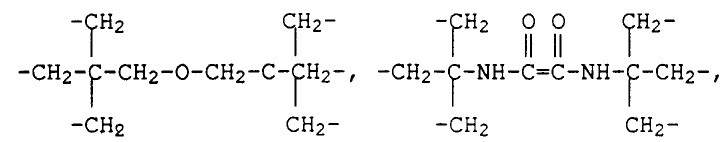 ou un groupe alcanehexayle, ou
lorsque Y = -HNR₁ et a = 1,
X représente un groupe alkyle en C₁-C₁₈, alcényle en C₃-C₁₈, cycloalkyle en C₅-C₁₂, phénylalkyle en C₇-C₉, phényle, 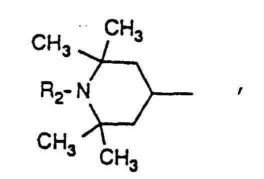 dans laquelle R₂ a la signification ci-dessus, ou X est en outre 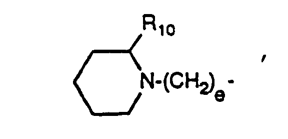 ou X forme ensemble avec R₁ un groupe de formule -CH₂CH₂CH₂CH₂CH₂- ou -CH₂CH₂OCH₂CH₂-, dans lequel
R₁₀ représente un atome d'hydrogène ou un groupe méthyle, et
e représente 2 ou 3, ou
lorsque Y = -HNR₁ et a = 2,
X représente -C_{f}H_{2f}-, 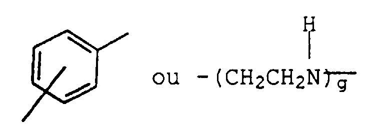 CH₂CH₂-, formules dans lesquelles
f représente un nombre entier dans l'intervalle de 2 à 10 et
g un un nombre entier dans l'intervalle de 1 à 6, et
dans le composé de formule II,
Z représente un atome d'hydrogène ou un groupe de formule 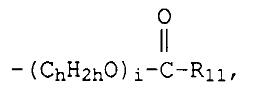 et
k représente un nombre entier de 0 à 6, formule dans laquelle
h est 2 ou 3,
i un nombre entier dans l'intervalle de 0 à 12 et
R₁₁ représente un groupe alcényle en C₈-C₃₀, à la condition que le composé de formule II contienne un groupe 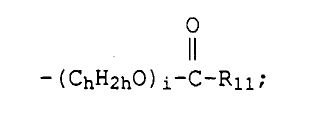
dans le composé de formule III
R₁₂ représente un groupe alkyle en C₁-C₁₈, cycloalkyle en C₅-C₁₂, phényle ou phénylalkyle en C₇-C₉,
R₁₅ représente un atome d'hydrogène, un groupe alkyle en C₁-C₁₈, cycloalkyle en C₅-C₁₂, phényle ou phénylalkyle en C₇-C₉,
s représente 0, 1 ou 2,
Q représente -CₘH₂ₘ-, 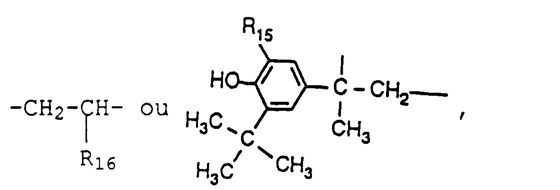 dans laquelle R₁₅ a la signification ci-dessus,
m un nombre entier dans l'intervalle de 0 à 3 et
R₁₆ un groupe alkyle en C₁-C₈ et
n un nombre entier dans l'intervalle de 1 à 6, formule dans laquelle
lorsque n = 1,
R₁₇ représente un atome d'hydrogène, un groupe alkyle en C₁-C₄₅, cycloalkyle en C₅-C₁₂, alcényle en C₂-C₁₈, un résidu monovalent d'un hexose, un résidu monovalent d'un hexitol, 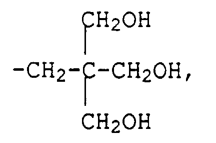 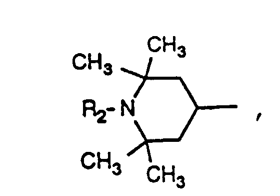 formule dans laquelle R₂ a la signification ci-dessus, ou R₁₇ représente en outre -CH₂CH₂-T₃R₁₉ ou 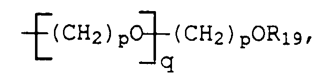 formules dans lesquelles
T₃ représente un atome d'oxygène, de soufre, 〉N-R₂₂,
R₁₉ 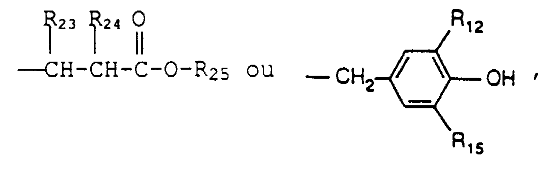 dans laquelle
R₁₂ et R₁₅ ont la signification ci-dessus, ou R₁₉ représente en outre un atome d'hydrogène, un groupe alkyle en C₁-C₂₄, phényle, cycloalkyle en C₅-C₁₂ ou 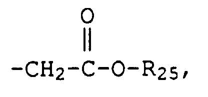 dans laquelle
p représente un nombre entier dans l'intervalle de 2 à 4,
q un nombre entier dans l'intervalle de 2 à 20,
R₂₂ représente un groupe alkyle en C₁-C₁₈, phényle ou phényle substitué par 1 à 3 groupes A₁, les groupes A₁ représentant indépendamment les uns des autres un groupe alkyle en C₁-C₁₂, un atome d'halogène, un groupe hydroxy, méthoxy ou éthoxy, ou R₂₂ représente en outre un groupe cycloalkyle en C₅-C₈,
R₂₃, R₂₄ représentent indépendamment l'un de l'autre un atome d'hydrogène ou un groupe méthyle à condition que R₂₃ et R₂₄ ne soient pas en même temps le groupe méthyle;
R₂₅ représente un atome d'hydrogène ou un groupe alkyle en C₁-C₂₄, ou
lorsque n = 2,
R₁₇ représente un groupe bivalent d'un hexose, un groupe bivalent d'un hexitol, 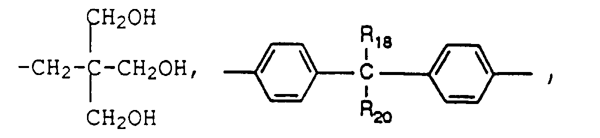 -CᵣH₂ᵣ-, 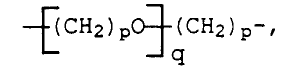 formules dans lesquelles p et q ont la signification ci-dessus,
-CH₂CH₂-T₄-CH₂CH₂-, -CH₂-CH=CH-CH₂-, -CH₂-C≡C-CH₂-, 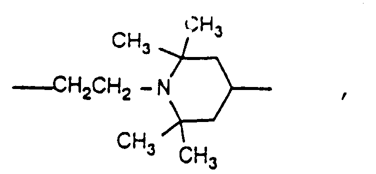 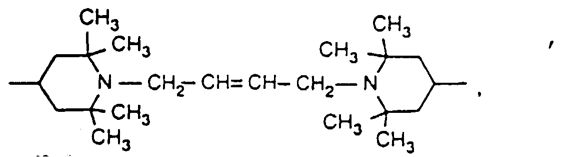 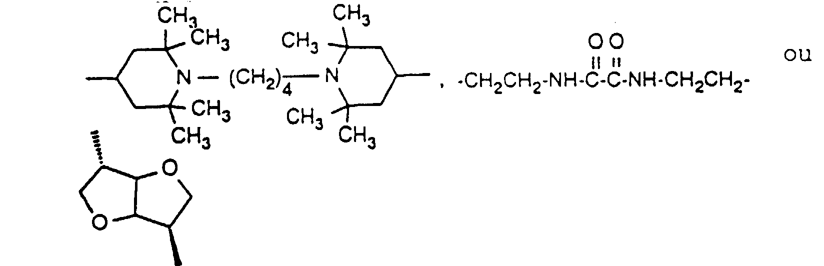 formules dans lesquelles
R₁₈, R₂₀ représentent indépendamment l'un de l'autre un atome d'hydrogène ou un groupe alkyle en C₁-C₁₂, ou forment ensemble le groupe -CH₂CH₂CH₂CH₂CH₂-,
r représente un nombre entier dans l'intervalle de 2 à 10,
T₄ représente un atome de soufre, 〉N-R₂₆ ou 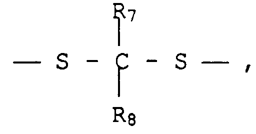 et dans lesquelles R₇ et R₈ ont le signification ci-dessus, et
R₂₆ représente un atome d'hydrogène, un groupe alkyle en C₁-C₁₈, phényle ou phényle substitué par 1 à 3 groupes A₁, les groupes A₁ ayant la signification décrite pour la formule I, ou R₂₆ représente en outre un groupe cycloalkyle en C₅-C₈ ou 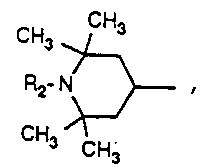 dans laquelle R₂ a la signification ci-dessus, ou
lorsque n = 3,
R₁₇ représente un résidu trivalent d'un hexose, un résidu trivalent d'un hexitol, 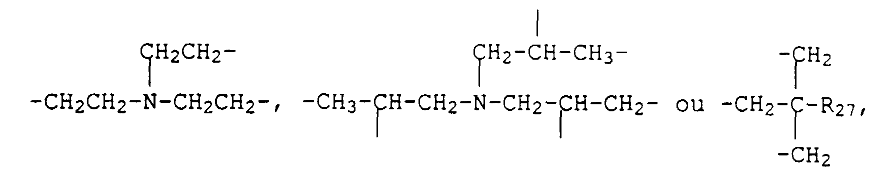 dans laquelle
R₂₇ représente un atome d'hydrogène, -CH₂OH, un groupe alkyle en C₁-C₄, alkylamido en C₁-C₁₈ ou 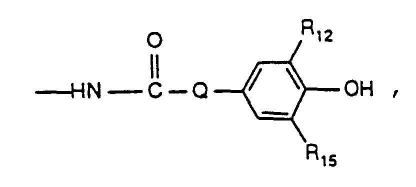 dans laquelle Q, R₁₂ et R₁₅ ont la signification ci-dessus, ou
lorsque n = 4,
R₁₇ représente un résidu tétravalent d'un hexose, un résidu tétravalent d'un hexitol, un groupe alcanetétrayle en C₄-C₁₀, 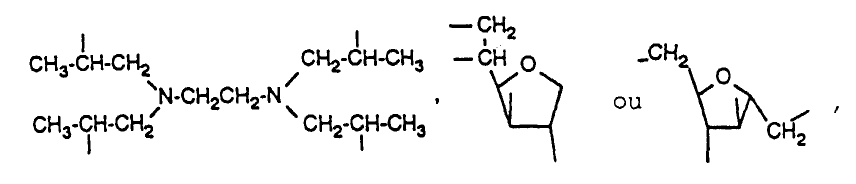 ou
lorsque n = 5,
R₁₇ représente un résidu pentavalent d'un hexose, un résidu pentavalent d'un hexitol, ou
lorsque n = 6,
R₁₇ représente un résidu hexavalent d'un hexitol, ou 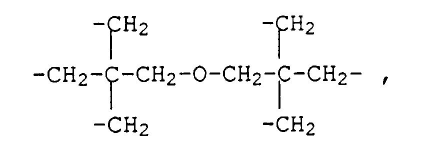
dans le composé de formule IV
D représente un atome de soufre, R₅₀-SH, 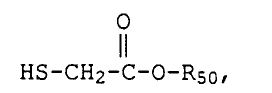 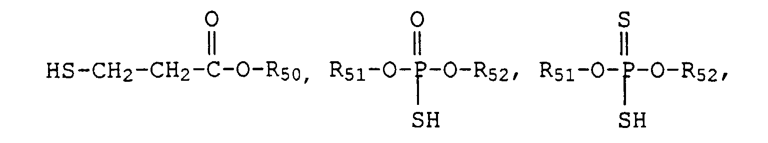 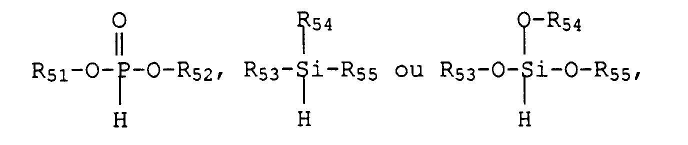
R₅₀ représente un groupe alkyle en C₁-C₁₈, cycloalkyle en C₅-C₁₂ non substitué ou substitué par un groupe alkyle en C₁-C₄,
R₅₁ et R₅₂ représentent indépendamment l'un de l'autre un groupe alkyle en C₁-C₁₈, phényle non substitué ou substitué par un groupe alkyle en C₁-C₁₈, et
R₅₃, R₅₄ et R₅₅ représentent indépendamment les uns des autres un groupe alkyle en C₁-C₁₈ ou phényle.

2. Produit selon la revendication 1, dans lequel dans le composé de formule III, s représente le nombre 1 ou 2.

3. Produit selon la revendication 1,
dans lequel dans le composé de formule I les groupes
Y représentent indépendamment les uns des autres OH, (HOCH₂CH₂)₂N- ou -HN_{R1} et
R₁ représente un atome d'hydrogène, un groupe alkyle en C₁-C₁₀, cycloalkyle en C₅-C₇, 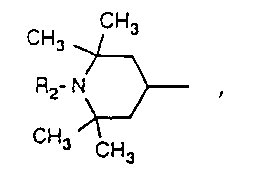 alcényle en C₃-C₆, benzyle ou phényle, formule dans laquelle
R₂ représente un atome d'hydrogène, un groupe alkyle en C₁-C₄, OH, -CH₂CN, alcoxy en C₆-C₁₂, cycloalcoxy en C₅-C₈, allyle, benzyle, acétyle ou HOCH₂CH₂-, et
a représente le nombre 1, 2, 3, 4 ou 6, dans lesquels
lorsque Y = OH et a = 1,
X représente un groupe alkyle en C₁-C₃₀, alcényle en C₃-C₁₈, -CH₂CH₂T₁(CH₂CH₂O)_{b}R₄ ou 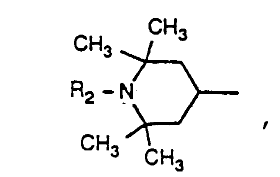 dans laquelle R₂ a la signification ci-dessus, et
T₁ représente un atome d'oxygène, de soufre ou 〉N-R₅,
R₄ représente un groupe alkyle en C₁-C₁₀,
b un nombre entier dans l'intervalle de 0 à 10 et
R₅ représente un atome d'hydrogène, un groupe alkyle en C₁-C₁₀ ou phényle, ou
lorsque Y = OH et a = 2,
X représente un groupe -CH₂CH₂T₂ (CH₂CH₂O)_{b}CH₂CH₂-, dans lequel b a la signification ci-dessus, 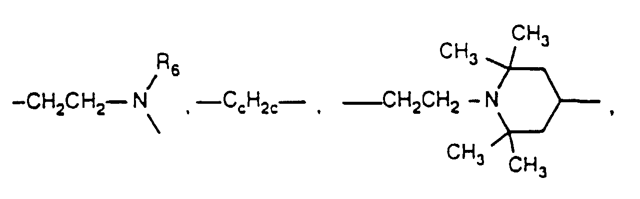 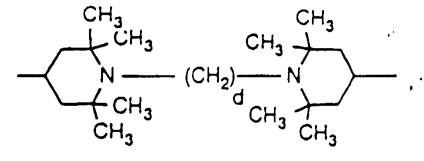 -CH₂-CH=CH-CH₂-, 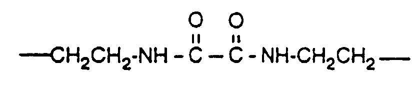 ou 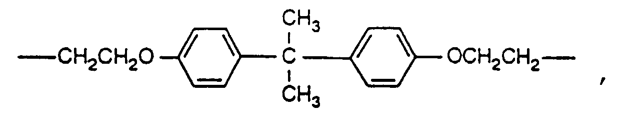 dans lesquelles
T₂ représente un atome d'oxygène, de soufre, 〉N-R₅, ou 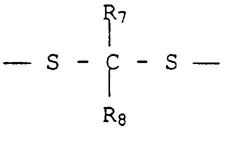 et R₅ a la signification ci-dessus,
R₆ représente un atome d'hydrogène, un groupe alkyle en C₁-C₁₀ ou phényle,
c représente un nombre entier dans l'intervalle de 2 à 10,
d un nombre entier dans l'intervalle de 2 à 6 et
R₇, R₈ représentent indépendamment l'un de l'autre un atome d'hydrogène, un groupe alkyle en C₁-C₁₀ ou phényle, ou R₇ et R₈ forment avec l'atome de carbone auquel ils sont liés, un cycle cycloalkyle en C₅-C₇, ou
lorsque Y = -HNR₁ et a = 1,
X représente un groupe alkyle en C₁-C₁₀, alcényle en C₃-C₁₈, cycloalkyle en C₅-C₇, benzyle, phényle, 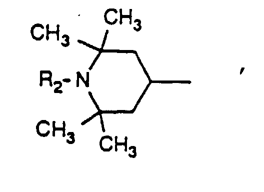 dans laquelle R₂ a la signification ci-dessus, ou X est en outre 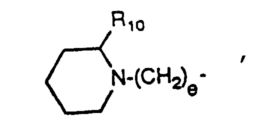 ou X forme ensemble avec R₁ un groupe de formule -CH₂CH₂CH₂CH₂CH₂- ou -CH₂CH₂OCH₂CH₂-, dans lequel
R₁₀ représente un atome d'hydrogène ou un groupe méthyle, et
e représente 2 ou 3, ou
dans le composé de formule II,
Z représente un atome d'hydrogène ou un groupe de formule 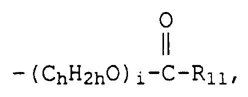 et
k représente un nombre entier dans l'intervalle de 0 à 4, formule dans laquelle
h est 2 ou 3,
i un nombre entier dans l'intervalle de 0 à 6 et
R₁₁ représente un groupe alcényle en C₈-C₂₀, à la condition que le composé de formule II contienne un groupe 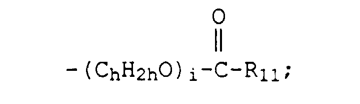
dans le composé de formule III
R₁₂ représente un groupe alkyle en C₁-C₆, cycloalkyle en C₅-C₇, phényle ou benzyle,
R₁₅ représente un atome d'hydrogène, un groupe alkyle en C₁-C₆, cycloalkyle en C₅-C₇, phényle ou benzyle,
s représente 1 ou 2,
Q représente -CₘH₂ₘ-, 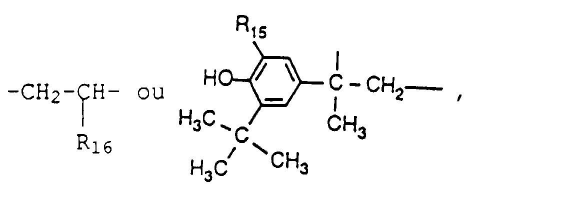 dans laquelle R₁₅ a la signification ci-dessus,
m un nombre entier dans l'intervalle de 0 à 3 et
R₁₆ un groupe alkyle en C₁-C₄ et
n un nombre entier dans l'intervalle de 1 à 6, formule dans laquelle
lorsque n = 1,
R₁₇ représente un atome d'hydrogène, un groupe alkyle en C₁-C₃₀, cycloalkyle en C₅-C₇, alcényle en C₂-C₁₈, un résidu monovalent d'un hexose, un résidu monovalent d'un hexitol, 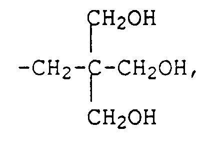 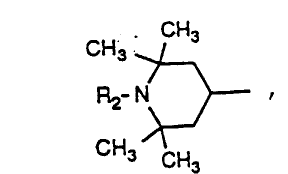 formule dans laquelle R₂ a la signification ci-dessus, ou R₁₇ représente en outre -CH₂CH₂-T₃R₁₉ ou 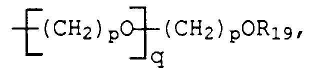 formules dans lesquelles
T₃ représente un atome d'oxygène, de soufre ou 〉N-R₂₂,
R₁₉ 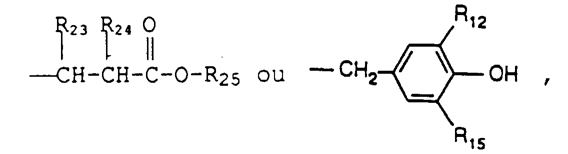 dans laquelle
R₁₂ et R₁₅ ont la signification ci-dessus, ou R₁₉ représente en outre un atome d'hydrogène, un groupe alkyle en C₁-C₁₈, phényle, cycloalkyle en C₅-C₇ ou 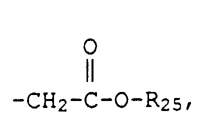 dans laquelle
p représente un nombre entier dans l'intervalle de 2 à 4,
q un nombre entier dans l'intervalle de 2 à 20, R₂₂ représente un groupe alkyle en C₁-C₁₀, phényle, ou cycloalkyle en C₅-C₈,
R₂₃, R₂₄ représentent indépendamment l'un de l'autre un atome d'hydrogène ou un groupe méthyle, à condition que R₂₃ et R₂₄ ne soient pas en même temps le groupe méthyle;
R₂₅ représente un atome d'hydrogène ou un groupe alkyle en C₁-C₁₈, ou
lorsque n = 2,
R₁₇ représente un résidu bivalent d'un hexose, un résidu bivalent d'un hexitol, 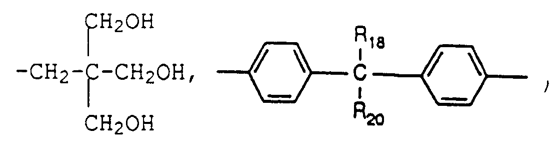 -CᵣH₂ᵣ-, 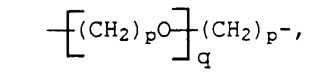 formules dans lesquelles p et q ont la signification ci-dessus, -CH₂CH₂-T₄-CH₂CH₂-, -CH₂-CH=CH-CH₂-, -CH₂-C≡C-CH₂-, 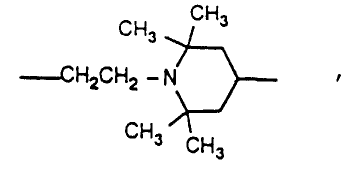 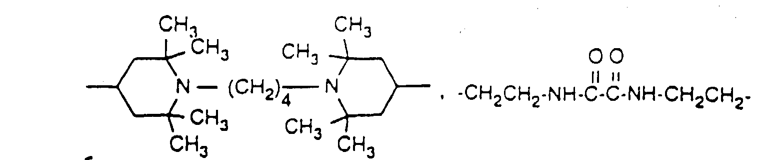 ou 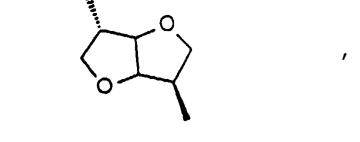 formules dans lesquelles
R₁₈, R₂₀ représentent indépendamment l'un de l'autre un atome d'hydrogène ou un groupe alkyle en C₁-C₆ ou forment ensemble le groupe -CH₂CH₂CH₂CH₂CH₂-,
r représente un nombre entier dans l'intervalle de 2 à 10,
T₄ représente un atome de soufre, 〉N-R₂₆ ou 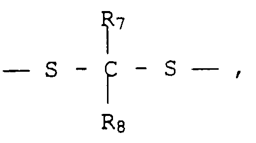 dans lesquelles R₇ et R₈ ont la signification ci-dessus, et
R₂₆ représente un atome d'hydrogène, un groupe alkyle en C₁-C₁₀, phényle, cycloalkyle en C₅-C₈ ou 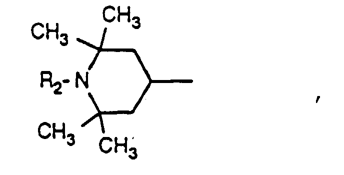
dans laquelle R₂ a la signification ci-dessus, ou dans le composé de formule IV
D représente un atome de soufre, R₅₀-SH, 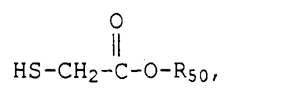 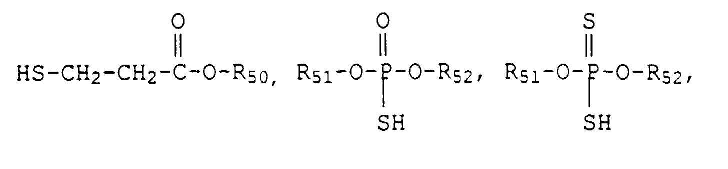 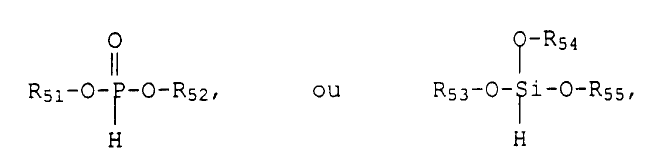
R₅₀ représente un groupe alkyle en C₁-C₁₈, cycloalkyle en C₅-C₁₂,
R₅₁ et R₅₂ représentent indépendamment l'un de l'autre un groupe alkyle en C₁-C₁₂, phényle non substitué ou substitué par un groupe alkyle en C₁-C₁₂, et
R₅₃, R₅₄ et R₅₅ représentent indépendamment les uns des autres un groupe alkyle en C₁-C₁₂ ou phényle.

4. Produit selon la revendication 1,
dans lequel dans le composé de formule I les groupes
Y représentent indépendamment les uns des autres OH, (HOCH₂CH₂)₂N- ou -HNR₁ et
R₁ représente un atome d'hydrogène, un groupe alkyle en C₁-C₄, ou 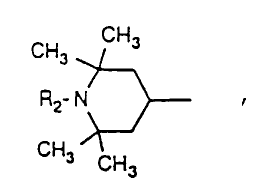 formule dans laquelle
R₂ représente un atome d'hydrogène, un groupe alkyle en C₁-C₄, OH, allyle, benzyle, acétyle ou HOCH₂CH₂-, et
a représente le nombre 1, 2, 3, 4 ou 6, dans lequel
lorsque Y = OH et a = 1,
X représente un groupe alkyle en C₁-C₁₈, alcényle en C₃-C₁₈, -CH₂CH₂T₁(CH₂CH₂O)_{b}R₄ ou 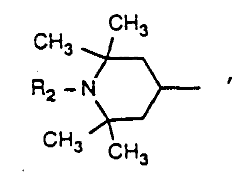 dans laquelle R₂ a la signification ci-dessus, et
T₁ représente un atome d'oxygène,
R₄ représente un groupe alkyle en C₁-C₄,
b un nombre entier dans l'intervalle de 0 à 10, ou
lorsque Y = OH et a = 2,
X représente un groupe -CH₂CH₂T₂ (CH₂CH₂O)_{b}CH₂CH₂-, dans lequel b a la signification ci-dessus, ou de plus X -C_{c}H_{2c}-, 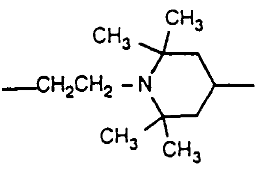 ou -CH₂-CH=CH-CH₂-, dans laquelle
T₂ représente un atome d'oxygène, de soufre ou 〉N-R₅,
R₆ représente un atome d'hydrogène,
b représente le nombre 0 ou 1, et
c représente un nombre entier dans l'intervalle de 2 à 8,
lorsque a = 3,
X représente 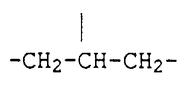 en C₃-C₁₀ ou N(CH₂CH₂-)₃, ou lorsque Y = OH et a = 4,
X représente 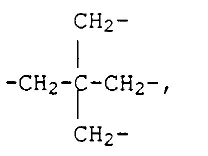 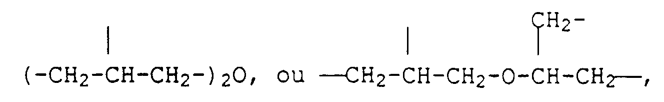 ou
lorsque Y = OH et a = 6,
X représente 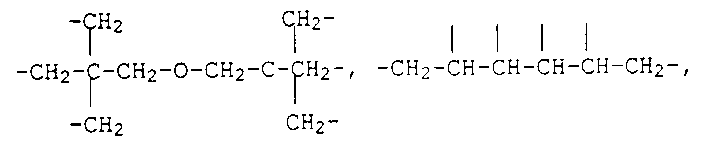 ou
lorsque Y = -HNR₁ et a = 1,
X représente un groupe alkyle en C₁-C₁₀, alcényle en C₃-C₁₈, cycloalkyle en C₅-C₇, ou, 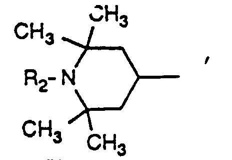 dans laquelle R₂ a la signification ci-dessus, ou
lorsque Y = -HNR₁ et a = 2,
X représente -C_{f}H_{2f}-, formule dans laquelle
f représente un nombre entier dans l'intervalle de 2 à 10, et
dans le composé de formule II, les groupes
Z représente un atome d'hydrogène ou un groupe de formule 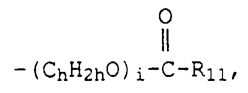 et
k représente 1, 2 ou 3,
h signifie 2 ou 3,
i un nombre entier dans l'intervalle de 0 à 4 et
R₁₁ représente un groupe alcényle en C₈-C₂₀, à la condition que le composé de formule II contienne un groupe 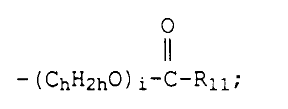 dans le composé de formule III
R₁₂ représente un groupe alkyle en C₁-C₆ ou cycloalkyle en C₅-C₇,
R₁₅ représente un atome d'hydrogène, un groupe alkyle en C₁-C₆ ou cycloalkyle en C₅-C₇,
s représente 1 ou 2,
Q représente -CₘH₂ₘ-, ou 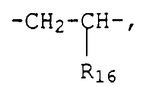
m un nombre entier dans l'intervalle de 0 à 3 et
R₁₆ un groupe alkyle en C₁-C₄ et
n un nombre entier dans l'intervalle de 1 à 6, formule dans laquelle
lorsque n = 1,
R₁₇ représente un atome d'hydrogène, un groupe alkyle en C₁-C₁₈, cycloalkyle en C₅-C₇, alcényle en C₂-C₁₈, un résidu monovalent d'un hexose, un résidu monovalent d'un hexitol, 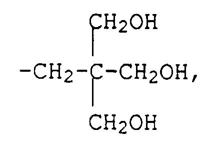 ou 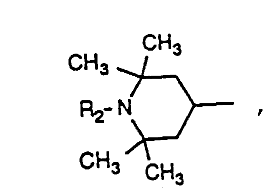 formule dans laquelle R₂ a la signification ci-dessus, ou R₁₇ représente en outre 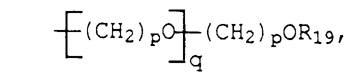 formule dans laquelle
R₁₉ représente un atome d'hydrogène, un groupe alkyle en C₁-C₁₈ ou cycloalkyle en C₅-C₇, dans laquelle
p représente un nombre entier dans l'intervalle de 2 à 4,
q un nombre entier dans l'intervalle de 2 à 10, ou
lorsque n = 2,
R₁₇ représente un résidu bivalent d'un hexose, un résidu bivalent d'un hexitol, 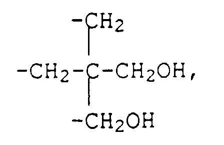 -CᵣH₂ᵣ-, 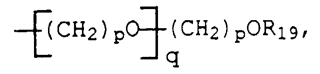 formules dans lesquelles p et q ont la signification ci-dessus,
-CH₂CH₂-T₄-CH₂CH₂- ou 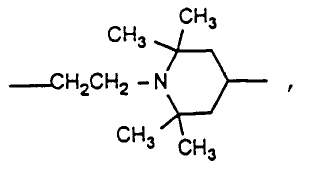
formules dans lesquelles
r représente un nombre entier dans l'intervalle de 2 à 10,
T₄ représente un atome de soufre, 〉N-R₂₆ et
R₂₆ représente un atome d'hydrogène, un groupe alkyle en C₁-C₁₀, cycloalkyle en C₅-C₈, ou
lorsque n = 3,
R₁₇ représente un résidu trivalent d'un hexose, un résidu trivalent d'un hexitol, 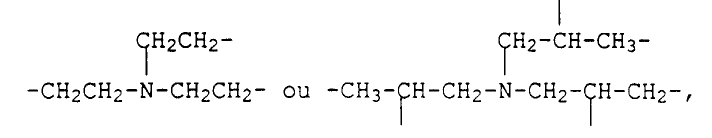 ou
lorsque n = 4,
R₁₇ représente un résidu tétravalent d'un hexose, un résidu tétravalent d'un hexitol, 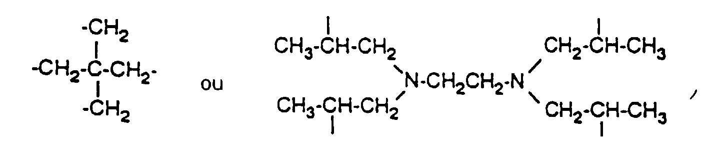 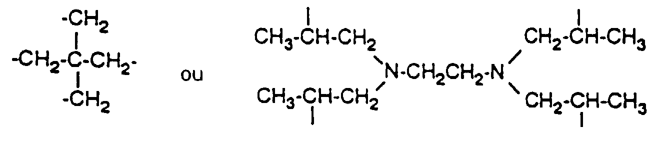
dans le composé de formule IV
D représente un atome de soufre, 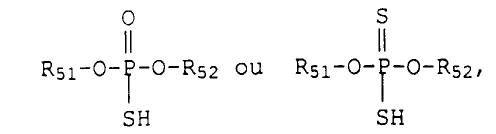 et
R₅₁ et R₅₂ représentent indépendamment l'un de l'autre un groupe alkyle en C₁-C₁₂, phényle non substitué ou substitué par un groupe alkyle en C₁-C₈.

5. Produit selon la revendication 1,
dans lesquels dans le composé de formule I les groupes
Y représentent indépendamment les uns des autres un groupe hydroxy, ou -HNR₁ et
a représente un nombre entier dans l'intervalle de 1 à 4, dans lesquels
lorsque a = 1,
X = 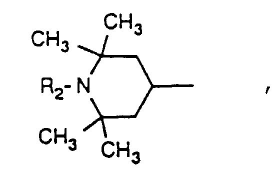 et
R₂ représente un atome d'hydrogène, un groupe méthyle ou HOCH₂CH₂-, ou
lorsque Y = OH et a = 2,
X représente un groupe -CH₂CH₂T₂(CH₂CH₂O)_{b}CH₂CH₂-, -C_{c}H_{2c}- ou 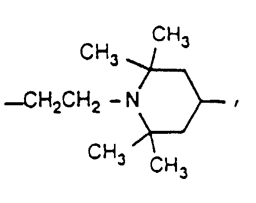 formules dans lesquelles
T₂ représente un atome d'oxygène, de soufre ou 〉N-R₅,
R₅ représente un atome d'hydrogène,
b représente le nombre 0 ou 1, et
c le nombre 2, 3ou 4, ou
lorsque Y = OH et a = 3,
X représente 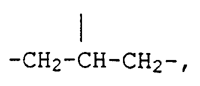 ou
lorsque Y = OH et a = 4,
X représente 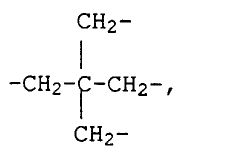
dans le composé de formule II, les groupes
Z représente un atome d'hydrogène ou un groupe de formule 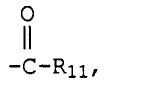
k représente le chiffre 1, et
R₁₁ représente un groupe alcényle en C₈-C₂₀, à la condition que le composé de formule II contienne un groupe 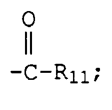 et
dans le composé de formule III
R₁₂ représente un groupe tert.-butyle,
R₁₅ représente un groupe alkyle en C₁-C₄ et est lié en position ortho par rapport au groupe OH,
s représente le chiffre 1,
Q représente -CₘH₂ₘ-, et est lié en position para par rapport au groupe OH, formule dans laquelle
m représente le nombre 2,
n représente 1, et
R₁₇ représente un groupe alkyle en C₁-C₄,
dans le composé de formule IV
D représente un atome de soufre, ou 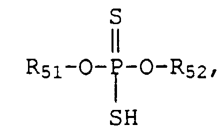 et
R₅₁ et R₅₂ représentent indépendamment l'un de l'autre un groupe alkyle en C₁-C₈.

6. Produit selon la revendication 1,
dans lequel, dans le composé de formule IV
D représente un atome de soufre, ou 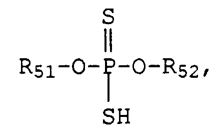 et
R₅₁ et R₅₂ représentent indépendamment l'un de l'autre un groupe alkyle en C₁-C₁₂, phényle non substitué ou substitué par un groupe alkyle en C₁-C₈.

7. Produit selon la revendication 1,
dans lequel dans le composé de formule III
R₁₂ représente un groupe alkyle en C₁-C₄ ou cyclohexyle,
R₁₅ représente un groupe alkyle en C₁-C₄ ou cyclohexyle et est lié en position ortho par rapport au groupe OH,
s représente le chiffre 1,
Q -CₘH₂ₘ et est lié en position para par rapport au groupe OH, formules dans lesquelles
m représente un nombre entier dans l'intervalle de O à 3, et
n un nombre entier dans l'intervalle de 1 à 4, dans lesquelles
lorsque n = 1,
R₁₇ représente un atome d'hydrogène, un groupe alkyle en C₁-C₁₀, cyclohexyle, alcényle en C₂-C₁₈, ou 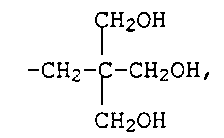 ou
lorsque n = 2,
R₁₇ représente 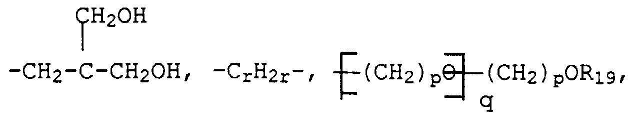 ou -CH₂CH₂-T₄-CH₂CH₂-, formules dans lesquelles
p représente un nombre entier dans l'intervalle de 2 à 4,
q représente un nombre entier dans l'intervalle de 2 à 10,
r représente un nombre entier dans l'intervalle de 2 à 6,
T₄ représente un atome de soufre ou 〉N-R₂₆ et
R₂₆ représente un atome d'hydrogène ou un groupe alkyle en C₁-C₄, ou
lorsque n = 3,
R₁₇ représente 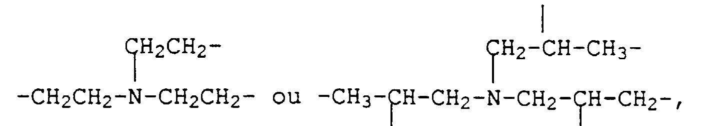 ou
lorsque n = 4,
R₁₇ représente

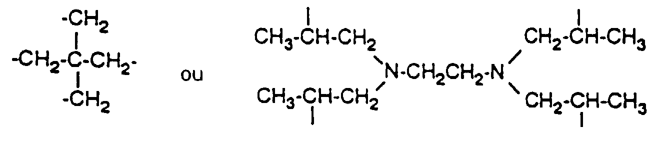

8. Produit selon la revendication 1,
dans lequel dans le composé de formule III
R₁₂ représente un groupe tert.-butyle,
R₁₅ représente un groupe alkyle en C₁-C₄ et est lié en position ortho par rapport au groupe OH,
s signifie le chiffre 1,
Q représente -CₘH₂ₘ- et est lié en position para par rapport au groupe OH, formules dans lesquelles
m représente le nombre 2 et
n un nombre entier 1, 2 ou 4, dans lesquelles
lorsque n = 1,
R₁₇ représente un groupe alkyle en C₁-C₄, ou
lorsque n = 2,
R₁₇ représente 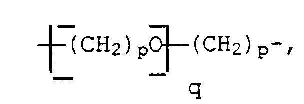 ou-CH₂CH₂-T₄-CH₂CH₂-, formules dans lesquelles
p représente le nombre 2,
q représente le nombre 2,
T₄ représente un atome de soufre, ou
lorsque n = 4,
R₁₇ représente

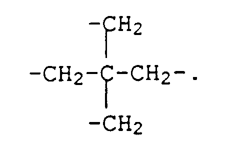

9. Produit selon la revendication 1, dans lequel le composé de formule I représente le pentaérythritol, le thiodiéthylèneglycol, le 1,4-butanediol, le 1,2-propanediol, le diéthylèneglycol, le triéthylèneglycol, la diéthanolamine ou la glycérine, le composé de formule II représente l'huile de tournesol, l'huile de coco, l'huile de colza, l'huile de germe de maïs, l'huile de chardon, l'huile d'olive, l'huile d'arachide, l'huile de coton, ou le Radiamuls ou un mélange de ceux-ci, le composé de formule III représente le 3-(3',5'-di-tert.-butyl-4'-hydroxy-phényl)propionate de méthyle et le 3-(3',5'-tert.-butyl-4'-hydroxy-5'-méthyl-phényl)propionate de méthyle, et le composé de formule IV représente le soufre ou le dithiophosphate de diisopropyle.

10. Produit selon la revendication 1, dans lequel le rapport molaire en quantité des composants a), b), c) et d) est de 0,1:1:0,1:0,1:0,1 jusqu'à 15:1:30:10.

11. Produit selon la revendication 1, dans lequel la teneur en poids du groupe actif E-2 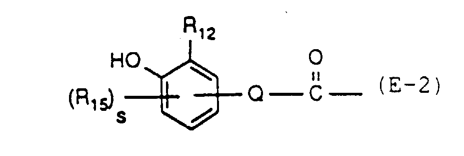 est de 30 à 80% en poids.

12. Produit selon la revendication 1, dans lequel on met d'abord à réagir ensemble les composants a) et b) et ensuite le composé intermédiaire ainsi obtenu avec le composant c) et on le met ensuite à réagir avec le composant d).

13. Composition, contenant
α) un matériau organique soumis à la dégradation thermique, par oxydation ou induite par la lumière et
β) au moins un produit selon la revendication 1.

14. Composition selon la revendication 13, dans laquelle le composant α) est un lubrifiant, un liquide hydraulique, un liquide d'usinage des métaux ou un polymère synthétique.

15. Composition selon la revendication 13, dans laquelle le composant α) est un lubrifiant choisi parmi les huiles minérales, les huiles synthétiques ou un mélange de celles-ci.

16. Composition selon la revendication 13, dans laquelle le composant α) est un polymère synthétique.

17. Composition selon la revendication 13, dans laquelle le composant α) est une polyoléfine ou un copolymère du styrène.

18. Composition selon la revendication 13, contenant en plus du composant α) et du composant β) d'autres additifs additionnels.

19. Composition selon la revendication 18, contenant comme additif supplémentaire des antioxydants de type amine.

20. Utilisation de produits selon la revendication 1 pour la stabilisation de polymères et d'huiles contre la dégradation thermique, par oxydation ou induite par la lumière.

21. Procédé de stabilisation de polymères ou d'huiles contre la dégradation thermique, par oxydation ou induite par la lumière, caractérisé en ce qu'on incorpore à celui-ci un produit selon la revendication 1.

22. Procédé de préparation des produits selon la revendication 1, caractérisé en ce que les composés a), b), c) et d) définis dans la revendication 1, sont mis à réagir dans un rapport en quantités molaires de 0,1:1:0,1:0,1 jusqu'à 15:1:30:10.
